# EUROPEAN PATENT APPLICATION

(11) **EP 4 458 827 A1**
(43) Date of publication of application: **06.11.2024**
(21) Application number: 22916849.7
(22) Date of filing: 30.12.2022
(51) Int. Cl.: C07D 487/04, A61K 31/519, A61K 31/5377, A61P 35/00

(54) **PYRAZOLOPYRIMIDINE DERIVATIVE AND ANTICANCER PHARMACEUTICAL COMPOSITION CONTAINING SAME AS ACTIVE INGREDIENT**

(30) Priority: 30.12.2021 KR 20210193467; 29.12.2022 KR 20220189965
(71) Applicant: Korea Research Institute of Chemical Technology, Daejeon 34114 (KR); Yonsei University, University-Industry Foundation(UIF)., Seoul 03722 (KR)
(72) Inventor: JEONG, Joon, Seoul 06272 (KR); SHIN, Hye Kyoung, Goyang-si, Gyeonggi-do 10551 (KR); AHN, Sun Joo, Daejeon 34114 (KR); LIM, Hee Jong, Daejeon 34114 (KR); SONG, Jin Sook, Daejeon 34114 (KR); KIM, Seong Hwan, Daejeon 34114 (KR); CHAE, Chong Hak, Daejeon 34114 (KR); KIM, Eun Hye, Daejeon 34114 (KR); CHOI, Ji Hye, Daejeon 34114 (KR); LIM, Jong Seung, Daejeon 34114 (KR); JANG, Eun Soo, Daejeon 34114 (KR); SONG, Jin Woo, Daejeon 34114 (KR); KIM, Do Hyun, Daejeon 34114 (KR)
(74) Representative: Kraus & Lederer PartGmbB
(86) International application number: PCT/KR2022/021745
(87) International publication number: WO 2023/128708

(57) **Abstract**

The present invention relates to a pharmaceutical composition for the prevention or treatment of tumors, containing a novel pyrazolopyrimidine compound or a pharmaceutically acceptable salt thereof as an active ingredient. The pharmaceutical composition is administered in combination with other anticancer agents, and thus can be effectively used as a composition for preventing or treating cancer.

## Description

### Technical Field

The present invention relates to a pharmaceutical composition containing, as an active ingredient, a novel pyrazolopyrimidine compound as a TTK inhibitor or a pharmaceutically acceptable salt thereof for the prevention or treatment of a tumor, and to a health food composition containing the pyrazolopyrimidine compound as an active ingredient for the prevention or alleviation of a tumor.

### Background Art

Among the medicines used to treat cancer are the taxanes and vinca alkaloids that act on microtubules to stabilize or destabilize microtubule dynamics. These perturb normal mitotic spindle function, preventing correct chromosome attachment and inducing mitotic arrest. This arrest is enforced by the spindle assembly checkpoint and prevents separation of sister chromatids to form the two daughter cells. Prolonged arrest in mitosis forces a cell into mitotic exit without cytokinesis or into mitotic catastrophe leading to cell death.

Although mitotic agents are broadly used in the treatment of solid tumors, the side effects associated with these agents and the resistance of many types of tumors to the current therapies require the development of new pharmaceutical compositions in the treatment of cancer.

The roles that the genes involved in the spindle assembly checkpoint play in normal development and their potential roles in disease such as cancer have been widely studied (Weaver BA et al.; and Musacchio A et al.). Many components are phosphorylated during mitosis, and several of them are kinases, one of which is the dual specificity kinase TTK. TTK expression is associated with highly proliferating cells and tissues with overexpression observed in a number of cancer cell lines and tumor types, and silencing of TTK in several species leads to failure of cells to arrest in mitosis in response to spindle poisons indicating its essential function in spindle assembly checkpoint signaling (Abrieu A et al.; and Stucke, VM et al.).

These findings suggest that pharmacological inhibitors of TTK and other components of the spindle assembly checkpoint should be of therapeutic value for treatment of proliferative disease including solid tumors, such as carcinomas and sarcomas, and the leukaemias and lymphoid malignancies. In addition, TTK inhibitors would be useful in the treatment of other disorders associated with uncontrolled cellular proliferation.

The dual specificity protein kinase TTK/Mps1 is a master regulator of mitosis and is overexpressed in a variety of patient cancers, so the potential of TTK inhibitors as anticancer drugs is actively being considered from various angles.

Under these situation, the present inventors measured the degree of inhibition of activities of various types of kinases through panel assay and verified the inhibition of pTTK expression in triple-negative breast cancer and colon cancer cells. The present inventors completed the present invention by identifying a novel-structured compound with a pyrazolopyrimidine skeleton having strong TTK inhibitory action and excellent selectivity for kinase.

### Disclosure of Invention

### Technical Problem

An aspect of the present invention is to provide a novel-structured compound with a pyrazolopyrimidine skeleton having strong TTK inhibitory action and excellent selectivity for TTK.

### Solution to Problem

In accordance with an aspect of the present invention, there is provided a compound of Chemical Formula 1 below, or a hydrate, solvate, prodrug, or pharmaceutically acceptable salt thereof:

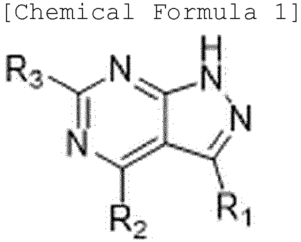

where:
R₁ is substituted or unsubstituted C₆-C₁₂ aryl, substituted or unsubstituted heteroaryl of 5 to 12 atoms, or substituted or unsubstituted heterocycloalkyl of 5 to 12 atoms;
the substituted aryl, heteroaryl, or heterocycloalkyl has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, C(=O), carbamoyl, C₁-C₆ alkylaminoketone, C₁-C₆ alkoxycarbonyl, C₁-C₆ alkylsulfonamino, C₃-C₆ cycloalkylcarboxamido or C₃-C₆ cycloalkylaminoketone;
R₂ is substituted or unsubstituted -NH-(C₆-C₁₂)aryl, substituted or unsubstituted -NH-(C₃-C₆)cycloalkyl, substituted or unsubstituted -NH-(C₃-C₆)heterocycloalkyl, or -NH-(CH₂)ₘ-(C₃-C₆) heterocycloalkyl (where m is an integer of 0, 1, or 2);
the substituted aryl, cycloalkyl, or heterocycloalkyl has one or more hydrogen atoms in a ring thereof, each of which is independently substituted with halogen, C₁-C₆ alkyl, halo C₁-C₆ alkyl, C₁-C₆ alkoxy, or halo C₁-C₆ alkoxy;
R₃ is substituted or unsubstituted -NH-(C₆-C₁₂) aryl or substituted or unsubstituted -NH-(C₃-C₆)cycloalkyl;
the substituted aryl or cycloalkyl has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, substituted or unsubstituted morpholine, substituted or unsubstituted piperazine, substituted or unsubstituted triazole, C₁-C₆ alkylaminoketone, substituted or unsubstituted -C(=O)-morpholine, substituted or unsubstituted -C(=O)-piperazine, or substituted or unsubstituted -C(=O)NH-piperidine; and
the substituted morpholine, piperazine, triazole, -C(=O)-morpholine, -C(=O)-piperazine, or -C(=O)NH-piperidine has one or more hydrogen atoms, each of which is independently substituted with halogen, C₁-C₆ alkyl, halo C₁-C₆ alkyl, C₁-C₆ alkoxy.

In accordance with another aspect of the present invention, there is provided a compound of Chemical Formula 2-1 below, or a hydrate, solvate, prodrug, or pharmaceutically acceptable salt thereof: 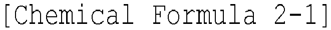 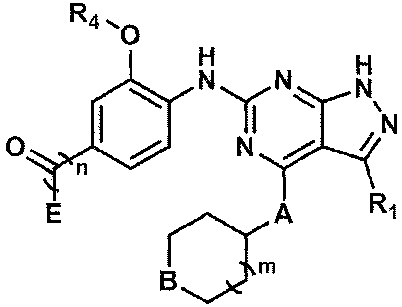 where:
R₁ is substituted or unsubstituted pyridine, substituted or unsubstituted pyrazole, substituted or unsubstituted imidazole, substituted or unsubstituted furan, substituted or unsubstituted thiazole, substituted or unsubstituted pyrimidine, substituted or unsubstituted triazole, substituted or unsubstituted oxadiazole, substituted or unsubstituted oxazole, substituted or unsubstituted isoxazole, substituted or unsubstituted benzimidazole, substituted or unsubstituted indazole, substituted or unsubstituted benzoxazole, substituted or unsubstituted 1,3,4-oxadiazolone, substituted or unsubstituted benzimidazole, substituted or unsubstituted indazole, or substituted or unsubstituted benzoxazole;
the substituted pyridine, pyrazole, imidazole, furan, thiazole, pyrimidine, triazole, oxadiazole, oxazole, isoxazole, benzimidazole, indazole, benzoxazole, 1,3,4-oxadiazolone, benzimidazole, indazole, or benzoxazole has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, C(=O), carbamoyl, C₁-C₆ alkylaminoketone, C₁-C₆ alkoxycarbonyl, C₁-C₆ alkylsulfonamino, C₃-C₆ cycloalkylcarboxamido or C₃-C₆ cycloalkylaminoketone;
R₄ is substituted with hydrogen or C₁-C₆ alkyl; 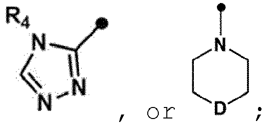
A is substituted with NH;
B is substituted with O, S, or CH₂;
D is substituted with O or NR₄;
m is an integer of 0, 1, or 2; and
n is an integer of 0 or 1.

In accordance with another aspect of the present invention, there is provided a compound of Chemical Formula 2-2 below, or a hydrate, solvate, prodrug, or pharmaceutically acceptable salt thereof:

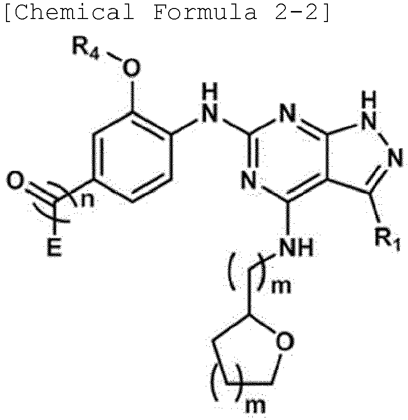

where:
R₁ is substituted or unsubstituted pyridine, substituted or unsubstituted pyrazole, substituted or unsubstituted imidazole, substituted or unsubstituted furan, substituted or unsubstituted thiazole, substituted or unsubstituted pyrimidine, substituted or unsubstituted triazole, substituted or unsubstituted oxadiazole, substituted or unsubstituted oxazole, substituted or unsubstituted isoxazole, substituted or unsubstituted benzimidazole, substituted or unsubstituted indazole, substituted or unsubstituted benzoxazole, substituted or unsubstituted 1,3,4-oxadiazolone, substituted or unsubstituted benzimidazole, substituted or unsubstituted indazole, or substituted or unsubstituted benzoxazole;
the substituted pyridine, pyrazole, imidazole, furan, thiazole, pyrimidine, triazole, oxadiazole, oxazole, isoxazole, benzimidazole, indazole, benzoxazole, 1,3,4-oxadiazolone, benzimidazole, indazole, or benzoxazole has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, C(=O), carbamoyl, C₁-C₆ alkylaminoketone, C₁-C₆ alkoxycarbonyl, C₁-C₆ alkylsulfonamino, C₃-C₆ cycloalkylcarboxamido or C₃-C₆ cycloalkylaminoketone;
R₄ is substituted with hydrogen or C₁-C₆ alkyl;
E is substituted with -NHR₄, 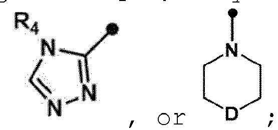
D is substituted with O or NR₄;
m is an integer of 0, 1, or 2; and
n is an integer of 0 or 1.

In accordance with another aspect of the present invention, there is provided a compound of Chemical Formula 3-1 below, or a hydrate, solvate, prodrug, or pharmaceutically acceptable salt thereof: 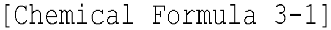 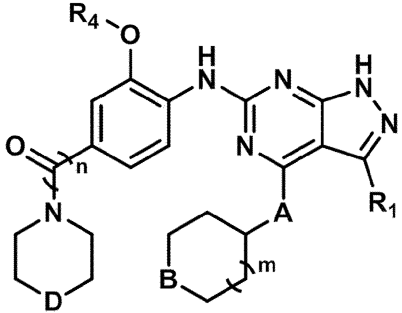 where:
R₁ is substituted or unsubstituted pyridine, substituted or unsubstituted pyrazole, substituted or unsubstituted imidazole, substituted or unsubstituted furan, substituted or unsubstituted thiazole, substituted or unsubstituted pyrimidine, substituted or unsubstituted triazole, substituted or unsubstituted oxadiazole, substituted or unsubstituted oxazole, substituted or unsubstituted isoxazole, substituted or unsubstituted benzimidazole, substituted or unsubstituted indazole, substituted or unsubstituted benzoxazole, substituted or unsubstituted 1,3,4-oxadiazolone, substituted or unsubstituted benzimidazole, substituted or unsubstituted indazole, or substituted or unsubstituted benzoxazole;
the substituted pyridine, pyrazole, imidazole, furan, thiazole, pyrimidine, triazole, oxadiazole, oxazole, isoxazole, benzimidazole, indazole, benzoxazole, 1,3,4-oxadiazolone, benzimidazole, indazole, or benzoxazole has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, C(=O), carbamoyl, C₁-C₆ alkylaminoketone, C₁-C₆ alkoxycarbonyl, C₁-C₆ alkylsulfonamino, C₃-C₆ cycloalkylcarboxamido or C₃-C₆ cycloalkylaminoketone;
R₄ is substituted with hydrogen or C₁-C₆ alkyl;
A is substituted with NH;
B is substituted with O, S, or CH₂;
D is substituted with O or NR₄;
m is an integer of 0, 1, or 2; and
n is an integer of 0 or 1.

In accordance with another aspect of the present invention, there is provided a compound of Chemical Formula 3-2 below, or a hydrate, solvate, prodrug, or pharmaceutically acceptable salt thereof:

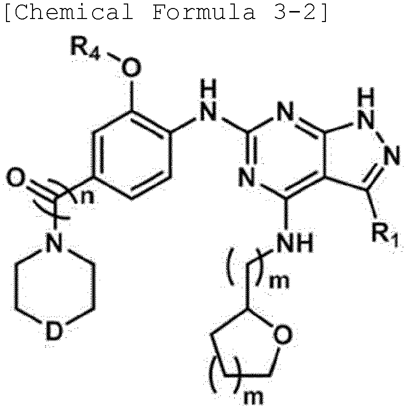

where:
R₁ is substituted or unsubstituted pyridine, substituted or unsubstituted pyrazole, substituted or unsubstituted imidazole, substituted or unsubstituted furan, substituted or unsubstituted thiazole, substituted or unsubstituted pyrimidine, substituted or unsubstituted triazole, substituted or unsubstituted oxadiazole, substituted or unsubstituted oxazole, substituted or unsubstituted isoxazole, substituted or unsubstituted benzimidazole, substituted or unsubstituted indazole, substituted or unsubstituted benzoxazole, substituted or unsubstituted 1,3,4-oxadiazolone, substituted or unsubstituted benzimidazole, substituted or unsubstituted indazole, or substituted or unsubstituted benzoxazole;
the substituted pyridine, pyrazole, imidazole, furan, thiazole, pyrimidine, triazole, oxadiazole, oxazole, isoxazole, benzimidazole, indazole, benzoxazole, 1,3,4-oxadiazolone, benzimidazole, indazole, or benzoxazole has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, C(=O), carbamoyl, C₁-C₆ alkylaminoketone, C₁-C₆ alkoxycarbonyl, C₁-C₆ alkylsulfonamino, C₃-C₆ cycloalkylcarboxamido or C₃-C₆ cycloalkylaminoketone;
R₄ is substituted with hydrogen or C₁-C₆ alkyl;
D is substituted with O or NR₄;
m is an integer of 0, 1, or 2; and
n is an integer of 0 or 1.

Furthermore, the present invention relates to a compound of Chemical Formula 1, Chemical Formula 2-1, Chemical Formula 2-2, Chemical Formula 3-1, or Chemical Formula 3-2, or a hydrate, solvate, prodrug, or pharmaceutically acceptable salt thereof.

In Chemical Formula 1, Chemical Formula 2-1, Chemical Formula 2-2, Chemical Formula 3-1, or Chemical Formula 3-2,
R₁ is substituted or unsubstituted pyridine, substituted or unsubstituted pyrazole, substituted or unsubstituted imidazole, substituted or unsubstituted furan, substituted or unsubstituted thiazole, substituted or unsubstituted pyrimidine, substituted or unsubstituted triazole, substituted or unsubstituted oxadiazole, substituted or unsubstituted oxazole, substituted or unsubstituted benzimidazole, substituted or unsubstituted indazole, substituted or unsubstituted benzoxazole, substituted or unsubstituted 1,3,4-oxadiazolone, substituted or unsubstituted benzimidazole, substituted or unsubstituted indazole, or substituted or unsubstituted benzoxazole; and
the substituted pyridine, pyrazole, imidazole, furan, thiazole, pyrimidine, triazole, oxadiazole, oxazole, benzimidazole, indazole, benzoxazole, or 1,3,4-oxadiazolone has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, C(=O), carbamoyl, C₁-C₆ alkylaminoketone, C₁-C₆ alkoxycarbonyl, C₁-C₆ alkylsulfonamino, or C₃-C₆ cycloalkylaminoketone.

The methods for preparing various-structured compounds with a pyrazolopyrimidine skeleton, of Chemical Formula 1 below, and reaction conditions therefor are as follows.

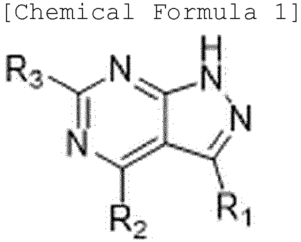

The compounds of a pyrazolopyrimidine framework represented by Chemical Formula 1 may be prepared by Reaction Scheme 1, Reaction Scheme 2, Reaction Scheme 3, Reaction Scheme 4, Reaction Scheme 5, Reaction Scheme 6, Reaction Scheme 7, or Reaction Scheme 8.

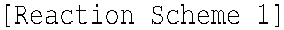

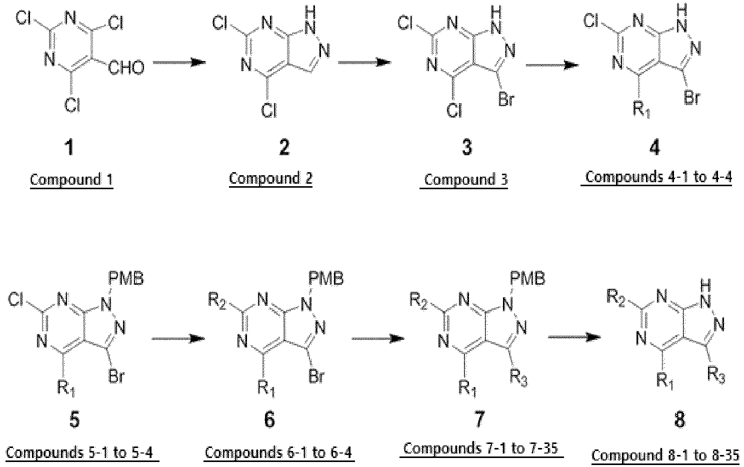

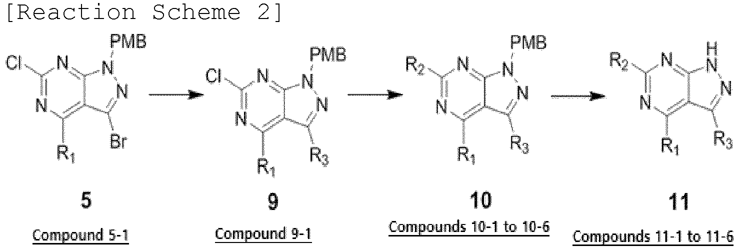

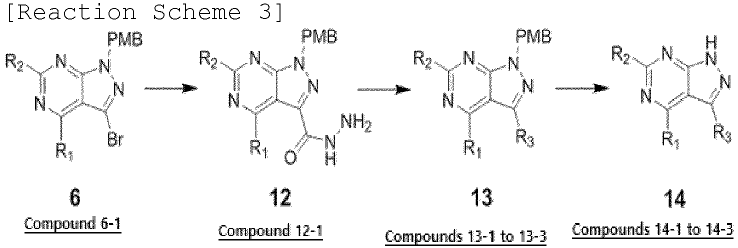

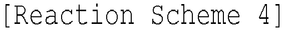

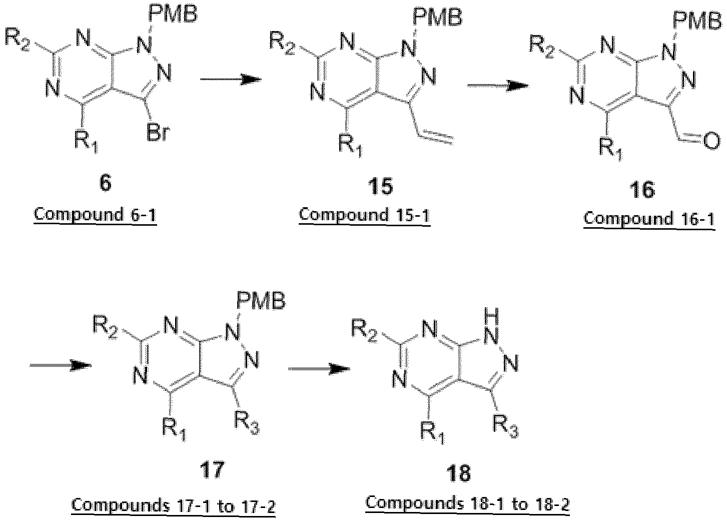

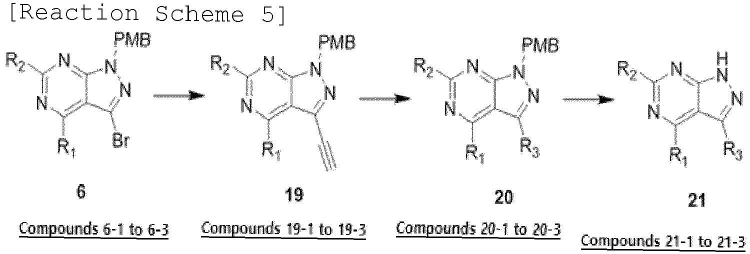

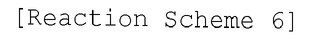

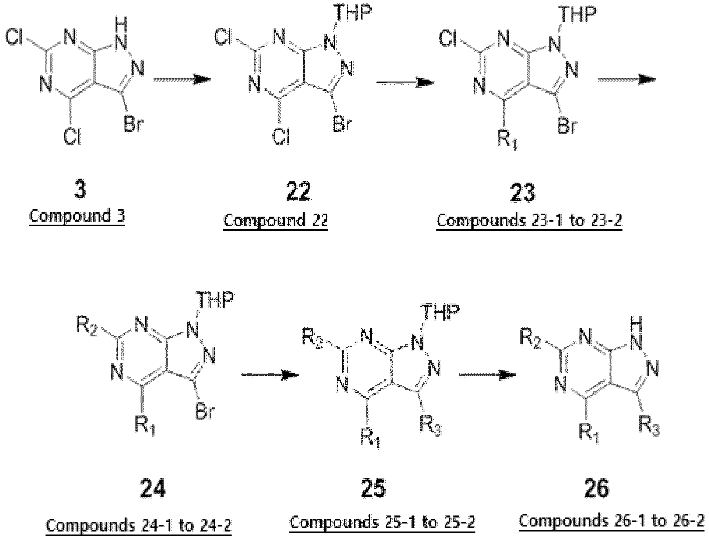

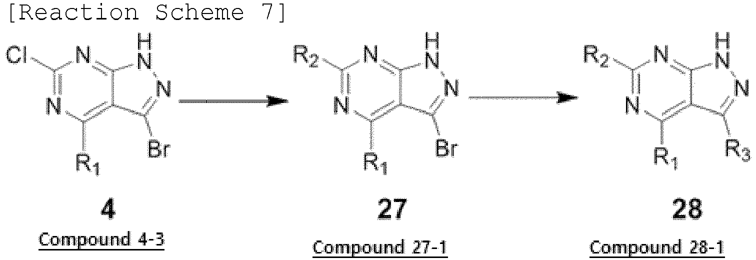

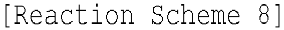

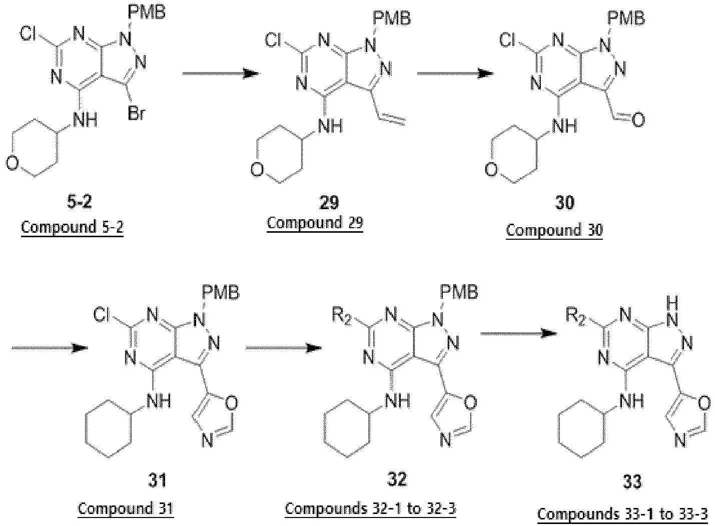

Furthermore, the present invention is directed to a compound represented by Chemical Formula 1 above, or a hydrate, solvate, prodrug, or pharmaceutically acceptable salt thereof, wherein the compound is any one selected from the group consisting of the following compounds:
N⁴-cyclohexyl-3-(5-fluoropyridine-3-yl)-N⁶-(2-methoxy-4-morpholinophenyl)-1H-pyrazolo[3,4-d] pyrimidine-4,6-diamine (Compound 8-1);
3-(4-(cyclohexylamino)-6-((2-methoxy-4-morpholinophenyl)amino)-1H-pyrazolo[3,4-d]pyrimidin-3-yl)benzamide (Compound 8-2);
methyl 4-(4-(cyclohexylamino)-6-((2-methoxy-4-morphonophenyl)amino)-1H-pyrazolo [3,4-d]pyrimidin-3-yl)benzoate (Compound 8-3);
N-(3-(4-(cyclohexylamino)-6-(2-methoxy-4-morpholinophenyl)amino)-1H-pyrazolo [3,4-d]pyrimidin-3-yl)phenyl)cyclopropanecarboxamide (Compound 8-4);
3-(4-(cyclohexylamino)-6-((2-methoxy-4-morphonophenyl)amino)-1H-pyrazolo [3,4-d]pyrimidin-3-yl)-N-methylbenzamide (Compound 8-5);
N-(3-(4-(cyclohexylamino)-6-((2-methoxy-4-morpholinophenyl)amino)-1H-pyrazolo [3,4-d]pyrimidin-3-yl)phenyl)methanesulfonamide (Compound 8-6);
N⁴-cyclohexyl-N⁶-(2-methoxy-4-morpholinophenyl)-3-(1-methyl-1H-pyrazol-4-yl)-1H-pyrazolo[3,4-d]pyrimidine-4,6-diamine (Compound 8-7);
N⁴-cyclohexyl-N⁶-(2-methoxy-4-morpholinophenyl)-1-(4-methoxybenzyl)-3-(1H-pyrazol-4-yl)-1H-pyrazolo[3,4-d]pyrimidine-4,6-diamine (Compound 8-8);
N⁴-cyclohexyl-N⁶-(4-morpholinocyclohexyl)-3-(pyridin-3-yl)-1H-pyrazolo[3,4-d]pyrimidine-4,6-diamine (Compound 8-9);
N⁴-cyclohexyl-N⁶-(2-methoxy-4-morpholinophenyl)-3-(1-methyl-1H-pyrazol-5-yl)-1H-pyrazolo[3,4-d]pyrimidine-4,6-diamine (Compound 8-10);
N⁴-cyclohexyl-3-(1-isopropyl-1H-imidazol-4-yl)-N⁶-(2-methoxy-4-morpholinophenyl)-1H-pyrazolo[3,4-d]pyrimidine-4,6-diamine (Compound 8-11);
N⁴-cyclohexyl-3-(1-(difluoromethyl)-1H-pyrazol-4-yl)-N⁶-(2-methoxy-4-morpholinophenyl)-1H-pyrazolo[3,4-d]pyrimidine-4,6-diamine (Compound 8-12);
N⁴-cyclohexyl-N⁶-(2-methoxy-4-morpholinophenyl)-3-(1-methyl-1H-pyrazol-3-yl)-1H-pyrazolo[3,4-d]pyrimidine-4,6-diamine (Compound 8-13);
N⁴-cyclohexyl-3-(furan-2-yl)-N⁶-(2-methoxy-4-morpholinophenyl)-1H-pyrazolo[3,4-d]pyrimidine-4,6-diamine (Compound 8-14);
N⁴-cyclohexyl-N⁶-(2-methoxy-4-morpholinophenyl)-3-(thiazol-5-yl)-1H-pyrazolo[3,4-d]pyrimidine-4,6-diamine (Compound 8-15);
3-(1H-benzo[d]imidazol-5-yl)-N⁴-cyclohexyl-N⁶-(2-methoxy-4-morpholinophenyl)-1H-pyrazolo[3,4-d]pyrimidine-4,6-diamine (Compound 8-16);
N⁴-cyclohexyl-3-(1H-indazol-5-yl)-N⁶-(2-methoxy-4-morpholinophenyl)-1H-pyrazolo[3,4-d]pyrimidine-4,6-diamine (Compound 8-17);
N⁴-cyclohexyl-N⁶-(2-methoxy-4-morpholinophenyl)-3-(2-methylbenzo[d]oxazol-6-yl)-1H-pyrazolo[3,4-d]pyrimidine-4,6-diamine (Compound 8-18);
N⁴-cyclohexyl-N⁶-(2-methoxy-4-morpholinophenyl)-3-(1-methyl-1H-imidazol-4-yl)-1H-pyrazolo[3,4-d]pyrimidine-4,6-diamine (Compound 8-19);
N⁴-cyclohexyl-3-(isoxazol-4-yl)-N⁶-(2-methoxy-4-morpholinophenyl)-1H-pyrazolo[3,4-d]pyrimidine-4,6-diamine(Compound 8-20);
3-(Isoxazol-4-yl)-N⁶-(2-methoxy-4-morpholinophenyl)-N⁴-(tetrahydro-2H-pyran-4-yl)-1H-pyrazolo[3,4-d]pyrimidine-4,6-diamine(Compound 8-21);
N⁶-(2-methoxy-4-morpholinophenyl)-3-(oxazol-2-yl)-N⁴-(tetrahydro-2H-pyran-4-yl)-1H-pyrazolo[3,4-d]pyrimidine-4,6-diamine(Compound 8-22);
N⁶-(2-methoxy-4-morpholinophenyl)-3-(1-methyl-1H-pyrazol-4-yl)-N⁴-(tetrahydro-2H-pyran-4-yl)-1H-pyrazolo[3,4-d]pyrimidine-4,6-diamine (Compound 8-23);
3-(1-(difluoromethyl)-1H-pyrazol-4-yl)-N⁶-(2-methoxy-4-morpholinophenyl)-N⁴-(tetrahydro-2H-pyran-4-yl)-1H-pyrazolo[3,4-d]pyrimidine-4,6-diamine (Compound 8-24);
N⁶-(2-methoxy-4-morpholinophenyl)-3-(1-methyl-1H-pyrazol-3-yl)-N⁴-(tetrahydro-2H-pyran-4-yl)-1H-pyrazolo[3,4-d]pyrimidine-4,6-diamine (Compound 8-25);
N⁶-(2-methoxy-4-morpholinophenyl)-3-(1H-pyrazol-4-yl)-N⁴-(tetrahydro-2H-pyran-4-yl)-1H-pyrazolo[3,4-d]pyrimidine-4,6-diamine (Compound 8-26);
N⁶-(2-methoxy-4-morpholinophenyl)-3-(pyridin-3-yl)-N⁴-(tetrahydro-2H-pyran-4-yl)-1H-pyrazolo[3,4-d]pyrimidine-4,6-diamine (Compound 8-27);
N⁶-(2-methoxy-4-morpholinophenyl)-3-(pyridin-4-yl)-N⁴-(tetrahydro-2H-pyran-4-yl)-1H-pyrazolo[3,4-d]pyrimidine-4,6-diamine (Compound 8-28);
N⁶-(2-methoxy-4-morpholinophenyl)-3-(pyrimidin-5-yl)-N⁴-(tetrahydro-2H-pyran-4-yl)-1H-pyrazolo[3,4-d]pyrimidine-4,6-diamine (Compound 8-29);
N⁴-cyclohexyl-3-(1-methyl-1H-pyrazol-4-yl)-N⁶-(4-morpholinocyclohexyl)-1H-pyrazolo[3,4-d]pyrimidine-4,6-diamine (Compound 8-30);
N⁴-cyclohexyl-N⁶-(2-methoxy-4-(4-methylpiperazin-1-yl)phenyl)-3-(1-methyl-1H-pyrazol-4-yl)-1H-pyrazolo[3,4-d]pyrimidine-4,6-diamine (Compound 11-1);
N⁴-cyclohexyl-N⁶-(2-methoxy-4-(4-methyl-4H-1,2,4-triazol-3-yl)phenyl)-3-(1-methyl-1H-pyrazol-4-yl)-1H-pyrazolo[3,4-d]pyrimidine-4,6-diamine (Compound 11-2);
4-((4-(cyclohexylamino)-3-(1-methyl-1H-pyrazol-4-yl)-1H-pyrazolo[3,4-d]pyrimidin-6-yl)amino)-3-methoxy-N-methylbenzamide (Compound 11-3);
(4-((4-(cyclohexylamino)-3-(1-methyl-1H-pyrazol-4-yl)-1H-pyrazolo[3,4-d]pyrimidin-6-yl)amino)-3-methoxyphenyl)(morpholino)methanone (Compound 11-4);
(4-((4-(cyclohexylamino)-3-(1-methyl-1H-pyrazol-4-yl)-1H-pyrazolo[3,4-d]pyrimidin-6-yl)amino)-3-methoxyphenyl)(4-methylpiperazin-1-yl)methanone (Compound 11-5);
4-((4-(cyclohexylamino)-3-(1-methyl-1H-pyrazol-4-yl)-1H-pyrazolo[3,4-d]pyrimidin-6-yl)amino)-3-methoxy-N-(1-methylpiperidin-4-yl)benzamide (Compound 11-6);
N⁴-cyclohexyl-N⁶-(2-methoxy-4-morpholinophenyl)-3-(4-methyl-4H-1,2,4-triazol-3-yl)-1H-pyrazolo[3,4-d]pyrimidine-4,6-diamine (Compound 14-1);
N⁴-cyclohexyl-N⁶-(2-methoxy-4-morpholinophenyl)-3-(1,3,4-oxadiazol-2-yl)-1H-pyrazolo[3,4-d]pyrimidine-4,6-diamine (Compound 14-2);
5-(4-(cyclohexylamino)-6-((2-methoxy-4-morpholinophenyl)amino)-1H-pyrazolo[3,4-d]pyrimidin-3-yl)-1,3,4-oxadiazol-2(3H)-one (Compound 14-3);
N⁴-cyclohexyl-N⁶-(2-methoxy-4-morpholinophenyl)-3-(oxazol-5-yl)-1H-pyrazolo[3,4-d]pyrimidine-4,6-diamine (Compound 18-1);
N⁴-cyclohexyl-3-(1H-imidazol-5-yl)-N⁶-(2-methoxy-4-morpholinophenyl)-1H-pyrazolo[3,4-d]pyrimidine-4,6-diamine (Compound 18-2);
N⁴-cyclohexyl-N⁶-(2-methoxy-4-morpholinophenyl)-3-(1H-1,2,3-triazol-4-yl)-1H-pyrazolo[3,4-d]pyrimidine-4,6-diamine (Compound 21-1);
N⁴-cyclohexyl-N⁶-(2-methoxy-4-morpholinophenyl)-3-(1-methyl-1H-1,2,3-triazol-4-yl)-1H-pyrazolo[3,4-d]pyrimidine-4,6-diamine (Compound 21-2);
N⁶-(2-methoxy-4-morpholinophenyl)-3-(1-methyl-1H-1,2,3-triazol-4-yl)-N⁴-(tetrahydro-2H-pyran-4-yl)-1H-pyrazolo[3,4-d]pyrimidine-4,6-diamine (Compound 21-3);
N⁴-cyclohexyl-N⁶-(2-isopropoxy-4-morpholinophenyl)-3-(1-methyl-1H-pyrazol-4-yl)-1H-pyrazolo[3,4-d]pyrimidine-4,6-diamine (Compound 26-1);
4-(cyclohexyloxy)-N-(2-methoxy-4-morpholynophenyl)-3-(1-methyl-1H-pyrazole-4-yl)-1H-pyrazolo[3,4-d]pyrimidine-6-amine (Compound 26-2);
N⁶-(2-methoxy-4-morpholinophenyl)-3-(1-methyl-1H-pyrazol-4-yl)-N⁴-(1-methylpiperidin-4-yl)-1H-pyrazolo[3,4-d]pyrimidine-4,6-diamine(Compound 28-1);
N⁶-(2-methoxy-4-morpholinophenyl)-3-(oxazol-5-yl)-N⁴-(tetra-2H-pyran-4-yl)-1H-pyrazolo[3,4-d]pyrimidine-4,6-diamine(Compound 33-1);
3-methoxy-N-methyl-4-((3-(oxazol-5-yl)-4-((tetrahydro-2H-pyran-4-yl)amino)-1H-pyrazolo[3,4-d]pyrimidin-6-yl)amino)benzamide(Compound 33-2);
N⁶-(2-methoxy-4-(4-methyl-4H-1,2,4-triazol-3-yl)phenyl)-3-(oxazol-5-yl)-N⁴-(tetrahydro-2H-pyran-4-yl)-1H-pyrazolo[3,4-d]pyrimidine-4,6-diamine(Compound 33-3);
N⁶-(2-methoxy-4-morpholinophenyl)-3-(1-methyl-1H-pyrazol-4-yl)-N⁴-((tetrahydrofuran-2-yl)methyl)-1H-pyrazolo[3,4-d]pyrimidine-4,6-diamine(Compound 8-34); and
3-(isoxazol-4-yl)-N⁶-(2-methoxy-4-morpholinophenyl)-N⁴-((tetrahydrofuran-2-yl)methyl)-1H-pyrazolo[3,4-d]pyrimidine-4,6-diamine(Compound 8-35).

In another aspect of the present specification, there is provided a pharmaceutical composition for prevention or treatment of cancer, the pharmaceutical composition containing a therapeutically effective amount of the compound represented by Chemical Formula 1 above, or a hydrate, solvate, prodrug, or pharmaceutically acceptable salt thereof, and a pharmaceutically acceptable carrier.

In another aspect of the present specification, the pharmaceutical composition may exhibit inhibitory activity on at least one protein kinase selected from the group consisting of TTK(h), ALK(h), IR(h) activated, IRR(h), and LTK(h).

In another aspect of the present specification, the pharmaceutical composition exhibits inhibitory activity on TTK kinase.

In another aspect of the present invention, there is provided a composition providing a synergistic anticancer effect by co-administration of the compound represented by Chemical Formula 1 above and another anticancer drug, wherein the anticancer drug is preferably selected from taxane-based anticancer drugs (paclitaxel, docetaxel, and cabazitaxel).

Examples of the anticancer drug include an antitumor alkylating agent, an antitumor antimetabolite, an antitumor antibiotic, a plant-derived antitumor agent, an antitumor platinum complex, an antitumor camptothecin derivative, an antitumor kinase inhibitor, an antitumor antibody, a hormonal antitumor agent, an antitumor viral agent, or an angiogenesis inhibitor.

The antitumor alkylating agent is nitrogen mustard N-oxide, cyclophosphamide, ifosfamide, melphalan, busulfan, mitobronitol, carboquone, thiotepa, ranimustine, nimustine, temozolomide, or carmustine.

The antitumor antimetabolite is methotrexate, 6-mercaptopurine riboside, mercaptopurine, 5-fluorouracil, tegafur, doxifluridine, carmofur, cytarabine, cytarabine ocfosfate, enocitabine, S-1, gemcitabine, fludarabine, or pemetrexed disodium.

The antitumor antibiotic is actinomycin D, doxorubicin, daunorubicin, neocarzinostatin, bleomycin, peplomycin, mitomycin C, aclarubicin, pirarubicin, epirubicin, zinostatin stimalamer, idarubicin, sirolimus, or valrubicin.

The plant-derived antitumor agent is vincristine, vinblastin, vindesine, etoposide, sobuzoxane, docetaxel, paclitaxel, or vinorelbine.

The antitumor platinum complex is cisplatin, carboplatin, nedaplatin, or oxaliplatin.

The antitumor camptothecin derivative is irinotecan, topotecan, or camptothecin.

The antitumor tyrosine kinase inhibitor is gefitinib, imatinib, or erlotinib.

The monoclonal antibody is cetuximab, bevacizumab, rituximab, bevacizumab, alemtuzumab, or trastuzumab.

The hormonal antitumor agent is goserelin, leuprolide, or tamoxifen.

The antitumor virus agent is Imlygic.

The angiogenesis inhibitor is avastin, bevacizumab, ranibizumab, pegaptanib, aflibercept, excitinib, cabozantinib, aflibercept, brivanib, thibosinib, ramucirumab, or motesanib.

The following terms in the present invention have the following meanings, unless otherwise indicated. Any undefined term has a meaning as understood in the art.

The term "alkyl" refers to a single-bonded, straight- or branched-chain hydrocarbon group having 1 to 20 carbon atoms, preferably 1 to 6 carbon atoms, and examples thereof include methyl, ethyl, n-propyl, isopropyl, n-butyl, isobutyl, tert-butyl, 1-methylpropyl, pentyl, and hexyl.

The term "alkoxy" refers to an oxygen group to which a single-bonded, straight- or branched-chain saturated hydrocarbon group having 1 to 20 carbon atoms, preferably 1 to 6 carbon atoms, is attached, and examples thereof include methoxy, ethoxy, propoxy, n-butoxy, tert-butoxy, and 1-methylpropoxy.

The term "alkoxyalkoxy" refers to an alkoxy group in which at least one hydrogen atom of an alkoxy group is replaced with another alkoxy group, and alkoxy is the same as descried above.

The terms "halo" and "halogen" are used in the accepted meaning to refer to a fluoro, chloro, bromo, or iodo substituent.

The term "haloalkyl" refers to an alkyl group in which at least one hydrogen atom of an alkyl group is replaced with a halo group, and the alkyl and halo groups are the same as described above.

The term "haloalkyl" refers to an alkoxy group in which at least one hydrogen atom of an alkoxy group is replaced with a halo group, and the halo and alkoxy groups are the same as described above.

The term "cycloalkyl" refers to a cyclic, single-bonded, saturated hydrocarbon group. Examples thereof include cyclopropyl, cyclobutyl, cyclopentyl, cyclohexyl, cycloheptyl, cyclooctyl, bicyclo[3.1.1] heptyl, spyro[4.5]decyl, spyro[5.5]undecyl, adamantyl, and norbonyl, but are not limited thereto.

The term "aryl" refers to an aromatic substituent having at least one ring with a covalent pi electron system, and examples thereof include phenyl, benzyl, naphthyl, anthryl, indanyl, biphenyl, and triphenyl, but are not limited thereto.

The term "heterocycloalkyl" refers to a cyclic, single-bonded saturated hydrocarbon group containing at least one heteroatom, such as N, O, or S, and examples thereof include aziridinyl, pyrrolidinyl, piperidinyl, oxopiperidinyl, morphorinyl, piperazinyl, oxopiperazinyl, morpholineyl, thiomorphorinyl, azepanyl, diazepanyl, oxazepanyl, thiazepanyl, deoxothiazepanyl, azetidinyl, azocanyl, tetrahydrofuranyl, tetrahydroisoquinolineyl, tetrahydropiranyl, tetrahydrobenzo[b]thiophenyl, oxazolidinyl, dioxanyl, dioxolanyl, azaspiro[5.5]undecanyl, azaspiro[3,3]heptanyl, or azaspiro[3.5]nonanyl, according to the numbers and kinds of heteroatoms and the number of carbon atoms within the ring, but are not limited thereto.

The term "heateroaryl" refers to an aromatic cyclic compound including at least one heteroatom, such as N, O, or S, and examples thereof include pyridyl, pyrrolyl, pyrrolidinyl, pyridinyl, furanyl, quinolidinyl, indolyl, pyrimidinyl, imidazolyl, 1,2,3-triazolyl, 1,2,4-triazolyl, tetrazolyl, pyranyl, thiophenyl, thiazolyl, dibenzothiophenyl, dibenzofuranyl, dibenzoselenophene, thiophene, benzofuran, benzothiophenyl, benzoselenophenyl, carbazolyl, indolocarbazolyl, pyridylindolyl, pyrrolodipyridinyl, pyrazolyl, imidazolyl, triazolyl, oxazolyl, thiazolyl, oxadiazolyl, oxatriazolyl, dioxazolyl, thiadiazolyl, pyridinyl, pyridazinyl, pyrazinyl, triazinyl, oxazinyl, oxathiazinyl, oxadiazinyl, indolyl, benzimidazolyl, indazolyl, indoxazinyl, benzoxazolyl, benzisoxazolyl, benzothiazolyl, quinolinyl, isoquinolinyl, cinnolinyl, quinazolinyl, quinoxalinyl, naphthyridinyl, phthalazinyl, pteridinyl, xanthenyl, acridinyl, phenazinyl, phenothiazinyl, phenoxazinyl, benzofuropyridinyl, furodipyridinyl, benzoimidazolyl, benzoxazolyl, benzothienopyridinyl, thienodipyridinyl, benzoselenophenopyridinyl, and selenophenodipyridinyl, but are not limited thereto.

In the present invention, the pharmaceutically acceptable salt refers to a salt or complex of Chemical Formula 1 having preferable biological activity. Examples of the salt include acid addition salts formed from inorganic acids (e.g., hydrochloric acid, hydrobromic acid, sulfuric acid, phosphoric acid, nitric acid, etc.), and salts formed from organic acids, such as acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, and poly-galacturonic acid, but are not limited thereto. The compound may be administered as a pharmaceutically acceptable quaternary salt known to a person skilled in the art, and particularly, examples thereof include chloride, bromide, iodide, -O-alkyl, toluene sulfonate, methyl sulfonate, sulfonate, phosphate, or carbohydrate (e.g., benzoate, succinate, acetate, glycolate, maleate, malate, fumarate, citrate, tartrate, ascorbate, cinnamoate, mandelate, and diphenyl acetate). The compound of Chemical Formula 1 of the present invention may include all salts, hydrates, solvates, and prodrugs that can be prepared by typical methods, as well as the pharmaceutically acceptable salt.

The acid addition salt according to the present invention may be prepared by a typical method, and for example, the acid addition salt may be prepared by dissolving a derivative of Chemical Formula 1 in an organic solvent, such as methanol, ethanol, acetone, dichloromethane, or acetonitrile, adding an organic acid or inorganic acid thereto to generate a precipitate, and then filtering and drying the precipitate, or may be prepared by distilling a solvent and an excess acid under reduced pressure, followed by drying and crystallization in an organic solvent.

In addition, a pharmaceutically acceptable metal salt may be prepared by using a base. An alkali metal or alkaline earth metal salt is obtained, for example, by dissolving the compound in an alkali metal hydroxide or alkaline earth metal hydroxide solution in excess amount, filtering the undissolved compound salt, evaporating the filtrate, and drying. Particularly, the preparation of a sodium, potassium, or calcium salt as the metal salt is pharmaceutically suitable. In addition, the corresponding salt is obtained by reacting an alkali metal or alkaline earth metal salt with a suitable silver salt (e.g., silver nitrate).

The present invention is directed to a pharmaceutical composition characterized in that a pyrazolopyrimidine is used to prevent or treat cancer.

The solid cancer may be any one of brain tumor, benign astrocytoma, malignant astrocytoma, pituitary adenoma, meningioma, primary central nervous system lymphoma, oligodendroglioma, craniopharyngioma, ependymoma, brain stem glioma, head and neck tumor, laryngeal cancer, oropharyngeal cancer, nasal cavity cancer, nasopharyngeal tumor, salivary gland tumor, hypopharyngeal cancer, thyroid cancer, oral cavity tumor, oral squamous cell carcinoma, Barrett's esophagus, thoracic tumor, small cell lung cancer, non-small cell lung cancer, thymic carcinoma, mediastinal neoplasm, esophageal cancer, neuroglioma, breast cancer, male breast cancer, triple negative breast cancer (TNBC), breast sporadic basaloid carcinoma (BLC), abdominal tumor, stomach cancer, liver cancer, gallbladder cancer, biliary tract cancer, pancreatic cancer, pancreatic ductal adenocarcinoma (PDAC), small intestine cancer, colon cancer, anal cancer, colonic mucosal adenoma, bladder cancer, kidney cancer, male genital tract tumor, penile cancer, prostate cancer, female genital tract tumor, cervical cancer, endometrial cancer, ovarian cancer, ovarian adenocarcinoma, uterine sarcoma, vulvar intraepithelial neoplasia, vaginal cancer, female external genitalia cancer, female urethral cancer, and skin cancer, but is not particularly limited thereto. Furthermore, the blood cancer may be selected from the group consisting of leukemia, malignant lymphoma, multiple myeloma, and aplastic anemia, but are not particularly limited thereto.

The pharmaceutical composition according to the present invention may be formulated in a suitable form together with a pharmaceutically acceptable carrier that is usually used. The term "pharmaceutically acceptable" means to a composition that is physiologically acceptable and does not cause allergic responses, such as gastrointestinal disorder or dizziness, or similar responses, when administered to humans. The composition may be formulated in the form of: an oral dosage form, such as a powder, granules, a tablet, a capsule, a suspension, an emulsion, a syrup, or an aerosol; a preparation for external application; a suppository; and a sterile injectable solution, according to a usual method for each form.

Examples of a carrier, excipients, and diluents that may be contained in the composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl paraoxybenzoate, propyl paraoxybenzoate, talc, magnesium stearate, and a mineral oil, but are not limited thereto. The composition may be formulated into a preparation by using a diluent or an excipient, such as a filler, a stabilizer, a binder, a disintegrant, or a surfactant. A solid preparation for oral administration includes a tablet, a pill, a powder, granules, a capsule, and the like. These solid preparations may be prepared by mixing the compound of the present invention with at least one excipient, for example, starch, microcrystal cellulose, sucrose or lactose, low-substituted hydroxypropyl cellulose, hypromellose, or the like. Alternatively, lubricants, such as magnesium stearate and talc, may be used in addition to the simple excipients. Examples of a liquid preparation for oral administration correspond to suspensions, oral solutions, emulsions, syrups, and the like, and may include simple diluents that are frequently used, such as water and liquid paraffin, as well as several types of excipients, for example, wetting agents, sweetening agents, flavoring agents, and preservatives. Examples of a preparation for parenteral administration include sterile aqueous solutions, non-aqueous solvents, suspensions, emulsions, freeze-dried preparations, and suppositories. As the non-aqueous solvent and the suspensions, propylene glycol, polyethylene glycol, a vegetable oil such as an olive oil, an injectable ester such as ethylolate, and the like may be used. As substrates for the suppositories, Witepsol, Macrogol, Tween 61, cacao butter, laurin butter, glycerol, gelatin, and the like may be used. For the formulation into a dosage form for parenteral administration, the pyrazolopyrimidine derivative compound of Chemical Formula 1 or pharmaceutically acceptable salt thereof may be sterilized, or mixed with an adjuvant, such as a preservative, a stabilizer, a hydrator, an emulsion promoter, a salt for osmotic pressure regulation, or a buffer, and other therapeutically useful substances in water to be prepared into a solution or a suspension, which is then formulated in an ample or a vial unit dosage form.

The pharmaceutical composition containing as an active ingredient the compound of Chemical Formula 1 disclosed in the present invention may be administered to mammalian animals, such as mice, livestock, and humans, via various routes. All manners of administration may be considered, and for example, the administration may be carried out through oral, rectal, intravenous, intramuscular, subcutaneous, endometrial, or intracerebroventricular injection. The dose may vary depending on the age, sex, or body weight of a subject to be treated, the particular disease or pathological condition to be treated, the severity of the disease or pathological condition, the time of administration, the route of administration, the absorption, distribution, and excretion rate of a drug, the kind of another drug used, the determination of a prescriber, and the like. The determination of the dose based on these factors is within the level of a person skilled in the art, and the general dose is in the range of approximately 0.01 mg/kg/day to approximately 2000 mg/kg/day. A more preferable dose is 1 mg/kg/day to 500 mg/kg/day. The administration may be performed once a day or divided into multiple doses. The dose is not intended to limit the scope of the present invention in any aspect.

The pharmaceutical composition of the present invention may be used, for cancer prevention or treatment, alone or in combination with surgery, hormone therapy, chemotherapy, and methods using a biological response regulator.

### Advantageous Effects of Invention

The present invention is directed to a pharmaceutical composition containing, as an active ingredient, a novel pyrazolopyrimidine compound as a TTK inhibitor or a pharmaceutically acceptable salt thereof for the prevention or treatment of a tumor, and to a health food composition containing the pyrazolopyrimidine compound as an active ingredient for the prevention or alleviation of a tumor, wherein these compositions can be advantageously used as compositions for preventing or treating cancer.

### Brief Description of Drawings

FIG. 1 shows the summary of luminance results of a specific protein, induced using an enhanced chemiluminescence kit, in order to investigate the inhibition of pTTK expression in MDA-MB-231 cells.
FIG. 2 shows the summary of luminance results of a specific protein, induced using an enhanced chemiluminescence kit, in order to investigate the inhibition of pTTK expression in HCT116 cells.
FIG. 3 shows graphs depicting the tumor volume over time, observed for a drug administration control group (Control), a CFI-402257 treatment group, and inventive example treatment groups, in nude mice injected with MDA-MB-231 cell line.
FIG. 4 shows graphs depicting the mouse body weight over time, observed for a drug administration control group (Control), a CFI-402257 treatment group, and inventive example treatment groups, in nude mice injected with MDA-MB-231 cell line.

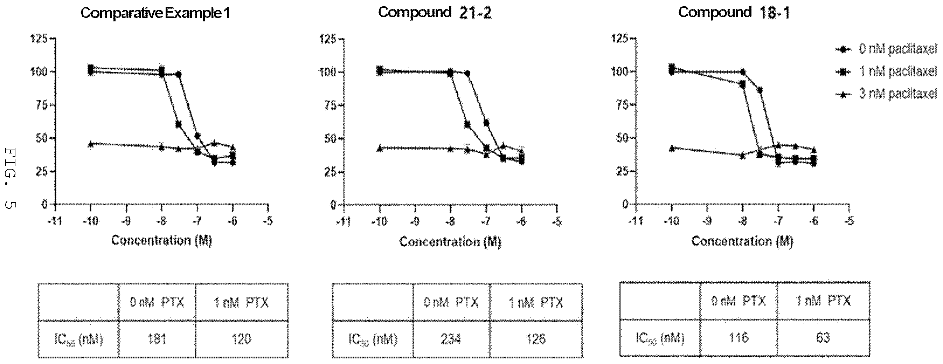

FIG. 5 shows the evaluation results of cytotoxicity when inventive example compounds and paclitaxel were applied to TTK-expressed MDA-MB-231 cells.
FIG. 6 shows the measurement results of the change in cell cycle distribution when inventive example compounds and paclitaxel were applied to TTK-expressed MDA-MB-231 cells.

### Best Mode for Carrying out the Invention

Hereinafter, preferable exemplary embodiments of the present disclosure will be described in detail. However, the present disclosure is not limited to the exemplary embodiments described herein and can be embodied in many different forms. Rather, these exemplary embodiments are provided so that the present disclosure will be thorough and complete and will fully convey the scope of the disclosure to those skilled in the art.

### <Example A. Preparation of Compound 8 by Reaction Scheme 1>

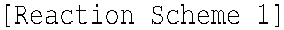

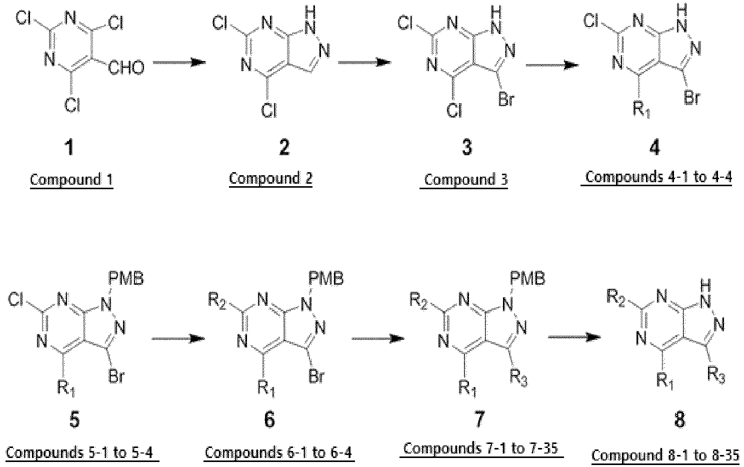

### Preparation Example 1. Synthesis of 4,6-dichloro-1H-pyrazolo[3,4-d]pyrimidine (Compound 2)

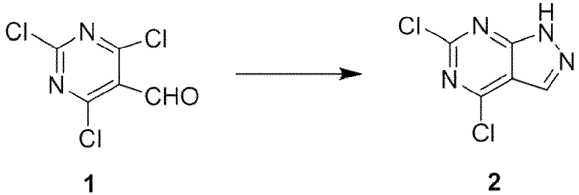

Compound **1** (4.63 g, 21.89 mmol) was dissolved in methanol (MeOH; 94 mL) and then stirred at -12°C, and hydrazine monohydrate (NH₂NH₂·H₂O; 1.2 mL, 24.08 mmol) was dissolved in MeOH (10 mL) and then added dropwise for 30 minutes. Triethylamine (TEA; 3.2 mL, 22.55 mmol) was dissolved in MeOH (10 mL) and then added dropwise at -12°C for 30 minutes, followed by stirring at room temperature for 40 minutes. The mixture was concentrated under reduced pressure and then washed with ethyl acetate (EA; 30 mL), and thereafter, the filtrate was concentrated under reduced pressure. The residue was dissolved in dichloromethane (MC; 7 mL), and then hexane (Hx; 10 mL) was added to generate a solid. The resultant solid was removed by filtration, and the solution was concentrated under reduced pressure, and then subjected to separation by silica gel column chromatography (EA:Hx = 2:3), thereby obtaining Compound **2** as a light yellow solid (3.20 g, 77%).

¹H-NMR (300 MHz, DMSO-d₆) δ 8.50 (s, 1H), 14.64 (s, 1H) .

### Preparation Example 2. Synthesis of 3-boromo-4,6-dichloro-1H-pyrazolo[3,4-d]pyrimidine (Compound 3)

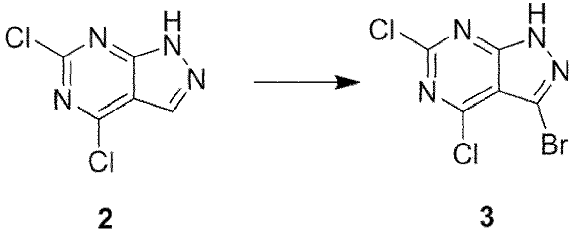

Compound **2** (15.0 g, 79.30 mmol) was dissolved in acetonitrile (MeCN; 150 mL), and then N-bromosuccinimide (NBS; 15.52 g, 87.23 mmol) was added, followed by stirring at 70°C for 15 hours. The mixture was concentrated under reduced pressure, and then EA (150 mL) was added to the residue, followed by washing with water (100 mL). Thereafter, the moisture was removed over sodium sulfate (Na₂SO₄), and the resultant product was concentrated under reduced pressure and purified by silica gel column chromatography (EA:Hx = 1:8) to obtain Compound **3** as a white solid (19.50 g, 91%).

¹H-NMR (300 MHz, DMSO-d₆) δ 14.97 (s, 1H).

### Preparative Example 3-1. Synthesis of 3-Bromo-6-chloro-N-cyclohexyl-1H-pyrazolo[3,4-d]pyrimidin-4-amine (Compound 4-1)

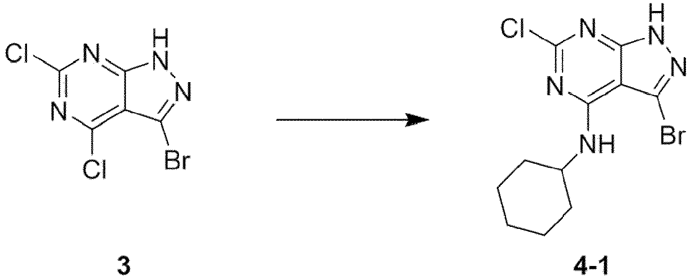

Compound **3** (5.0 g, 18.60 mmol) was dissolved in ethanol (EtOH; 250 mL), and then N,N-diisopropylethylamine (DIPEA; 4.2 mL, 24.18 mmol) was added. Thereafter, cyclohexylamine (2.75 mL, 24.18 mmol) was added dropwise at -15°C for 3 minutes, followed by stirring at room temperature for 21 hours. EA (100 mL) and tetrahydrofuran (THF; 20 mL) were added to the reaction product, and then the mixture was washed with water (40 mL). The water layer (40 mL) was extracted with EA (20 mL). The organic layer was combined, followed by washing with salt water (30 mL), and then the moisture was removed (Na₂SO₄), and the resultant product was concentrated under reduced pressure. The residue was purified by silica gel column chromatography (EA:Hx = 1:8) to obtain Compound **4-1** as a white solid (4.19 g, 67%).

¹H-NMR (500 MHz, DMSO-d₆) δ 1.16-1.26 (m, 1H), 1.32-1.42 (m, 2H), 1.44-1.53 (m, 2H), 1.56-1.64 (m, 1H), 1.68-1.76 (m, 2H), 1.86-1.94 (m, 2H), 4.02-4.14 (m, 1H), 6.70 (d, J = 8.1 Hz, 1H), 13.97 (br s, 1H).

### Preparative Example 3-2. Synthesis of 3-Bromo-6-chloro-N-(tetrahydro-2H-pyran-4-yl)-1H-pyrazolo[3,4-d]pyrimidin-4-amine (Compound 4-2)

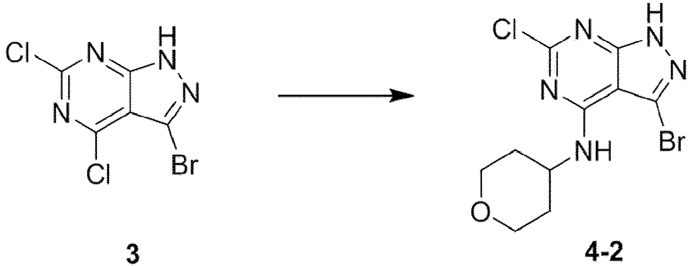

Compound **3** (400 mg, 1.49 mmol) and 4-aminotetrahydropyran (181 mg, 1.79 mmol) were subjected to the same preparation method as in Compound **4-1** to obtain Compound **4-2** as a white solid (286 mg, 57%).

¹H NMR (300 MHz, DMSO-d₆) δ 1.67-1.92 (m, 4H), 3.44 (td, J = 11.6, 2.6 Hz, 2H), 3.84-3.95 (m, 2H), 4.23-4.40 (m, 1H), 6.90 (d, J = 7.9 Hz, 1H), 14.00 (s, 1H).

### Preparative Example 3-3. Synthesis of 3-Bromo-6-chloro-N-(1-methylpiperidin-4-yl)-1H-pyrazolo[3,4-d]pyrimidin-4-amine (Compound 4-3)

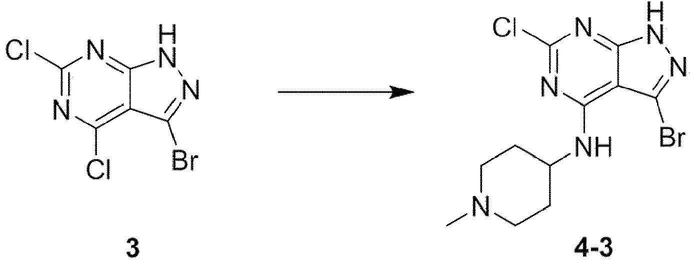

Compound **3** (400 mg, 1.49 mmol) and 4-amino-1-methylpiperidine (204 mg, 1.79 mmol) were subjected to the same preparation method as in Compound **4-1** to obtain Compound **4-3** as a white solid (220 mg, 42%).

¹H NMR (300 MHz, DMSO-d₆) δ 1.68-1.82 (m, 2H), 1.83-1.93 (m, 2H), 2.06-2.18 (m, 2H), 2.22 (s, 3H), 2.72-2.82 (m, 2H), 4.04-1.15 (m, 1H), 6.80 (d, J = 7.9 Hz, 1H).

### Preparative Example 3-4. Synthesis of 3-Bromo-6-chloro-N-((tetrahydrofuran-2-yl)methyl)-1H-pyrazolo[3,4-d]pyrimidin-4-amine (Compound 4-4)

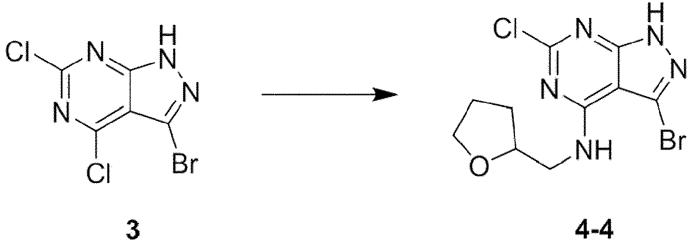

Compound **3** (3g, 11.20mmol) and (tetrahydrofuran-2-yl)methanamine (1.38mL, 13.44mmol) were subjected to the same preparation method as in Compound **4-1** to obtain Compound **4-4** as a white solid (1.9 g, 51%).

¹H NMR (500 MHz, DMSO-d₆) δ 1.54 - 1.66 (m, 1H), 1.85 (s, 3H), 3.53 - 3.70 (m, 3H), 3.81 (s, 1H), 4.10 (s, 1H), 7.22 (s, 1H).

### Preparative Example 4-1. Synthesis of 3-Bromo-6-chloro-N-cyclohexyl-1-(4-methoxybenzyl)-1H-pyrazolo[3,4-d]pyrimidin-4-amine (Compound 5-1)

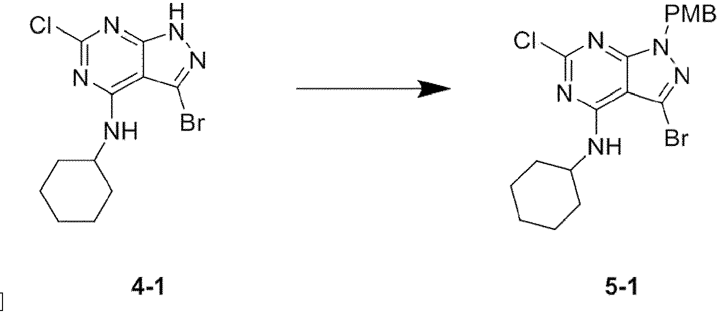

Compound **4-1** (4.19 g, 12.67 mmol) and cesium carbonate (Cs₂CO₃; 4.95 g, 15.20 mmol) were dissolved in N,N-dimethylformamide (DMF; 63 mL) and then p-methoxybenzyl chloride (PMBCl; 1.80 mL, 13.30 mmol) was added dropwise at 0°C and stirred at 30°C for 12 hours. EA (150 mL) was added to the mixture and then washed with water (80 mL), and thereafter, the water layer was extracted with EA (40 mL). The organic layer was combined, followed by washing with water (20 X 2 mL), and then the moisture was removed (Na₂SO₄), and the resultant product was concentrated under reduced pressure. The residue was purified by silica gel column chromatography (EA:Hx = 1:9) to obtain Compound **5-1** as a white solid (3.71 g, 64%).

¹H-NMR (500 MHz, DMSO-d₆) δ 1.14-1.25 (m, 1H), 1.31-1.42 (m, 2H), 1.44-1.58 (m, 2H), 1.56-1.64 (m, 1H), 1.68-1.76 (m, 2H), 1.86-1.92 (m, 2H), 3.72 (s, 3H), 4.05-4.14 (m, 1H), 5.34 (s, 2H), 6.80 (d, J = 8.1 Hz, 1H), 6.89 (d, J = 8.6 Hz, 2H), 7.19 (d, J = 8.6 Hz, 2H).

### Preparative Example 4-2. Synthesis of 3-Bromo-6-chloro-1-(4-methoxybenzyl)-N-(tetrahydro-2H-pyran-4-yl)-1H-pyrazolo[3,4-d]pyrimidin-4-amine (Compound 5-2)

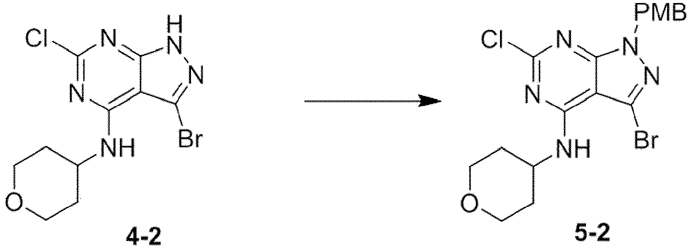

Compound **4-2** (1.45 g, 4.35 mmol) was subjected to the same preparation method as in Compound **5-1** to obtain Compound **5-2** as a white solid (1.38 mg, 68%).

¹H-NMR (300 MHz, DMSO-d₆) δ 1.68-1.90 (m, 4H), 3.44 (td, J = 11.3, 2.6 Hz, 2H), 3.72 (s, 3H), 3.89 (dt, J = 11.8, 3.8 Hz, 2H), 4.25-4.39 (m, 1H), 5.35 (s, 2H), 6.89 (d, J = 8.7 Hz, 2H), 7.20 (d, J = 8.6 Hz, 2H).

### Preparative Example 4-3. Synthesis of 3-Bromo-6-chloro-1-(4-methoxybenzyl)-N-((tetrahydrofuran-2-yl)methyl)-1H-pyrazolo[3,4-d]pyrimidin-4-amine (Compound 5-4)

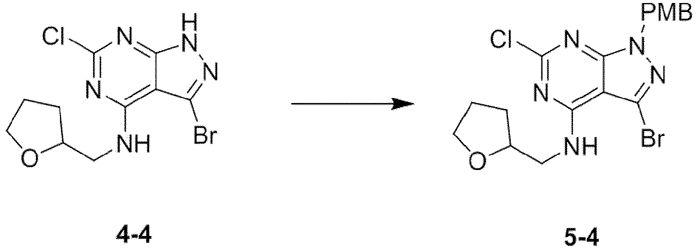

Compound **4-4** (1.5 g, 4.51 mmol) was subjected to the same preparation method as in Compound **5-1** to obtain Compound **5-4** as a white solid (0.912 g, 45%).

¹H NMR (300 MHz, Chloroform-d) δ 1.43 (d, J = 1.0 Hz, 1H), 1.62 (s, 3H), 2.00 (d, J = 31.8 Hz, 4H), 3.58 (dt, J = 13.1, 6.2 Hz, 1H), 3.75 - 3.98 (m, 7H), 4.63 (d, J = 4.5 Hz, 1H), 5.38 (s, 2H), 6.58 (s, 1H), 6.86 (s, 3H), 7.31 (s, 3H).

### Preparation Example 5-1. Synthesis of 3-bromo-N⁴-cyclohexyl-N⁶-(2-methoxy-4-morpholinophenyl)-1-(4-methoxybenzyl)-1H-pyrazolo[3,4-d]pyrimidine-4,6-diamine (Compound 6-1)

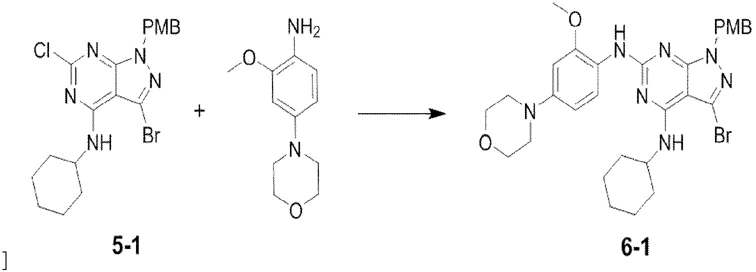

Compound **5-1** (3.71 g, 8.23 mmol) and 2-methoxy-4-morpholinoaniline (2.05 g, 9.87 mmol) were dissolved in 2-butanol (2-BuOH; 45 mL), and then trifluoroacetic acid (TFA; 0.63 mL, 8.23 mmol) was added, and stirred at 110°C for 22 hours. The mixture was concentrated under reduced pressure, and then water (20 mL) was added to the residue. Thereafter, the pH was adjusted to 7 by addition of an aqueous solution of saturated sodium bicarbonate (sat'd NaHCO₃), followed by extraction with EA (60 mL). The organic layer was subjected to moisture removal (Na₂SO₄) and concentrated under reduced pressure. The residue was separated by silica gel column chromatography (EA:Hx = 1:2) to obtain Compound **6-1** as a light yellow solid (4.89 g, 89%).

¹H NMR (500 MHz, DMSO-d₆) δ 1.19-1.26 (m, 1H), 1.31-1.46 (m, 4H), 1.56-1.63 (m, 1H), 1.68-1.75 (m, 2H), 1.90-1.96 (m, 2H), 3.06-3.11 (m, 4H), 3.71 (s, 3H), 3.73-3.76 (m, 4H), 3.84 (s, 3H), 3.99-4.07 (m, 1H), 5.23 (s, 2H), 6.09 (d, J = 7.9 Hz, 1H), 6.50 (dd, J = 8.9, 2.6 Hz, 1H), 6.65 (d, J = 2.6 Hz, 1H), 6.89 (d, J = 8.6 Hz, 2H), 7.20 (d, J = 8.6 Hz, 2H), 7.65 (s, 1H), 8.02 (d, J = 8.8 Hz, 1H).

### Preparation Example 5-2. Synthesis of 3-bromo-N⁴-cyclohexyl-1-(4-methoxybenzyl)-N⁶-(4-morpholinocyclohexyl)-1H-pyrazolo[3,4-d]pyrimidine-4,6-diamine (Compound 6-2)

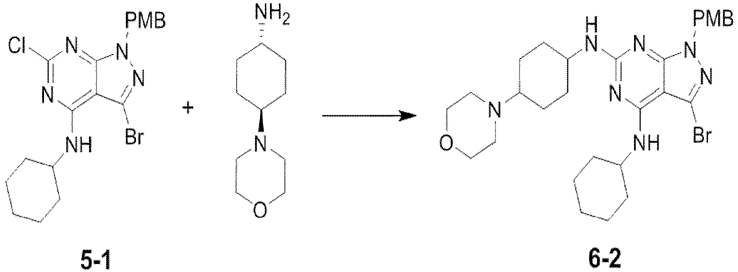

Compound **5-1** (102 mg, 0.226 mmol) and trans-4-morpholinocyclohexan-1-amine (62 mg, 0.339 mmol) were subjected to the same preparation method as in Compound **6-1** to obtain Compound **6-2** as a white solid (100 mg, 74%) .

¹H NMR (500 MHz, DMSO-d₆) δ 1.16-1.54 (m, 18H), 1.85-1.88(m, 5H), 1.99-2.10 (m, 4H), 2.21-2.32 (m, 3H), 3.73-3.85 (m, 4H), 4.01-4.11 (m, 1H), 4.95 (m, 1H),5,26 (s, 2H), 5.70 (m, 1H), 6.93 (d, J = 8.6 Hz, 2H), 7.20 (d, J = 8.6 Hz, 2H).

### Preparation Example 5-3. Synthesis of 3-Bromo-N⁶-(2-methoxy-4-morpholinophenyl)-1-(4-methoxybenzyl)-N⁴-(tetrahydro-2H-pyran-4-yl)-1H-pyrazolo[3,4-d]pyrimidine-4,6-diamine (Compound 6-3)

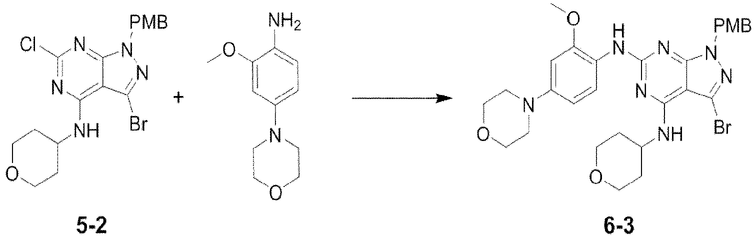

Compound **5-2** (150 mg, 0.33 mmol) was subjected to the same preparation method as in Compound **6-1** to obtain Compound **6-3** as a white solid (128 mg, 62%).

¹H NMR (500 MHz, DMSO-d₆) δ 1.60-1.71 (m, 2H), 1.85-1.92 (m, 2H), 3.07-3.11 (m, 4H), 3.41 (t, J = 11.2 Hz, 2H), 3.71 (s, 3H), 3.73-3.77 (m, 4H), 3.83 (s, 3H), 3.86-3.92 (m, 2H), 1.17-4.27 (m, 1H), 5.23 (s, 2H), 6.24 (d, J = 7.6 Hz, 1H), 6.51 (dd, J = 8.8, 2.5 Hz, 1H), 6.65 (d, J = 2.5 Hz, 1H), 6.89 (d, J = 8.6 Hz, 2H), 7.20 (d, J = 8.6 Hz, 2H), 7.70 (s, 1H), 7.97 (br s, 1H).

### Preparation Example 5-4. Synthesis of 3-Bromo-N⁶-(2-methoxy-4-morpholinophenyl)-1-(4-methoxybenzyl)-N⁴-((tetrahydrofuran-2-yl)methyl)-1H-pyrazolo[3,4-d]pyrimidine-4,6-diamine (Compound 6-4)

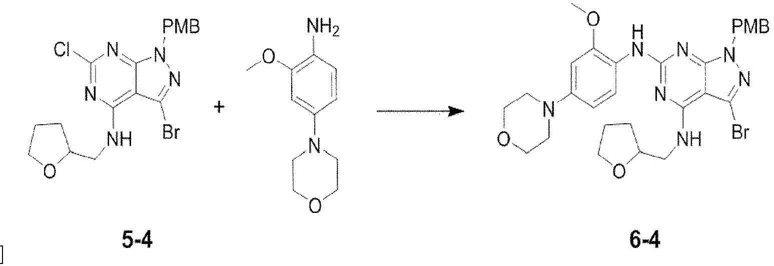

Compound **5-4** (912 mg, 2.014mmol) was subjected to the same preparation method as in Compound **6-1** to obtain Compound **6-4** as a white solid (434 mg, 35%).

¹H NMR (500 MHz, DMSO-d₆) δ 3.09 (s, 4H), 3.52 (s, 1H), 3.65 (s, 2H), 3.73 (d, J = 16.3 Hz, 8H), 3.84 (s, 3H), 4.09 (d, J = 1.9 Hz, 1H), 5.24 (s, 2H), 6.65 (d, J = 2.5 Hz, 3H), 6.90 (s, 2H), 7.20 (s, 2H), 7.67 (s, 1H), 8.02 (s, 1H).

### Example 1. Preparation of N⁴-cyclohexyl-3-(5-fluoropyridin-3-yl)-N⁶-(2-methoxy-4-morpholinophenyl)-1H-pyrazolo[3,4-d]pyrimidine-4,6-diamine (Compound 8-1)

The target compound was prepared in two steps (Suzuki coupling and PMB removal) according to the following reaction scheme.

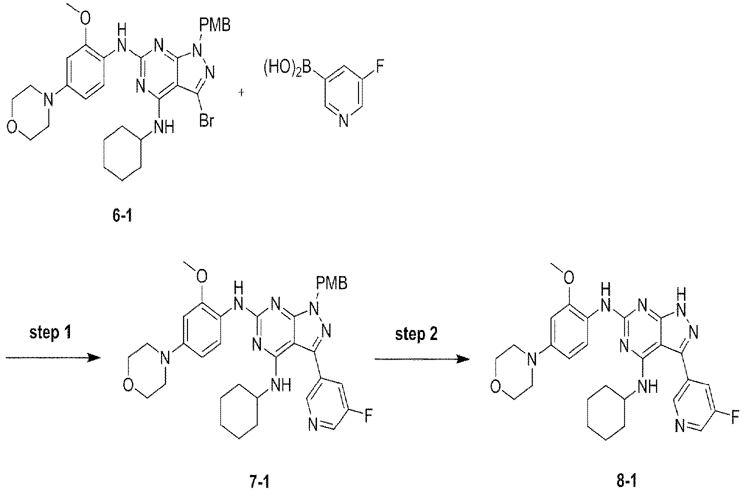

### Step 1. Synthesis of Compound 7-1

Compound **6-1** (20 mg, 0.03 mmol), 3-fluoropyridine-5-boronic acid (7 mg, 0.04 mmol), and potassium carbonate (K₂CO₃; 9 mg, 0.06 mmol) were dissolved in dioxane-H₂O (4:1 in v/v, 2 mL) and then purged with argon gas for 5 minutes. Thereafter, tetrakis(triphenylphosphine) palladium (Pd(PPh₃)₄; 2 mg, 0.001 mmol) was added, followed by stirring at 100°C for 2 hours. The mixture was subjected to moisture removal over sodium sulfate (Na₂SO₄) and filtered through a celite pad, and thereafter the residue was washed with EA (5 mL), and then the filtrate was concentrated under reduced pressure. The residue was separated by silica gel column chromatography (EA:Hx = 1:3) to obtain Compound **7-1** as a yellow solid (11.3 mg, 55%).

¹H NMR (500 MHz, CDCl₃) δ .78-2.1 (m, 9H), 3.15 (t, J = 4.7 Hz, 4H), 3.77 (s, 3H), 3.90 (t, J = 4.7 Hz, 4H), 3.94 (s, 3H), 4.12-4.21 (m, 1H), 5.44 (s, 2H), 6.57 (s, 2H), 6.85 (d, J = 8.2 Hz, 2H), 7.39 (d, J = 8.3 Hz, 2H), 7.44 (s, 1H), 7.74 (d, J = 9.0 Hz, 1H), 8.10 (s, 1H), 8.55 (d, J = 2.8 Hz, 2H), 8.74 (s, 1H).

### Step 2. Preparation of Compound 8-1

Compound **7-1** (11.3 mg, 0.01 mmol) was dissolved in trifluoroacetic acid (TFA; 1 mL), and then methanesulfonic acid (MsOH; 0.1 mL) was added dropwise, followed by stirring at 60°C for 2 hours. The mixture was concentrated under reduced pressure, and the pH of the mixture was adjusted to 7 by saturated sodium bicarbonate. Ethyl acetate (EA; 5 mL) was added, and then the mixture was washed with water (5 mL). Thereafter, the organic extract was dried over sodium sulfate (Na₂SO₄) and concentrated under reduced pressure. The residue was separated by silica gel column chromatography (EA:Hx = 1:2) to obtain Compound **8-1** as a light yellow solid (4.9 mg, 53%).

¹H NMR (500 MHz, DMSO-d₆) δ 1.11-1.21 (m, 1H), 1.23-1.43 (m, 4H), 1.59 (d, J = 12.9 Hz, 1H), 1.69 (d, J = 10.1 Hz, 2H), 1.92 (d, J = 10.1 Hz, 2H), 3.09 (t, J = 4.7 Hz, 4H), 3.76 (t, J = 4.6 Hz, 4H), 3.86 (s, 3H), 3.97-4.10 (m, 1H), 6.14 (d, J = 7.6 Hz, 1H), 6.50 (d, J = 8.8 Hz, 1H), 6.66 (s, 1H), 7.47 (s, 1H), 7.92 (d, J = 9.7 Hz, 1H), 8.04 (d, J = 8.7 Hz, 1H), 8.65 (d, J = 2.8 Hz, 1H), 8.72 (s, 1H), 13.19 (s, 1H).

### Example 2. Preparation of 3-(4-(Cyclohexylamino)-6-((2-methoxy-4-morpholinophenyl)amino)-1H-pyrazolo[3,4-d]pyrimidin-3-yl)benzamide (Compound 8-2)

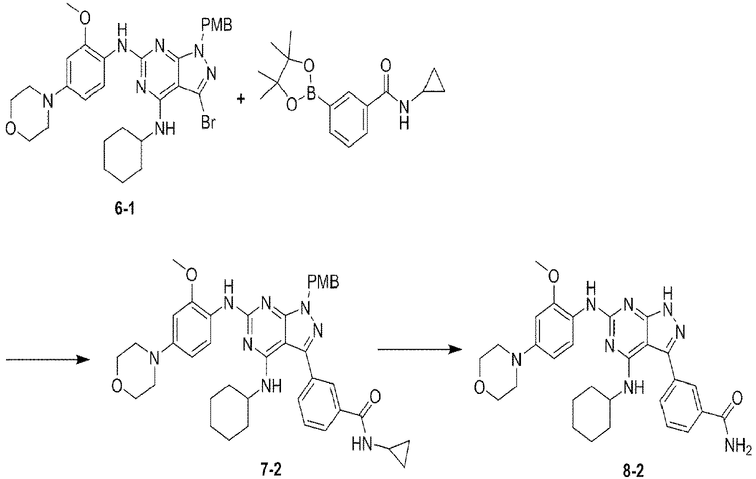

### Step 1. Synthesis of Compound 7-2

Compound **6-1** (50 mg, 0.08 mmol) and N-cyclopropyl-3-(4,4,5,5-tetramethyl-1,3,2-dioxaborolan-2-yl)benzamide (35 mg, 0.12 mmol) were subjected to the same preparation method as in Compound **7-1** to obtain Compound **7-2** as a pale brown solid (42.4 mg, 75%).

¹H-NMR (500 MHz, DMSO-d₆) δ 0.53-0.57 (m, 2H), 0.67-0.72 (m, 2H), 1.14-1.26 (m, 3H), 1.28-1.37 (m, 2H), 1.49-1.62 (m, 3H), 1.85-1.92 (m, 2H), 2.84-2.90 (m, 1H), 3.09 (t, J = 4.7 Hz, 4H), 3.70 (s, 3H), 3.75 (t, J = 4.8 Hz, 4H), 3.87 (s, 3H), 3.98-4.06 (m, 1H), 5.35 (s, 2H), 5.64 (d, J = 5.9 Hz, 1H), 6.51 (dd, J = 8.9, 2.6 Hz, 1H), 6.67 (d, J = 2.5 Hz, 1H), 6.89 (d, J = 8.7 Hz, 2H), 7.26 (d, J = 8.5 Hz, 2H), 7.51 (s, 1H), 7.60 (t, J = 7.7 Hz, 1H), 7.78 (d, J = 7.5 Hz, 1H), 7.91 (d, J = 7.8 Hz, 1H), 8.11 (d, J = 1.8 Hz, 1H), 8.19 (d, J = 8.8 Hz, 1H), 8.54 (d, J = 4.3 Hz, 1H).

### Step 2. Preparation of Compound 8-2

Compound **7-2** (42.4 mg, 0.06 mmol) was subjected to the same preparation method as in Compound **8-1** in step 2 of Example 1 to obtain Compound **8-2** as a pale brown solid (29 mg, 88%).

¹H NMR (500 MHz, DMSO-d₆) δ 1.14-1.27 (m, 3H), 1.34 (q, J = 12.7, 11.7 Hz, 2H), 1.49-1.63 (m, 3H), 1.88-1.95 (m, 2H), 3.09 (t, J = 4.7 Hz, 4H), 3.75 (t, J = 4.6 Hz, 4H), 3.85 (s, 3H), 3.97-4.05 (s, 1H), 5.52 (d, J = 7.6 Hz, 1H), 6.49 (dd, J = 8.8, 2.5 Hz, 1H), 6.65 (d, J = 2.0 Hz, 1H), 7.43 (s, 1H), 7.46 (s, 1H), 7.62 (t, J = 7.7 Hz, 1H), 7.80 (d, J = 7.6 Hz, 1H), 7.97 (d, J = 7.8 Hz, 1H), 8.05-8.11 (m, 2H), 8.19 (s, 1H), 13.01 (s, 1H).

### Example 3. Preparation of Methyl 4-(4-(cyclohexyl amino)-6-((2-methoxy-4-morpholinophenyl)amino)-1H-pyrazolo[3,4-d]pyrimidin-3-yl)benzoate (Compound 8-3)

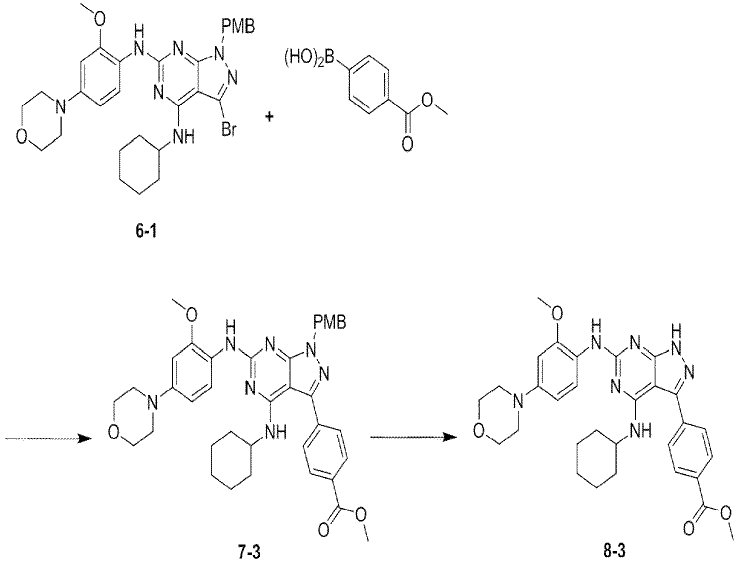

### Step 1. Synthesis of Compound 7-3

Compound **6-1** (60 mg, 0.09 mmol) and (4-(methoxycarbonyl)phenyl)boronic acid (26.0 mg, 0.14 mmol) were subjected to the same preparation method as in Compound **7-1** to obtain Compound **7-3** as a white solid (42 mg, 64%).

¹H NMR (400 MHz, DMSO-d₆) δ 1.20-1.37 (m, 5H), 1.51-1.70 (m, 3H), 1.94 (d, J = 11.6 Hz, 2H), 2.64 (s, 3H), 3.04-3.15 (m, 4H), 3.71 (s, 3H), 3.73-3.78 (m, 4H), 3.87 (s, 3H), 3.98-4.04 (m, 1H), 5.36 (s, 2H), 5.80 (d, J = 8.6 Hz, 1H), 6.52 (d, J = 8.9 Hz, 1H), 6.67 (s, 1H), 6.89 (d, J = 8.1 Hz, 2H), 7.27 (d, J = 8.2 Hz, 2H), 7.54 (s, 1H), 7.80 (d, J = 7.9 Hz, 2H), 8.10 (d, J = 7.9 Hz, 2H), 8.17 (d, J = 8.9 Hz, 1H).

### Step 2. Preparation of Compound 8-3

The same preparation method as in Compound **8-1** in step 2 of Example 1 was used to obtain Compound **8-3** as a pale brown solid (29 mg, 88%).

¹H NMR (500 MHz, DMSO-d₆) δ 1.13-1.20 (m, 1H), 1.26 (q, J = 11.6 Hz, 2H), 1.36 (q, J = 12.8, 12.2 Hz, 2H), 1.58 (d, J = 12.3 Hz, 1H), 1.66 (d, J = 12.3 Hz, 2H), 1.95 (d, J = 10.9 Hz, 2H), 2.65 (s, 3H), 3.06-3.14 (m, 4H), 3.74-3.78 (m, 4H), 3.86 (s, 3H), 3.99-4.08 (m, 1H), 5.67 (d, J = 7.5 Hz, 1H), 6.50 (d, J = 8.9 Hz, 1H), 6.66 (s, 1H), 7.47 (s, 1H), 7.82 (d, J = 7.1 Hz, 2H), 8.05 (d, J = 8.7 Hz, 1H), 8.12 (d, J = 7.8 Hz, 2H), 13.12 (s, 1H) .

### Example 4. Preparation of N-(3-(4-(cyclohexylamino)-6-((2-methoxy-4-morpholinophenyl)amino)-1H-pyrazolo[3,4-d]pyrimidin-3-yl)phenyl)cyclopropanecarboxamide (Compound 8-4)

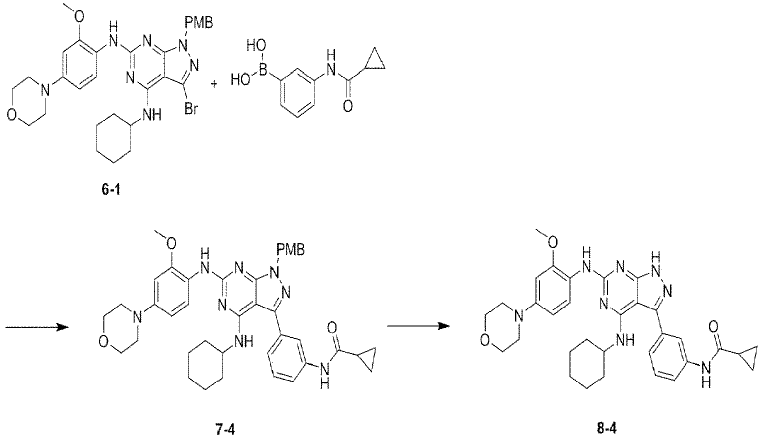

### Step 1. Synthesis of Compound 7-4

Compound **6-1** (50 mg, 0.08 mmol) and (3-(cyclopropanecarboxamido)phenyl)boronic acid (25 mg, 0.12 mmol) were subjected to the same preparation method as in Compound **7-1** to obtain Compound **7-4** as a white solid (47.8 mg, 84%).

¹H-NMR (400 MHz, DMSO-d₆) δ 0.78-0.86 (m, 3H), 1.22-1.38 (m, 6H), 1.51-1.63 (m, 3H), 1.77-1.83 (m, 1H), 1.84-1.92 (m, 2H), 3.06-3.12 (m, 4H), 3.71 (s, 3H), 3.73-3.77 (m, 4H), 3.87 (s, 3H), 3.95-4.06 (m, 1H), 5.34 (s, 2H), 5.75 (d, J = 7.7 Hz, 1H), 6.51 (d, J = 8.8 Hz, 1H), 6.67 (s, 1H), 6.89 (d, J = 8.5 Hz, 2H), 7.25 (d, J = 8.3 Hz, 2H), 7.30 (d, J = 7.2 Hz, 1H), 7.42-7.52 (m, 3H), 8.10 (s, 1H), 8.19 (d, J = 8.6 Hz, 1H), 10.40 (s, 1H) .

### Step 2. Preparation of Compound 8-4

Compound **7-4** (15.7 mg, 0.02 mmol) was subjected to the same preparation method as in Compound **8-1** to obtain Compound **8-4** as a pale brown solid (8.8 mg, 67%).

¹H NMR (500 MHz, DMSO-d₆) δ 0.81-0.84 (m, 3H), 1.12-1.38 (m, 6H), 1.50-1.63 (m, 3H), 1.78-1.84 (m, 1H), 1.85-1.91 (m, 2H), 3.06-3.11 (m, 4H), 3.73-3.78 (m, 4H), 3.85 (s, 3H), 3.97-4.07 (m, 1H), 5.66 (d, J = 7.6 Hz, 1H), 6.48 (dd, J = 8.8, 2.5 Hz, 1H), 6.65 (d, J = 2.6 Hz, 1H), 7.29-7.31 (m, 1H), 7.40 (s, 1H), 7.44-7.50 (m, 2H), 8.07 (d, J = 8.7 Hz, 1H), 8.13 (s, 1H), 10.41 (s, 1H), 12.94 (s, 1H).

### Example 5. Preparation of 3-(4-(cyclohexylamino)-6-((2-methoxy-4-morpholinophenyl)amino)-1H-pyrazolo[3,4-d]pyrimidin-3-yl)-N-methylbenzamide (Compound 8-5)

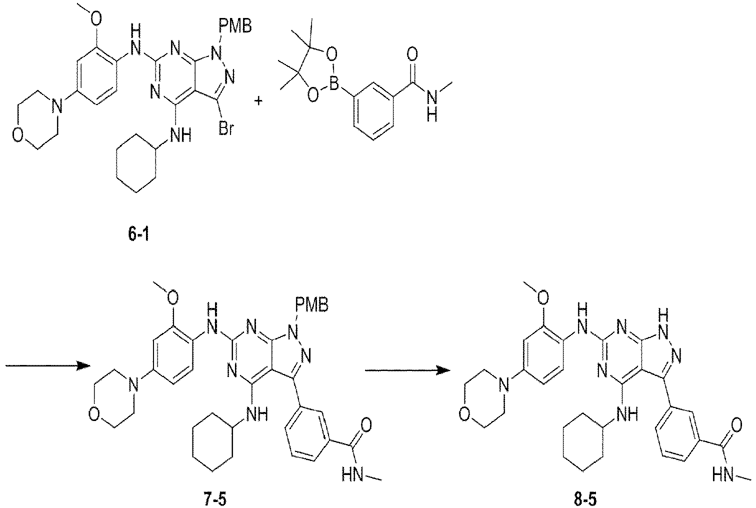

### Step 1. Synthesis of Compound 7-5

Compound **6-1** (50 mg, 0.08 mmol) and N-methyl-3-(4,4,5,5-tetramethyl-1,3,2-dioxaborolan-2-yl)benzamide (32 mg, 0.12 mmol) were subjected to the same preparation method as in Compound **7-1** to obtain Compound **7-5** as a brown solid (32.5 mg, 59%).

¹H-NMR (500 MHz, DMSO-d₆) δ 0.83-0.92 (m, 2H), 1.17-1.34 (m, 4H), 1.48-1.61 (m, 2H), 1.85-1.92 (m, 2H), 2.80 (d, J = 4.4 Hz, 3H), 3.09 (t, J = 4.8 Hz, 4H), 3.70 (s, 3H), 3.75 (t, J = 4.8 Hz, 4H), 3.87 (s, 3H), 3.98-4.05 (m, 1H), 5.36 (s, 2H), 5.66 (d, J = 7.4 Hz, 1H), 6.52 (dd, J = 8.5, 2.4 Hz, 1H), 6.67 (d, J = 2.5 Hz, 1H), 6.89 (d, J = 8.6 Hz, 2H), 7.27 (d, J = 8.5 Hz, 2H), 7.51 (s, 1H), 7.61 (t, J = 7.7 Hz, 1H), 7.78 (d, J = 7.6 Hz, 1H), 7.91 (d, J = 7.8 Hz, 1H), 8.11 (d, J = 16.8 Hz, 1H), 8.19 (d, J = 8.9 Hz, 1H), 8.55 (q, J = 4.1 Hz, 1H).

### Step 2. Preparation of Compound 8-5

Compound **7-5** (32.5 mg, 0.04 mmol) was subjected to the same preparation method as in Compound **8-1** to obtain Compound **8-5** as a pale brown solid (10.5 mg, 39%).

¹H NMR (500 MHz, DMSO-d₆) δ 1.16-1.28 (m, 3H), 1.29-1.39 (m, 2H), 1.48-1.62 (m, 3H), 1.85-1.93 (m, 2H), 2.81 (d, J = 4.4 Hz, 3H), 3.06-3.11 (m, 4H), 3.72-3.78 (m, 4H), 3.85 (s, 3H), 3.99-4.06 (m, 1H), 5.53 (d, J = 7.6 Hz, 1H), 6.49 (dd, J = 8.9, 2.5 Hz, 1H), 6.65 (d, J = 2.5 Hz, 1H), 7.43 (s, 1H), 7.63 (t, J = 7.7 Hz, 1H), 7.80 (dt, J = 7.7, 1.3 Hz, 1H), 7.92 (dt, J = 7.8, 1.4 Hz, 1H), 8.07 (d, J = 8.7 Hz, 1H), 8.13-8.16 (m, 1H), 8.56 (q, J = 4.2 Hz, 1H), 13.02 (br s, 1H).

### Example 6. Preparation of N-(3-(4-(cyclohexylamino)-6-((2-methoxy-4-morpholinophenyl)amino)-1H-pyrazolo[3,4-d]pyrimidin-3-yl)phenyl)methanesulfonamide (Compound 8-6)

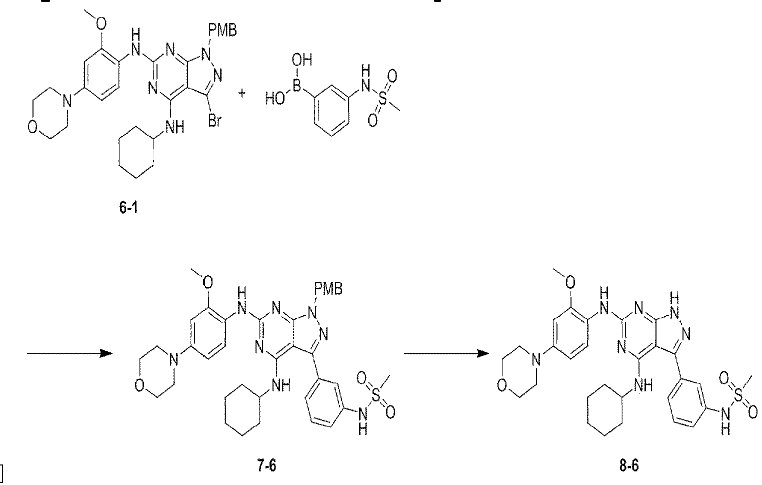

### Step 1. Synthesis of Compound 7-6

Compound **6-1** (50 mg, 0.08 mmol) and (3-(methylsulfonamido)phenyl)boronic acid (26 mg, 0.12 mmol) were subjected to the same preparation method as in Compound **7-1** to obtain Compound **7-6** as a white solid (48.5 mg, 84%).

¹H NMR (400 MHz, DMSO-d₆) δ 1.20-1.39 (m, 5H), 1.51-1.67 (m, 3H), 1.86-1.95 (m, 2H), 3.04 (s, 3H), 3.06-3.11 (m, 4H), 3.70 (s, 3H), 3.73-3.77 (m, 4H), 3.87 (s, 3H), 3.94-4.01 (m, 1H), 5.34 (s, 2H), 5.64 (d, J = 7.5 Hz, 1H), 6.49-6.54 (m, 1H), 6.65-6.68 (m, 1H), 6.89 (d, J = 8.6 Hz, 2H), 7.25 (d, J = 8.8 Hz, 2H), 7.30-7.36 (m, 2H), 7.44-7.51 (m, 3H), 8.17 (d, J = 8.9 Hz, 1H), 9.94 (s, 1H).

### Step 2. Preparation of Compound 8-6

Compound **7-6** (16.5 mg, 0.02 mmol) was subjected to the same preparation method as in Compound **8-1** to obtain Compound **8-6** as a pale brown solid (5.5 mg, 40%).

¹H-NMR (500 MHz, DMSO-d₆) δ 1.25 (q, J = 10.7, 10.1 Hz, 3H), 1.35 (q, J = 11.9, 11.4 Hz, 2H), 1.51-1.65 (m, 3H), 1.88-1.95 (m, 2H), 3.05 (s, 3H), 3.08 (t, J = 4.8 Hz, 4H), 3.75 (t, J = 4.8 Hz, 4H), 3.85 (s, 3H), 3.95-4.04 (m, 1H), 5.54 (d, J = 7.6 Hz, 1H), 6.46-6.51 (m, 1H), 6.65 (d, J = 2.2 Hz, 1H), 7.30 (d, J = 8.2 Hz, 1H), 7.35 (d, J = 7.6 Hz, 1H), 7.42 (s, 1H), 7.46 (s, 1H), 7.50 (t, J = 7.8 Hz, 1H), 8.05 (d, J = 8.7 Hz, 1H), 9.96 (br s, 1H), 12.96 (s, 1H).

### Example 7. Preparation of N⁴-cyclohexyl-N⁶-(2-methoxy-4-morpholinophenyl)-3-(1-methyl-1H-pyrazol-4-yl)-1H-pyrazolo[3,4-d]pyrimidine-4,6-diamine (Compound 8-7)

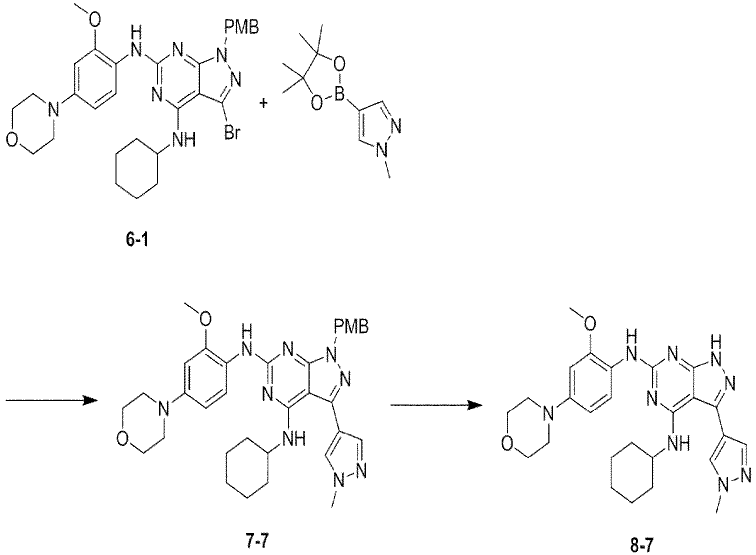

### Step 1. Synthesis of Compound 7-7

Compound **6-1** (30 mg, 0.04 mmol) and 1-methyl-4-(4,4,5,5-tetramethyl-1,3,2-dioxaborolan-2-yl)-1H-pyrazole (15 mg, 0.07 mmol) were subjected to the same preparation method as in Compound **7-1** to obtain Compound **7-7** as a mixture (48.5 mg).

### Step 2. Preparation of Compound 8-7

Compound **7-7** (50.8 mg, 0.08 mmol) was subjected to the same preparation method as in Compound **8-1** to obtain Compound **8-7** as a brown solid (13.6 mg, 33%).

¹H NMR (300 MHz, DMSO-d₆) δ 1.16-1.43 (m, 5H), 1.52-1.73 (m, 3H), 1.90-2.03 (2H), 3.08 (t, J = 4.8 Hz, 4H), 3.75 (t, J = 4.8 Hz, 4H), 3.85 (s, 3H), 3.93 (s, 3H), 3.98-4.04 (m, 1H), 5.61 (d, J = 7.7 Hz, 1H), 6.48 (dd, J = 8.9, 2.5 Hz, 1H), 6.65 (d, J = 2.5 Hz, 1H), 7.39 (s, 1H), 7.71 (s, 1H), 8.04 (s, 1H), 8.08 (d, J = 8.8 Hz, 1H), 12.78 (s, 1H).

### Example 8. Preparation of N⁴-cyclohexyl-N⁶-(2-methoxy-4-morpholinophenyl)-1-(4-methoxybenzyl)-3-(1H-pyrazol-4-yl)-1H-pyrazolo[3,4-d]pyrimidine-4,6-diamine (Compound 8-8)

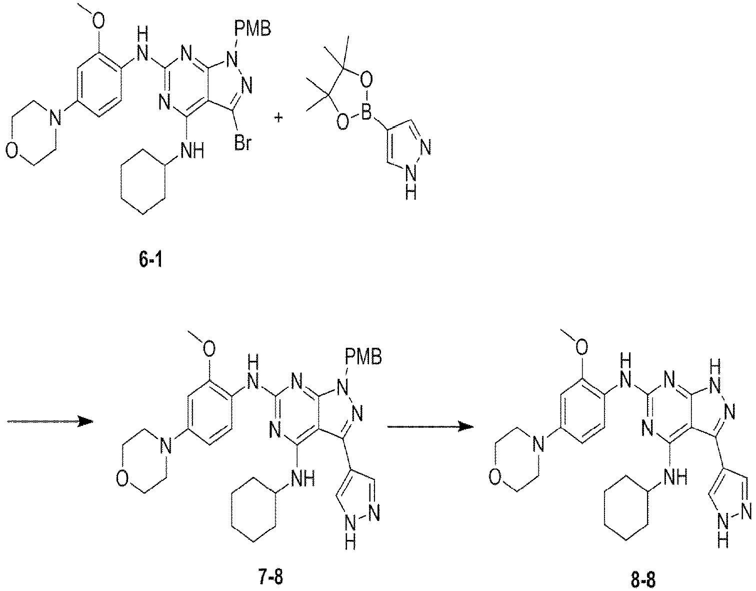

### Step 1. Synthesis of Compound 7-8

Compound **6-1** (500 mg, 0.80 mmol) and 4-(4,4,5,5-tetramethyl-1,3,2-dioxaborolan-2-yl)-1H-pyrazole (234 mg, 1.20 mmol) were subjected to the same preparation method as in Compound **7-1** to obtain Compound **7-8** as a pale brown solid (286.9 mg, 58%).

¹H NMR (500 MHz, DMSO-d₆) δ 1.21-1.39 (m, 5H), 1.52-1.60 (m, 1H), 1.60-1.66 (m, 2H), 1.91-1.97 (m, 2H), 3.04-3.11 (m, 4H), 3.70 (s, 3H), 3.73-3.77 (m, 4H), 3.86 (s, 3H), 3.96-4.01 (m, 1H), 5.29 (s, 2H), 5.66 (d, J = 7.6 Hz, 1H), 6.50 (dd, J = 8.9, 2.6 Hz, 1H), 6.66 (d, J = 2.6 Hz, 1H), 6.88 (d, J = 8.7 Hz, 2H), 7.23 (d, J = 8.7 Hz, 2H), 7.45 (s, 1H), 7.78 (br s, 1H), 8.08 (br s, 1H), 8.20 (d, J = 8.8 Hz, 1H), 13.22 (s, br 1H).

### Step 2. Preparation of Compound 8-8

Compound **7-8** (75.1 mg, 0.12 mmol) was subjected to the same preparation method as in Compound **8-1** to obtain Compound **8-8** as a pale brown solid (28.1 mg, 46%).

¹H NMR (500 MHz, DMSO-d₆) δ 1.16-1.32 (m, 3H), 1.32-1.42 (m, 2H), 1.54-1.68 (m, 3H), 1.90-2.02 (m, 2H), 3.08 (t, J = 4.7 Hz, 4H), 3.75 (t, J = 4.7 Hz, 4H), 3.85 (s, 3H), 3.95-4.06 (m, 1H), 5.55 (d, J = 7.6 Hz, 1H), 6.48 (dd, J = 8.8, 2.5 Hz, 1H), 6.65 (d, J = 2.5 Hz, 1H), 7.37 (s, 1H), 7.82 (br s, 1H), 8.09 (d, J = 8.8 Hz, 1H), 12.77 (s, 1H), 13.20 (br s, 1H).

### Example 9. Preparation of N⁴-cyclohexyl-N⁶-(4-morpholinocyclohexyl)-3-(pyridin-3-yl)-1H-pyrazolo[3,4-d]pyrimidine-4,6-diamine (Compound 8-9)

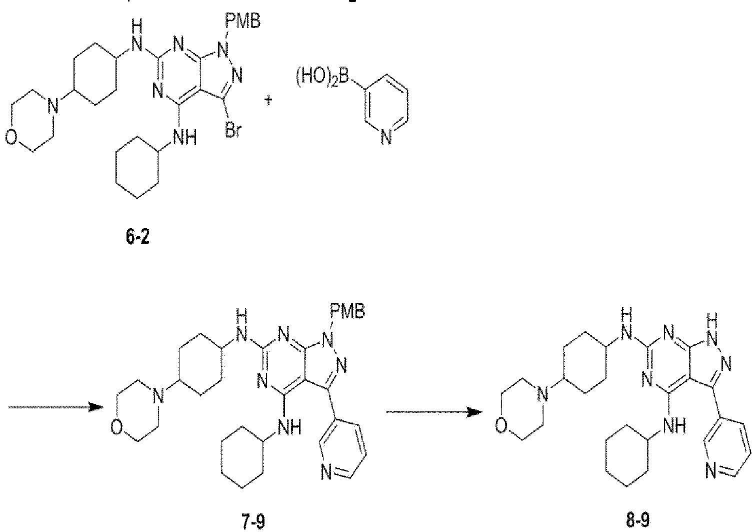

### Step 1. Synthesis of Compound 7-9

Compound **6-2** (30 mg, 0.05 mmol) and pyridin-3-ylboronic acid (10 mg, 0.07 mmol) were subjected to the same preparation method as in Compound **7-1** to obtain Compound **7-9** as a mixture (34.0 mg).

### Step 2. Preparation of Compound 8-9

Compound **7-9** (34.0 mg, 0.05 mmol) was subjected to the same preparation method as in Compound **8-1** in step 2 of Example 1 to obtain Compound **8-9** as a white solid (7.1 mg, 26%).

¹H NMR (400 MHz, DMSO-d₆) δ 1.07-1.41 (m, 9H), 1.45-1.73 (m, 3H), 1.77-2.07 (m, 6H), 2.09-2.27 (m, 1H), 2.39-2.50 (m, 4H), 3.49-3.62 (m, 4H), 3.65 (s, 1H), 4.02 (s, 1H), 5.23-5.69 (m, 1H), 6.50 (d, J = 6.5 Hz, 1H), 7.53 (s, 1H), 8.00 (d, J = 6.5 Hz, 1H), 8.63 (s, 1H), 8.81 (s, 1H), 12.65-13.14 (m, 1H).

### Example 10. Preparation of N⁴-cyclohexyl-N⁶-(2-methoxy-4-morpholinophenyl)-3-(1-methyl-1H-pyrazol-5-yl)-1H-pyrazolo[3,4-d]pyrimidine-4,6-diamine (Compound 8-10)

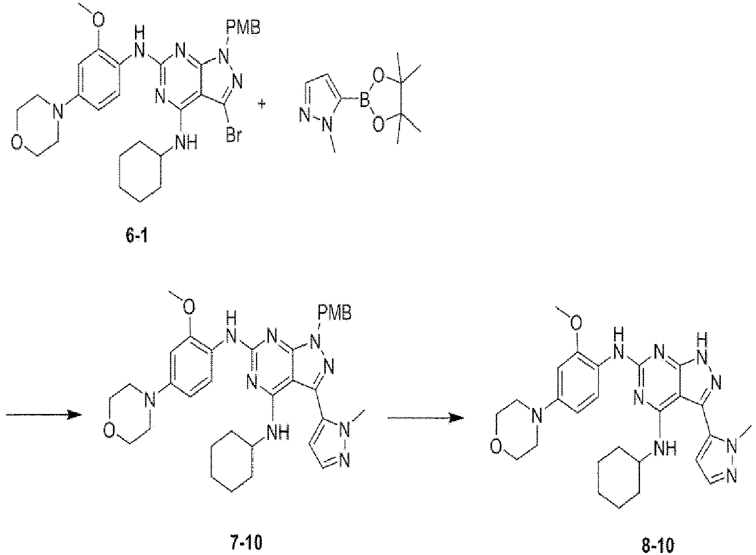

### Step 1. Synthesis of Compound 7-10

Compound **6-1** (50 mg, 0.08 mmol) and 1-methylpyrazole-5-boronic acid (20 mg, 0.1043 mmol) were subjected to the same preparation method as in Compound **7-1** to obtain Compound **7-10** as a white solid (40 mg, 80%) .

¹H NMR (300 MHz, DMSO-d₆) δ 0.86 (d, J = 2.1 Hz, 1H), 1.24 (s, 10H), 1.34 (s, 1H), 1.58 (d, J = 22.3 Hz, 3H), 3.09 (t, J = 4.9 Hz, 5H), 3.71 (s, 3H), 3.75 (t, J = 4.8 Hz, 5H), 3.88 (d, J = 13.6 Hz, 6H), 5.36 (s, 2H), 5.74 (d, J = 7.5 Hz, 1H), 6.49 - 6.54 (m, 1H), 6.60 (d, J = 1.9 Hz, 1H), 6.67 (d, J = 2.6 Hz, 1H), 6.90 (d, J = 8.5 Hz, 2H), 7.26 (d, J = 8.6 Hz, 2H), 7.55 - 7.66 (m, 6H), 8.15 (d, J = 8.7 Hz, 1H).

### Step 2. Preparation of Compound 8-10

Compound **7-10** (40 mg, 0.0641mmol) was subjected to the same preparation method as in Compound **8-1** in step 2 of Example 1 to obtain Compound **8-10** as a white solid (18.8 mg, 58.7%).

¹H NMR (400 MHz, DMSO-d₆) δ 1.35 (d, J = 11.6 Hz, 2H), 1.61 (s, 3H), 1.88 - 1.99 (m, 3H), 3.06 - 3.10 (m, 4H), 3.73 - 3.76 (m, 4H), 3.84 (s, 3H), 3.89 - 3.92 (m, 3H), 4.00 (s, 1H), 5.62 (d, J = 7.7 Hz, 1H), 6.48 (dd, J = 8.8, 2.6 Hz, 1H), 6.58 (d, J = 2.2 Hz, 1H), 6.65 (d, J = 2.7 Hz, 1H), 7.48 (s, 1H), 7.62 (d, J = 2.0 Hz, 1H), 8.01 (d, J = 8.8 Hz, 1H), 13.20 (s, 1H).

### Example 11. Preparation of N⁴-cyclohexyl-3-(1-isopropyl-1H-imidazol-4-yl)-N⁶-(2-methoxy-4-morpholinophenyl)-1H-pyrazolo[3,4-d]pyrimidine-4,6-diamine (Compound 8-11)

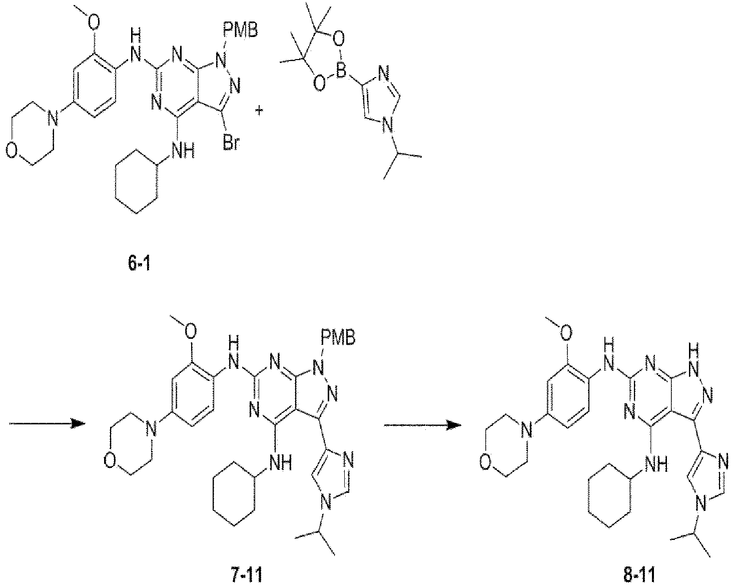

### Step 1. Synthesis of Compound 7-11

Compound **6-1** (50 mg, 0.08 mmol) and 1-isopropyl-4-(4,4,5,5-tetramethyl-1,3,2-dioxaborolan-2-yl)-1H-imidazole (113 mg, 0.4818 mmol) were subjected to the same preparation method as in Compound **7-1** to obtain Compound **7-11** as a white solid (98 mg, 94.2%).

¹H NMR (300 MHz, DMSO-d₆) δ 1.23 (d, J = 2.8 Hz, 3H), 1.35 (d, J = 5.7 Hz, 2H), 1.46 (d, J = 6.7 Hz, 9H), 1.78 (s, 3H), 1.99 (s, 3H), 3.08 (t, J = 4.8 Hz, 4H), 3.70 (s, 3H), 3.75 (t, J = 4.8 Hz, 4H), 3.87 (s, 3H), 4.12 (s, 2H), 4.48 (q, J = 6.7 Hz, 1H), 5.29 (s, 2H), 6.48 - 6.52 (m, 1H), 6.66 (d, J = 2.5 Hz, 1H), 6.88 (d, J = 8.7 Hz, 2H), 7.19 (d, J = 8.5 Hz, 2H), 7.31 (s, 1H), 7.60 (t, J = 6.7 Hz, 1H), 7.76 (d, J = 1.4 Hz, 1H), 8.00 (d, J = 1.4 Hz, 1H), 8.30 (d, J = 8.8 Hz, 1H), 10.76 (d, J = 7.4 Hz, 1H).

### Step 2. Preparation of Compound 8-11

Compound **7-11** (95 mg, 0.1457mmol) was subjected to the same preparation method as in Compound **8-1** in step 2 of Example 1 to obtain Compound **8-11** as a white solid (23.5 mg, 30%).

¹H NMR (400 MHz, DMSO-d₆) δ 1.46 (dt, J = 8.4, 4.2 Hz, 9H), 1.58 (s, 2H), 1.77 (s, 3H), 1.99 (d, J = 2.0 Hz, 3H), 3.07 (dd, J = 6.9, 3.4 Hz, 4H), 3.74 (d, J = 6.0 Hz, 4H), 3.84 - 3.87 (m, 3H), 4.11 (s, 1H), 4.47 - 4.53 (m, 1H), 6.47 (d, J = 8.6 Hz, 1H), 6.64 (d, J = 2.9 Hz, 1H), 7.22 (s, 1H), 7.70 - 7.74 (m, 1H), 7.98 (d, J = 1.7 Hz, 1H), 8.19 (d, J = 8.4 Hz, 1H), 10.65 (d, J = 7.5 Hz, 1H), 12.59 (s, 1H).

### Example 12. Preparation of N⁴-cyclohexyl-3-(1-(difluoromethyl)-1H-pyrazol-4-yl)-N⁶-(2-methoxy-4-morpholinophenyl)-1H-pyrazolo[3,4-d]pyrimidine-4,6-diamine (Compound 8-12)

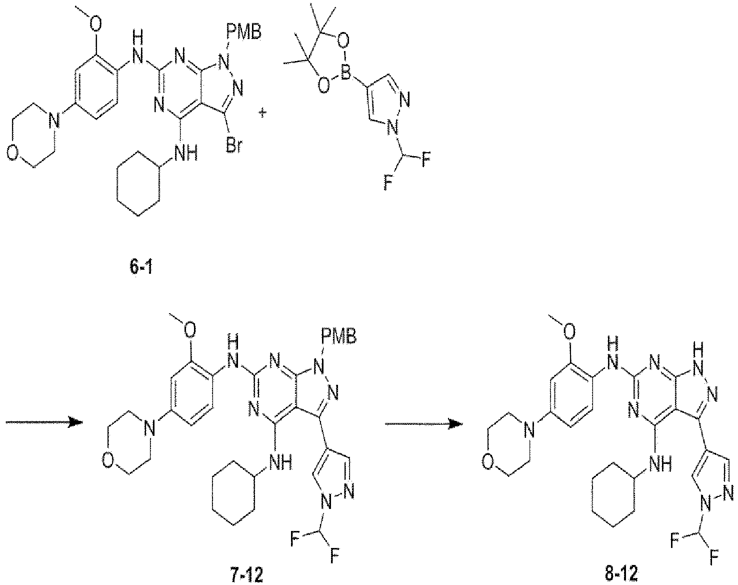

### Step 1. Synthesis of Compound 7-12

Compound **6-1** (50 mg, 0.08 mmol) and 1-(difluoromethyl)-4-(4,4,5,5-tetramethyl-1,3,2-dioxaborolan-2-yl)-1H-pyrazole (25 mg, 0.12 mmol) were subjected to the same preparation method as in Compound **7-1** to obtain Compound **7-12** as a white solid (55 mg, 99%) .

¹H-NMR (400 MHz, DMSO-d₆) δ 9.62 (d, J= 7.2 Hz, 1H), 8.25 (d, J= 8.9 Hz, 1H), 7.85 (d, J= 2.4 Hz, 1H), 7.66 - 7.54 (m, 6H), 7.38 (s, 1H), 7.21 (d, J= 8.4 Hz, 2H), 6.88 (d, J= 8.5 Hz, 2H), 6.66 (d, J= 2.3 Hz, 2H), 6.51 (d, J= 8.0 Hz, 2H), 5.32 (s, 2H), 3.96 (s, 3H), 3.87 (s, 3H), 3.75 (t, J= 4.8 Hz, 4H), 3.70 (s, 3H), 3.11 - 3.06 (m, 4H), 2.08 (s, 3H), 1.88 - 1.19 (m, 21H).

### Step 2. Preparation of Compound 8-12

Compound **7-12** (55 mg, 0.0833 mmol) was subjected to the same preparation method as in Compound **8-1** in step 2 of Example 1 to obtain Compound **8-12** as a pale brown solid (24 mg, 54%).

¹H NMR (300 MHz, DMSO-d₆) δ 1.34 (q, J = 10.6, 9.7 Hz, 6H), 1.66 (s, 3H), 1.96 (d, J = 10.0 Hz, 2H), 3.09 (t, J = 4.8 Hz, 5H), 3.76 (t, J = 4.8 Hz, 5H), 5.76 (d, J = 7.6 Hz, 1H), 6.49 (dd, J = 8.8, 2.5 Hz, 1H), 6.66 (d, J = 2.5 Hz, 1H), 7.42 (s, 1H), 8.03 - 8.12 (m, 3H), 8.55 (s, 1H), 12.94 (s, 1H).

### Example 13. Preparation of N⁴-cyclohexyl-N⁶-(2-methoxy-4-morpholinophenyl)-3-(1-methyl-1H-pyrazol-3-yl)-1H-pyrazolo[3,4-d]pyrimidine-4,6-diamine (Compound 8-13)

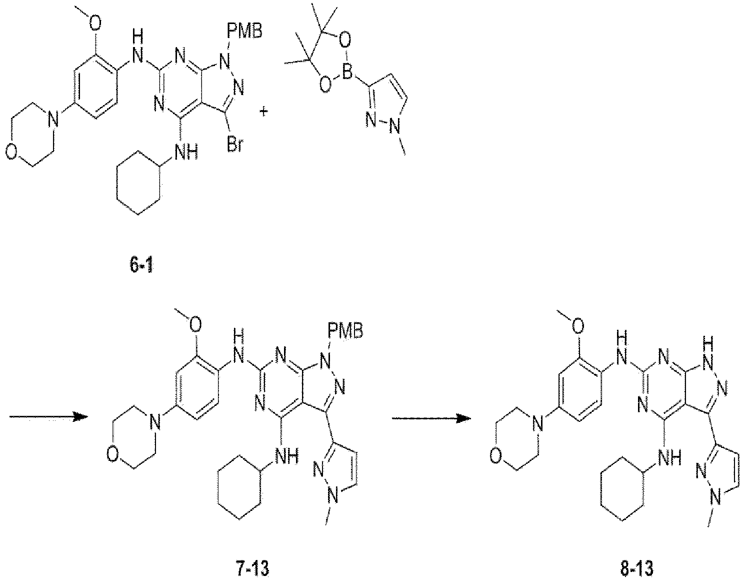

### Step 1. Synthesis of Compound 7-13

Compound **6-1** (50 mg, 0.08 mmol) and 1-methyl-3-(4,4,5,5-tetramethyl-1,3,2-dioxaborolan-2-yl)-1H-pyrazole (20 mg, 0.1043 mmol) were subjected to the same preparation method as in Compound **7-1** to obtain Compound **7-13** as a white solid (40 mg, 80%).

¹H NMR (300 MHz, DMSO-d₆) δ 0.86 (d, J = 2.1 Hz, 1H), 1.24 (s, 10H), 1.34 (s, 1H), 1.58 (d, J = 22.3 Hz, 3H), 3.09 (t, J = 4.9 Hz, 5H), 3.71 (s, 3H), 3.75 (t, J = 4.8 Hz, 5H), 3.88 (d, J = 13.6 Hz, 6H), 5.36 (s, 2H), 5.74 (d, J = 7.5 Hz, 1H), 6.49 - 6.54 (m, 1H), 6.60 (d, J = 1.9 Hz, 1H), 6.67 (d, J = 2.6 Hz, 1H), 6.90 (d, J = 8.5 Hz, 2H), 7.26 (d, J = 8.6 Hz, 2H), 7.55 - 7.66 (m, 6H), 8.15 (d, J = 8.7 Hz, 1H).

### Step 2. Preparation of Compound 8-13

Compound **7-13** (55 mg, 0.0881 mmol) was subjected to the same preparation method as in Compound **8-1** in step 2 of Example 1 to obtain Compound **8-13** as a white solid (23.2 mg, 52%).

¹H NMR (300 MHz, DMSO-d₆) δ 1.44 (q, J = 9.4, 8.9 Hz, 6H), 1.62 (s, 1H), 1.77 (s, 3H), 2.01 - 2.11 (m, 3H), 3.08 (t, J = 4.8 Hz, 4H), 3.72 - 3.78 (m, 4H), 3.85 (s, 3H), 3.96 (s, 3H), 6.48 (dd, J = 8.8, 2.5 Hz, 1H), 6.66 (dd, J = 10.2, 2.4 Hz, 2H), 7.29 (s, 1H), 7.84 (d, J = 2.3 Hz, 1H), 8.14 (d, J = 8.7 Hz, 1H), 9.53 (d, J = 7.3 Hz, 1H), 12.80 (s, 1H).

### Example 14. Preparation of N⁴-cyclohexyl-3-(furan-2-yl)-N⁶-(2-methoxy-4-morpholinophenyl)-1H-pyrazolo[3,4-d]pyrimidine-4,6-diamine (Compound 8-14)

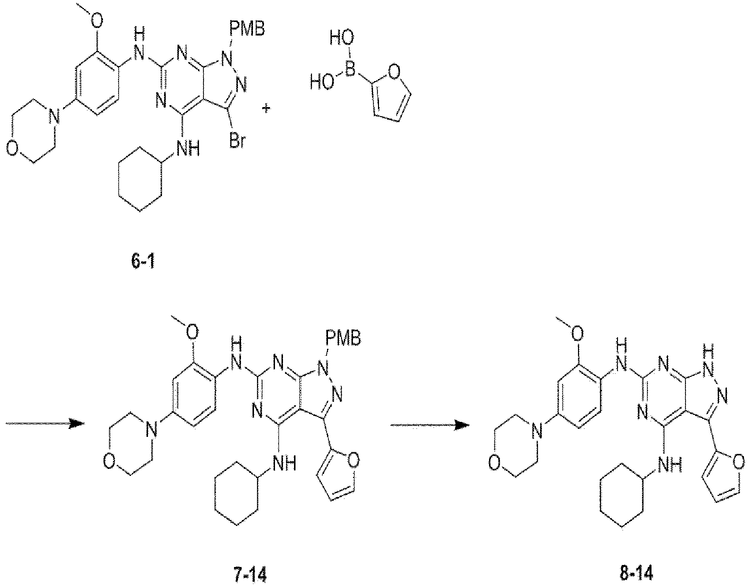

### Step 1. Synthesis of Compound 7-14

Compound **6-1** (50 mg, 0.08 mmol) and furan-2-ylboronic acid (12 mg, 0.1043 mmol) were subjected to the same preparation method as in Compound **7-1** to obtain Compound **7-14** as a white solid (14 mg, 28%).

¹H NMR (300 MHz, DMSO-d₆) δ 1.23 (d, J = 2.9 Hz, 11H), 1.35 (d, J = 5.7 Hz, 7H), 1.74 (d, J = 11.6 Hz, 3H), 3.07 - 3.11 (m, 4H), 3.70 (s, 3H), 3.75 (t, J = 4.8 Hz, 5H), 3.86 (s, 3H), 5.32 (s, 2H), 6.48 - 6.53 (m, 1H), 6.64 - 6.73 (m, 3H), 6.88 (d, J = 7.9 Hz, 3H), 7.09 - 7.13 (m, 1H), 7.22 (d, J = 8.6 Hz, 2H), 7.52 (s, 1H), 7.96 - 7.99 (m, 1H), 8.15 (d, J = 8.8 Hz, 1H), 9.05 (s, 1H) .

### Step 2. Preparation of Compound 8-14

Compound **7-14** (14 mg, 0.0229mmol) was subjected to the same preparation method as in Compound **8-1** in step 2 of Example 1 to obtain Compound **8-14** as a pale brown solid (4.7 mg, 42.7%).

¹H NMR (400 MHz, DMSO-d₆) δ 1.27 - 1.49 (m, 8H), 1.61 (s, 2H), 1.68 - 1.79 (m, 3H), 1.95 - 2.08 (m, 3H), 3.07 - 3.11 (m, 4H), 3.74 - 3.77 (m, 4H), 3.85 (d, J = 1.4 Hz, 3H), 4.10 (s, 1H), 6.49 (dd, J = 8.6, 2.2 Hz, 1H), 6.65 (d, J = 2.0 Hz, 1H), 6.71 (dt, J = 3.3, 1.6 Hz, 1H), 6.91 (d, J = 3.4 Hz, 1H), 7.05 (d, J = 7.5 Hz, 1H), 7.43 (s, 1H), 7.96 (d, J = 1.8 Hz, 1H), 8.05 (dd, J = 8.7, 1.3 Hz, 1H), 13.04 (s, 1H).

### Example 15. Preparation of N⁴-cyclohexyl-N⁶-(2-methoxy-4-morpholinophenyl)-3-(thiazol-5-yl)-1H-pyrazolo[3,4-d]pyrimidine-4,6-diamine (Compound 8-15)

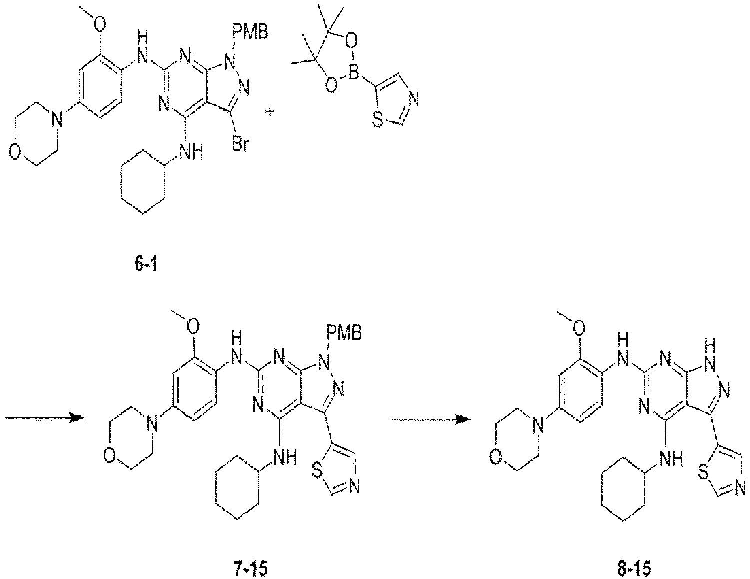

### Step 1. Synthesis of Compound 7-15

Compound **6-1** (50 mg, 0.08 mmol) and 5-(4,4,5,5-tetramethyl-1,3,2-dioxaborolan-2-yl)thiazole (42 mg, 0.2007 mmol) were subjected to the same preparation method as in Compound **7-1** to obtain Compound **7-15** as a mixture (17.6 mg)

### Step 2. Preparation of Compound 8-15

Compound **7-15** (17.6 mg, 0.0287mmol) was subjected to the same preparation method as in Compound **8-1** to obtain Compound **8-15** as a white solid (11.4 mg, 80.0%).

¹H NMR (400 MHz, DMSO-d₆) δ 1.35 (d, J = 8.0 Hz, 5H), 1.59 (d, J = 12.9 Hz, 2H), 1.68 (s, 3H), 1.99 (d, J = 6.6 Hz, 3H), 3.09 (t, J = 4.8 Hz, 4H), 3.76 (d, J = 4.3 Hz, 4H), 3.85 (s, 3H), 4.03 (d, J = 7.0 Hz, 1H), 6.01 (d, J = 7.7 Hz, 1H), 6.49 (dd, J = 8.8, 2.6 Hz, 1H), 6.65 (d, J = 2.6 Hz, 1H), 7.49 (s, 1H), 8.01 (d, J = 8.8 Hz, 1H), 8.17 (s, 1H), 9.19 (s, 1H), 13.15 (s, 1H) .

### Example 16. Preparation of 3-(1H-benzo[d]imidazol-5-yl)-N⁴-cyclohexyl-N⁶-(2-methoxy-4-morpholinophenyl)-1H-pyrazolo[3,4-d]pyrimidine-4,6-diamine (Compound 8-16)

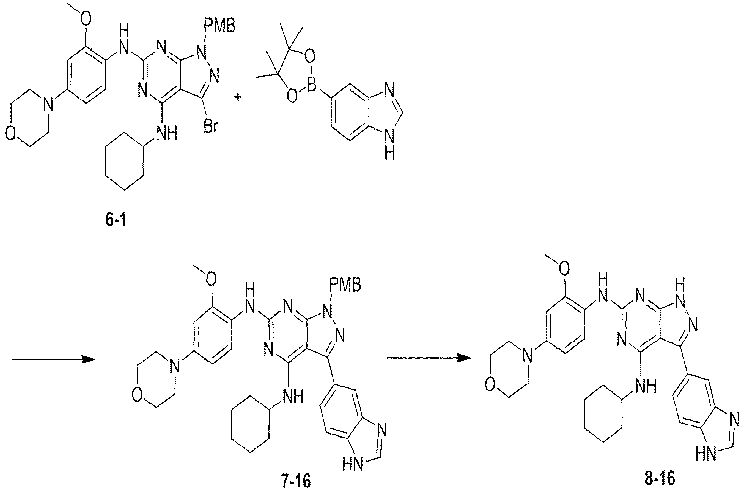

### Step 1. Synthesis of Compound 7-16

Compound **6-1** (50 mg, 0.08 mmol) and 1H-benzimidazole-5-boronic acid (25 mg, 0.1043 mmol) were subjected to the same preparation method as in Compound **7-1** to obtain Compound **7-16** as a pale brown solid (14.5 mg, 27.8%).

¹H NMR (300 MHz, DMSO-d₆) δ 1.23 (d, J = 2.0 Hz, 9H), 1.33 - 1.40 (m, 3H), 1.51 - 1.60 (m, 4H), 1.91 (s, 4H), 3.71 (s, 4H), 3.79 (d, J = 11.9 Hz, 4H), 3.88 (s, 1H), 3.97 (s, 3H), 4.08 (t, J = 10.3 Hz, 4H), 4.44 (t, J = 11.3 Hz, 3H), 5.41 (s, 2H), 5.76 (s, 1H), 6.91 (d, J = 8.7 Hz, 2H), 7.29 - 7.33 (m, 2H), 7.49 (s, 1H), 7.71 (s, 2H), 7.97 (d, J = 2.4 Hz, 1H), 8.33 (s, 1H), 8.62 (d, J = 9.0 Hz, 1H), 12.71 (d, J = 18.7 Hz, 1H).

### Step 2. Preparation of Compound 8-16

Compound **7-16** (14.5 mg, 0.0219mmol) was subjected to the same preparation method as in Compound **8-1** to obtain Compound **8-16** as a pale brown solid (1.3 mg, 11.8%).

¹H NMR (300 MHz, Methanol-d₄) δ 1.44 (d, J = 9.8 Hz, 4H), 1.62 (s, 4H), 2.03 (s, 3H), 3.14 (t, J = 4.8 Hz, 3H), 3.83 - 3.90 (m, 3H), 4.03 - 4.20 (m, 2H), 6.57 (dd, J = 8.9, 2.6 Hz, 1H), 6.70 (d, J = 2.5 Hz, 1H), 7.60 (d, J = 8.4 Hz, 1H), 7.88 (d, J = 25.9 Hz, 2H), 8.34 (t, J = 4.4 Hz, 2H).

### Example 17. Preparation of N⁴-cyclohexyl-3-(1H-indazol-5-yl)-N⁶-(2-methoxy-4-morpholinophenyl) -1H-pyrazolo[3,4-d]pyrimidine-4,6-diamine (Compound 8-17)

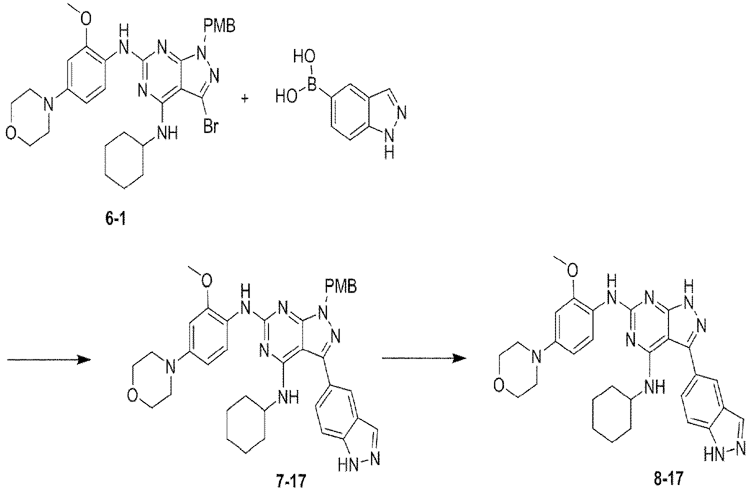

### Step 1. Synthesis of Compound 7-17

Compound **6-1** (50 mg, 0.08 mmol) and 1H-Indazole-5-boronic acid (17 mg, 0.1043 mmol) were subjected to the same preparation method as in Compound **7-1** to obtain Compound **7-17** as a white solid (49 mg, 94.2%).

¹H NMR (300 MHz, DMSO-d₆) δ 1.21 (d, J = 17.6 Hz, 9H), 1.34 (s, 2H), 1.54 (s, 3H), 1.91 (s, 3H), 3.10 (t, J = 4.7 Hz, 4H), 3.71 (d, J = 0.9 Hz, 3H), 3.76 (t, J = 4.5 Hz, 4H), 3.88 (s, 3H), 4.03 (d, J = 8.1 Hz, 1H), 5.35 (s, 2H), 5.63 (d, J = 7.6 Hz, 1H), 6.52 (d, J = 8.9 Hz, 1H), 6.68 (s, 1H), 6.90 (d, J = 8.4 Hz, 2H), 7.29 (d, J = 8.4 Hz, 2H), 7.49 (s, 1H), 7.62 (d, J = 8.6 Hz, 1H), 7.70 (d, J = 8.6 Hz, 1H), 8.02 (s, 1H), 8.18 (d, J = 1.4 Hz, 1H), 8.22 (d, J = 8.8 Hz, 1H), 13.27 (s, 1H).

### Step 2. Preparation of Compound 8-17

Compound **7-17** (49 mg, 0.0742mmol) was subjected to the same preparation method as in Compound **8-1** to obtain Compound **8-17** as a white solid (11.2 mg, 28%).

¹H NMR (400 MHz, DMSO-d₆) δ 1.04 - 1.42 (m, 10H), 1.53 (s, 3H), 1.91 (s, 3H), 3.09 (t, J = 4.8 Hz, 4H), 3.75 (t, J = 4.7 Hz, 4H), 3.86 (s, 3H), 4.03 (s, 1H), 5.51 (d, J = 7.6 Hz, 1H), 6.49 (dd, J = 8.8, 2.5 Hz, 1H), 6.66 (d, J = 2.5 Hz, 1H), 7.41 (s, 1H), 7.63 (dd, J = 8.4, 1.5 Hz, 1H), 7.71 (d, J = 8.6 Hz, 1H), 8.01 (s, 1H), 8.09 (d, J = 8.9 Hz, 1H), 8.18 (s, 1H), 12.89 (s, 1H), 13.26 (s, 1H).

### Example 18. Preparation of N⁴-cyclohexyl-N⁶-(2-methoxy-4-morpholinophenyl)-3-(2-methylbenzo[d]oxazol-6-yl)-1H-pyrazolo[3,4-d]pyrimidine-4,6-diamine (Compound 8-18)

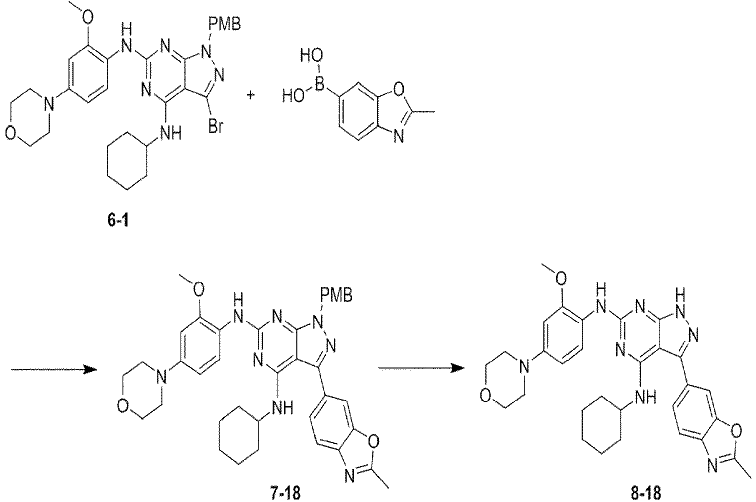

### Step 1. Synthesis of Compound 7-18

Compound **6-1** (50 mg, 0.08 mmol) and (2-Methyl-1,3-benzoxazol-6-yl) boronic acid (18.5 mg, 0.1043 mmol) were subjected to the same preparation method as in Compound **7-1** to obtain Compound **7-18** as a white solid (51 mg, 94.4%).

¹H NMR (300 MHz, DMSO-d₆) δ 1.10 - 1.42 (m, 10H), 1.58 (s, 4H), 1.89 (s, 3H), 2.67 (s, 3H), 3.10 (t, J = 4.8 Hz, 4H), 3.71 (s, 3H), 3.76 (t, J = 4.8 Hz, 4H), 3.87 (s, 3H), 4.03 (d, J = 6.7 Hz, 1H), 5.36 (s, 2H), 5.75 (d, J = 7.8 Hz, 1H), 6.52 (dd, J = 8.9, 2.5 Hz, 1H), 6.67 (d, J = 2.5 Hz, 1H), 6.89 (d, J = 8.7 Hz, 2H), 7.28 (d, J = 8.6 Hz, 2H), 7.51 (s, 1H), 7.55 - 7.66 (m, 4H), 7.80 (d, J = 8.2 Hz, 1H), 7.87 - 7.88 (m, 1H), 8.20 (d, J = 8.8 Hz, 1H).

### Step 2. Preparation of Compound 8-18

Compound **7-18** (51 mg, 0.0755mmol) was subjected to the same preparation method as in Compound **8-1** to obtain Compound **8-18** as a white solid (21.6 mg, 51.4%).

¹H NMR (400 MHz, DMSO-d₆) δ 1.11 - 1.42 (m, 8H), 1.55 (d, J = 22.4 Hz, 3H), 1.91 (d, J = 11.2 Hz, 3H), 2.67 (d, J = 3.6 Hz, 3H), 3.09 (dt, J = 6.8, 3.8 Hz, 4H), 3.76 (dt, J = 6.6, 3.7 Hz, 4H), 3.86 (d, J = 3.6 Hz, 3H), 4.01 - 4.06 (m, 1H), 5.62 (d, J = 7.7 Hz, 1H), 6.50 (dt, J = 9.1, 3.1 Hz, 1H), 6.66 (t, J = 3.0 Hz, 1H), 7.43 (d, J = 3.3 Hz, 1H), 7.62 (dq, J = 8.2, 1.6 Hz, 1H), 7.81 (dd, J = 8.2, 3.5 Hz, 1H), 7.89 (q, J = 1.5 Hz, 1H), 8.08 (dd, J = 8.9, 3.4 Hz, 1H), 12.99 (d, J = 3.2 Hz, 1H).

### Example 19. Preparation of N⁴-cyclohexyl-N⁶-(2-methoxy-4-morpholinophenyl)-3-(1-methyl-1H-imidazol-4-yl)-1H-pyrazolo[3,4-d]pyrimidine-4,6-diamine (Compound 8-19)

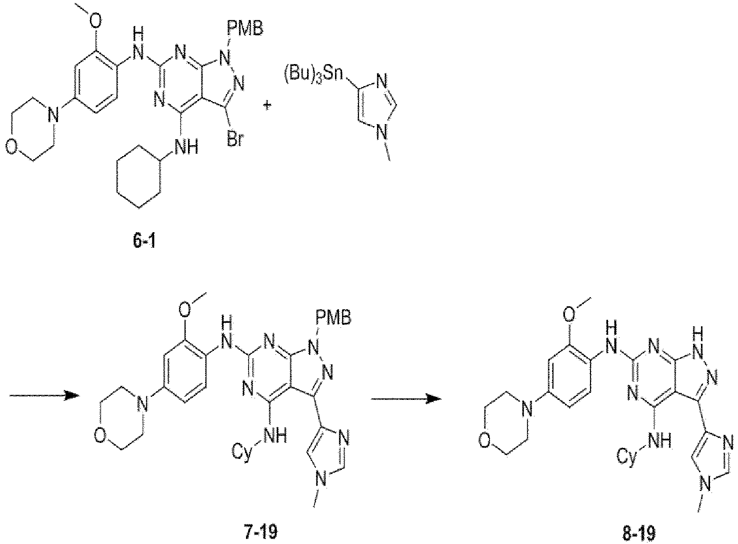

### Step 1. Synthesis of Compound 7-19

Compound **6-1** (60 mg, 0.096 mmol) was dissolved in DMF (1 mL) and then purged with argon gas to remove oxygen. Thereafter, 1-methyl-4-(tributylstannyl)-1H-imidazole (56.9 mg, 0.153 mmol), Pd(PPh₃)₄ (5.54 mg, 0.0048 mmol), and lithium chloride (LiCl; 2 mg, 0.048 mmol) were added, and the mixture was stirred at 120°C for 40 minutes in a Biotage microwave reactor. After completion of the reaction, 1 M potassium fluoride (KF; 5 mL) was added, and then the mixture was stirred at room temperature for 30 minutes and subjected to extraction with EA (20 mL X 2). The organic layer was washed with water (5 mL X 2), subjected to moisture removal (Na₂SO₄), and concentrated under reduced pressure. The residue was separated by silica gel column chromatography (EA:Hx=3:1) to obtain compound **7-19** as a white solid (57.1 mg, 95.4%).

¹H NMR (500 MHz, DMSO) δ 10.66 (d, J = 7.7 Hz, 1H), 8.30 (d, J = 8.8 Hz, 1H), 7.86 (d, J = 1.5 Hz, 1H), 7.61 (d, J = 1.4 Hz, 1H), 7.31 (s, 1H), 7.23 - 7.12 (m, 2H), 6.92 - 6.83 (m, 2H), 6.65 (d, J = 2.6 Hz, 1H), 6.50 (dd, J = 8.9, 2.7 Hz, 1H), 5.28 (s, 2H), 4.12 (s, 1H), 3.87 (s, 3H), 3.78 - 3.72 (m, 4H), 3.71 (d, J = 11.2 Hz, 6H), 3.11 - 3.03 (m, 4H), 1.97 (d, J = 13.4 Hz, 2H), 1.76 (s, 2H), 1.57 (s, 1H), 1.50 - 1.33 (m, 5H), 1.26 (d, J = 16.3 Hz, 2H).

### Step 2. Preparation of Compound 8-19

Compound **7-19** (47 mg, 0.075 mmol) was subjected to the same preparation method as in Compound **8-1** to obtain Compound **8-19** as a yellow solid (27.0 mg, 71.8%).

¹H NMR (400 MHz, DMSO) δ 12.63 (s, 1H), 10.65 (s, 1H), 8.17 (d, J = 7.8 Hz, 1H), 7.87 (s, 1H), 7.61 (s, 1H), 7.31 (s, 1H), 6.66 (s, 1H), 6.49 (d, J = 9.4 Hz, 1H), 4.13 (s, 1H), 3.97 - 3.58 (m, 10H), 3.08 (d, J = 4.2 Hz, 4H), 1.96 (t, J = 6.7 Hz, 2H), 1.78 (d, J = 5.9 Hz, 2H), 1.58 (t, J = 7.9 Hz, 1H), 1.45 (t, J = 8.8 Hz, 5H) .

### Example 20. Preparation of N⁴-cyclohexyl-3-(isoxazol-4-yl)-N⁶-(2-methoxy-4-morpholinophenyl)-1H-pyrazolo[3,4-d]pyrimidine-4,6-diamine(Compound 8-20)

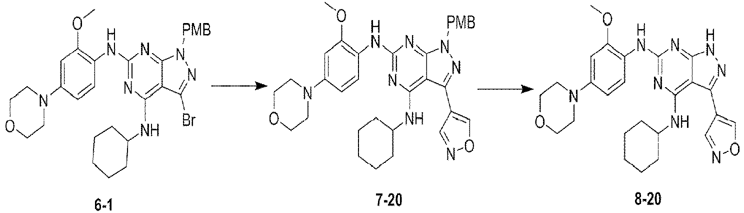

### Step 1. Synthesis of Compound 7-20

Compound **6-1** (100 mg, 0.161mmol) and 4-(4,4,5,5-tetramethyl-1,3,2-dioxaborolan-2-yl)isoxazole (63 mg, 0.321mmol) were subjected to the same preparation method as in Compound **7-1** to obtain Compound **7-20** (48 mg, 49%).

¹H NMR (500 MHz, Chloroform-d) δ 1.45 (s, 3H), 1.66 (s, 1H), 1.79 (s, 2H), 2.14 (d, J = 8.8 Hz, 2H), 3.13 (s, 4H), 3.76 (s, 3H), 3.84 - 3.95 (m, 9H), 4.16 - 4.25 (m, 1H), 5.23 - 5.37 (m, 3H), 6.55 (s, 2H), 6.84 (d, J = 8.6 Hz, 2H), 7.28 - 7.32 (m, 2H), 7.42 (s, 1H), 7.44 - 7.50 (m, 1H), 7.55 (d, J = 1.8 Hz, 1H), 7.67 (dd, J = 11.6, 7.6 Hz, 1H), 8.50 (d, J = 8.7 Hz, 1H).

### Step 2. Synthesis of Compound 8-20

Compound **7-20** (30 mg, 0.0491mmol) was subjected to the same preparation method as in Compound **8-1** to obtain Compound **8-20** (24 mg, 99%).

¹H NMR (500 MHz, DMSO-d₆) δ 1.23 (s, 1H), 1.36 (d, J = 10.6 Hz, 4H), 1.60 (s, 1H), 1.75 (s, 2H), 1.86 (s, 1H), 3.07 (s, 4H), 3.59 (s, 4H), 3.75 (s, 5H), 3.84 (s, 4H), 6.46 (d, J = 10.0 Hz, 1H), 6.64 (d, J = 2.6 Hz, 1H), 7.24 (s, 1H), 7.37 (s, 1H), 8.02 (s, 1H), 8.13 (d, J = 8.7 Hz, 1H), 8.62 (d, J = 7.2 Hz, 1H), 12.52 (s, 1H) .

### Example 21. Preparation of 3-(Isoxazol-4-yl)-N⁶-(2-methoxy-4-morpholinophenyl)-N⁴-(tetrahydro-2H-pyran-4-yl)-1H-pyrazolo[3,4-d]pyrimidine-4,6-diamine(Compound 8-21)

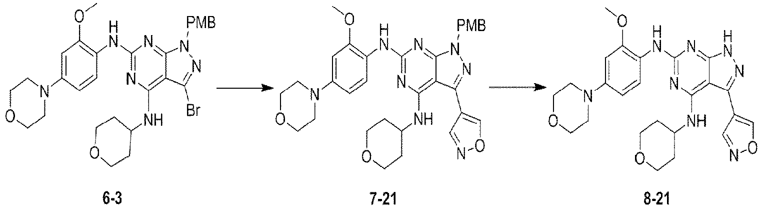

### Step 1. Preparation of Compound 7-21

Compound **6-3** (100 mg, 0.160mmol) and 4-(4,4,5,5-tetramethyl-1,3,2-dioxaborolan-2-yl)isoxazole (62 mg, 0.320mmol) were subjected to the same preparation method as in Compound **7-1** to obtain Compound **7-21** (57 mg, 58%).

¹H NMR (500 MHz, Chloroform-d) δ 1.66 (d, J = 7.6 Hz, 2H), 2.08 - 2.16 (m, 3H), 3.14 (s, 4H), 3.59 (d, J = 2.2 Hz, 2H), 3.76 (s, 3H), 3.89 (s, 4H), 3.93 (s, 3H), 3.95 (s, 2H), 4.04 (d, J = 11.7 Hz, 2H), 4.42 (d, J = 6.9 Hz, 1H), 5.32 (s, 3H), 6.55 (s, 2H), 6.84 (d, J = 8.7 Hz, 2H), 7.30 (d, J = 8.5 Hz, 2H), 7.41 (s, 1H), 8.47 (d, J = 8.6 Hz, 1H).

### Step 2. Preparation of Compound 8-21

Compound **7-21** (57 mg, 0.091 mmol) was subjected to the same preparation method as in Compound **8-1** to obtain Compound **8-21** as an ivory solid (30 mg, 66%).

¹H NMR (500 MHz, DMSO-d₆) δ 1.35 (s, 3H), 1.61 (s, 2H), 1.76 (s, 2H), 2.36 (s, 1H), 2.64 (s, 1H), 3.07 (s, 3H), 3.59 (s, 2H), 3.75 (s, 3H), 3.84 (s, 2H), 6.46 (s, 1H), 6.64 (s, 2H), 7.24 (s, 1H), 7.37 (s, 1H), 8.02 (s, 1H), 8.13 (d, J = 8.8 Hz, 1H), 8.61 (s, 1H), 12.52 (s, 1H) .

### Example 22. Preparation of N⁶-(2-methoxy-4-morpholinophenyl)-3-(oxazol-2-yl)-N⁴-(tetrahydro-2H-pyran-4-yl)-1H-pyrazolo[3,4-d]pyrimidine-4,6-diamine(Compound 8-22)

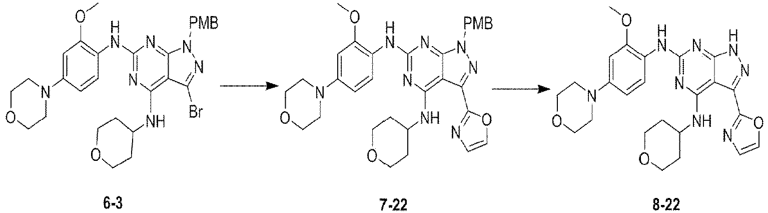

### Step 1. Preparation of Compound 7-22

Compound **6-3** (100 mg, 0.1601 mmol) and LiCl (3.4 mg, 0.080 mmol) were added to DMF (1 mL), and 2-(tribylstannyl)oxazole (0.01 mL, 0.3202 mmol) was added, and thereafter, oxygen was removed. Thereafter, Pd(PPh₃)₄ (3.4 mg, 0.008 mmol) was added, and then the mixture was stirred at 120°C for 40 minutes. The mixture was filtered through a celite pad and washed with EA (10 mL), and then the organic layer was washed with water (10 mL), subjected to moisture removal (Na₂SO₄), and concentrated under reduced pressure. The residue was separated by silica gel column chromatography (EA:HX = 2:1) to obtain Compound **7-22** (37 mg, 37%).

### Step 2. Preparation of Compound 8-22

Compound **7-22** (37 mg, 0.063 mmol) was subjected to the same preparation method as in Compound **8-1** of Example 1 to obtain Compound **8-22** as an ivory solid (11 mg, 37%).

¹H NMR (300 MHz, DMSO-d₆) δ 13.38 (s, 1H), 9.59 (d, J = 7.2 Hz, 1H), 8.31 (s, 1H), 7.98 (d, J = 8.7 Hz, 1H), 7.55 (d, J = 9.4 Hz, 2H), 6.65 (d, J = 2.5 Hz, 1H), 6.50 (dd, J = 8.9, 2.5 Hz, 1H), 4.28 (s, 1H), 3.91 (d, J = 11.8 Hz, 2H), 3.84 (s, 3H), 3.76 (t, J = 4.8 Hz, 4H), 3.53 (t, J = 10.2 Hz, 2H), 3.09 (t, J = 4.8 Hz, 4H), 2.05 (d, J = 12.7 Hz, 2H), 1.65 - 1.51 (m, 2H).

### Example 23. Preparation of N⁶-(2-methoxy-4-morpholinophenyl)-3-(1-methyl-1H-pyrazol-4-yl)-N⁴-(tetrahydro-2H-pyran-4-yl)-1H-pyrazolo[3,4-d]pyrimidine-4,6-diamine (Compound 8-23)

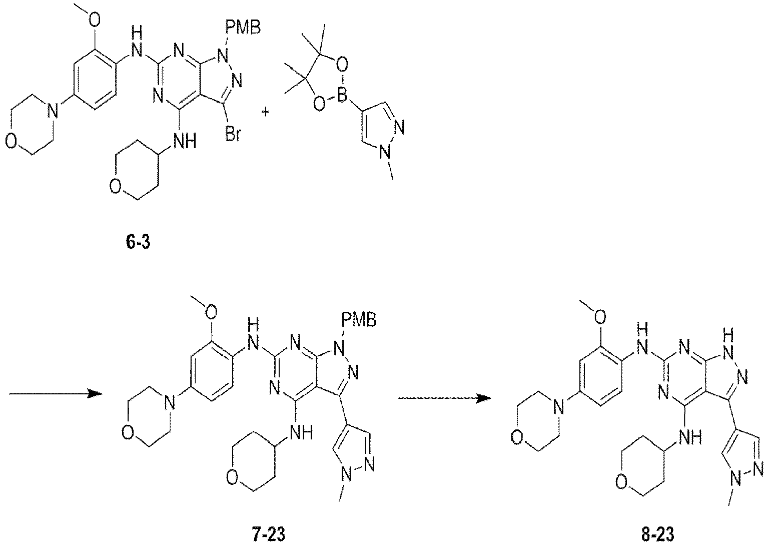

### Step 1. Synthesis of Compound 7-23

Compound **6-3** (30 mg, 0.04 mmol) and 1-methyl-4-(4,4,5,5-tetramethyl-1,3,2-dioxaborolan-2-yl)-1H-pyrazole (15 mg, 0.07 mmol) were subjected to the same preparation method as in Compound **7-1** to obtain Compound **7-23** as a pale brown solid (29.1 mg, 96%).

¹H-NMR (300 MHz, DMSO-d₆) δ 1.47-1.62 (m, 2H), 1.88-1.99 (m, 2H), 3.03-3.11 (m, 4H), 3.43 (t, J = 11.2 Hz, 2H), 3.70 (s, 3H), 3.71-3.78 (m, 4H), 3.80-3.84 (m, 2H), 3.85 (s, 3H), 3.91 (s, 3H), 4.13-4.23 (m, 1H), 5.29 (s, 2H), 5.89 (d, J = 7.3 Hz, 1H), 6.52 (dd, J = 8.9, 2.5 Hz, 1H), 6.65 (d, J = 2.5 Hz, 1H), 6.88 (d, J = 8.6 Hz, 2H), 7.22 (d, J = 8.6 Hz, 2H), 7.50 (s, 1H), 7.71 (s, 1H), 8.04 (s, 1H), 8.15 (d, J = 8.8 Hz, 1H).

### Step 2. Preparation of Compound 8-23

Compound **7-23** (27.8 mg, 0.04 mmol) was subjected to the same preparation method as in Compound **8-1** to obtain Compound **8-23** as a pale brown solid (13.4 mg, 59%) .

¹H NMR (300 MHz, DMSO-d₆) δ 1.47-1.67 (m, 2H), 1.90-2.02 (m, 2H), 3.03-3.15 (m, 4H), 3.39-3.51 (m, 2H), 3.72-3.79 (m, 4H), 3.84 (s, 3H), 3.85-3.90 (m, 2H), 3.93 (s, 3H), 4.12-4.27 (m, 1H), 5.78 (d, J = 7.4 Hz, 1H), 6.50 (dd, J = 8.8, 2.5 Hz, 1H), 6.65 (d, J = 2.5 Hz, 1H), 7.43 (s, 1H), 7.73 (s, 1H), 8.01-8.10 (m, 2H), 12.79 (s, 1H).

### Example 24. Preparation of 3-(1-(difluoromethyl)-1H-pyrazol-4-yl)-N⁶-(2-methoxy-4-morpholinophenyl)-N⁴-(tetrahydro-2H-pyran-4-yl)-1H-pyrazolo[3,4-d]pyrimidine-4,6-diamine (Compound 8-24)

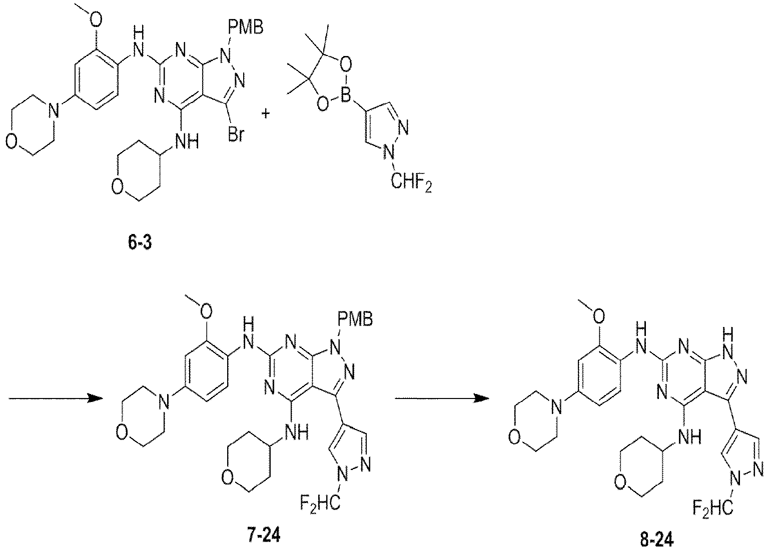

### Step 1. Synthesis of Compound 7-24

Compound **6-3** (50 mg, 0.08 mmol) and 1-(difluoromethyl)-4-(4,4,5,5-tetramethyl-1,3,2-dioxaborolan-2-yl)-1H-pyrazole (29 mg, 0.12 mmol) were subjected to the same preparation method as in Compound **7-1** to obtain Compound **7-24** as a white solid (56.3 mg, > 99%) .

¹H-NMR (400 MHz, DMSO-d₆) δ 1.42 - 1.21 (m, 7H), 1.62 (d, J= 28.0 Hz, 4H), 1.97 (s, 2H), 3.09 (t, J= 4.9 Hz, 4H), 3.71 (s, 3H), 3.79 - 3.72 (m, 4H), 3.87 (s, 3H), 5.32 (s, 2H), 5.93 (d, J= 7.5 Hz, 1H), 6.51 (dd, J= 8.9, 2.5 Hz, 1H), 6.67 (d, J= 2.5 Hz, 1H), 6.92 - 6.80 (m, 2H), 7.28 - 7.15 (m, 2H), 7.50 (s, 1H), 7.90 (s, 1H), 8.08 (d, J= 7.2 Hz, 1H), 8.18 (d, J= 8.8 Hz, 1H), 8.55 (s, 1H).

### Step 2. Preparation of Compound 8-24

Compound **7-24** (22 mg, 0.03 mmol) was subjected to the same preparation method as in Compound **8-1** to obtain Compound **8-24** as a pale brown solid (29 mg, > 99%).

¹H-NMR (400 MHz, DMSO-d₆) δ 1.58 (dt, J= 11.8, 5.6 Hz, 2H), 2.00 - 1.89 (m, 3H), 3.12 - 3.06 (m, 5H), 3.48 - 3.38 (m, 4H), 3.75 (dd, J= 6.0, 3.6 Hz, 5H), 3.84 (s, 7H), 4.18 (dd, J= 7.0, 3.7 Hz, 1H),

5.99 (d, J= 7.4 Hz, 1H), 6.51 - 6.47 (m, 1H), 6.65 (d, J= 2.5 Hz, 1H), 7.46 (s, 1H), 8.02 (d, J= 8.8 Hz, 1H), 8.09 (d, J= 3.4 Hz, 1H), 8.53 (s, 1H), 12.95 (s, 1H) .

### Example 25. Preparation of N⁶-(2-methoxy-4-morpholinophenyl)-3-(1-methyl-1H-pyrazol-3-yl)-N⁴-(tetrahydro-2H-pyran-4-yl)-1H-pyrazolo[3,4-d]pyrimidine-4,6-diamine (Compound 8-25)

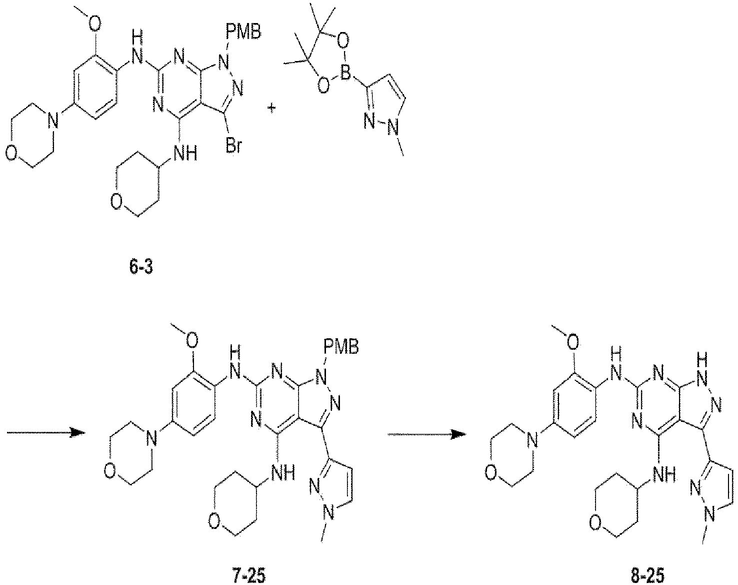

### Step 1. Synthesis of Compound 7-25

Compound **6-3** (50 mg, 0.08 mmol) and 1-methyl-3-(4,4,5,5-tetramethyl-1,3,2-dioxaborolan-2-yl)-1H-pyrazole (25 mg, 0.12 mmol) were subjected to the same preparation method as in Compound **7-1** to obtain Compound **7-25** as a white solid (54.2 mg, > 99%).

¹H-NMR (400 MHz, DMSO-d₆) δ 1.88 - 1.19 (m, 21H), 2.08 (s, 3H), 3.11 - 3.06 (m, 4H), 3.70 (s, 3H), 3.75 (t, J= 4.8 Hz, 4H), 3.87 (s, 3H), 3.96 (s, 3H), 5.32 (s, 2H), 6.51 (d, J= 8.0 Hz, 2H), 6.66 (d, J= 2.3 Hz, 2H), 6.88 (d, J= 8.5 Hz, 2H), 7.21 (d, J= 8.4 Hz, 2H), 7.38 (s, 1H), 7.66 - 7.54 (m, 6H), 7.85 (d, J= 2.4 Hz, 1H), 8.25 (d, J= 8.9 Hz, 1H), 9.62 (d, J= 7.2 Hz, 1H).

### Step 2. Preparation of Compound 8-25

Compound **7-25** (22 mg, 0.03 mmol) was subjected to the same preparation method as in Compound **8-1** to obtain Compound **8-25** as a white solid (24 mg, > 99%).

¹H-NMR (400 MHz, DMSO-d₆) δ 1.68 - 1.54 (m, 4H), 2.09 (d, J= 12.9 Hz, 4H), 3.08 (t, J= 4.9 Hz, 6H), 3.53 (t, J= 11.0 Hz, 4H), 3.75 (t, J= 4.7 Hz, 6H), 3.85 (s, 4H), 3.97 (s, 9H), 4.24 (s, 1H), 6.49 (dd, J= 8.9, 2.6 Hz, 2H), 6.66 (dd, J= 12.1, 2.4 Hz, 3H), 7.34 (s, 1H), 7.84 (d, J= 2.3 Hz, 1H), 8.09 (d, J= 8.8 Hz, 2H), 9.61 (d, J= 6.8 Hz, 1H), 12.83 (s, 1H).

### Example 26. Preparation of N⁶-(2-methoxy-4-morpholinophenyl)-3-(1H-pyrazol-4-yl)-N⁴-(tetrahydro-2H-pyran-4-yl)-1H-pyrazolo[3,4-d]pyrimidine-4,6-diamine (Compound 8-26)

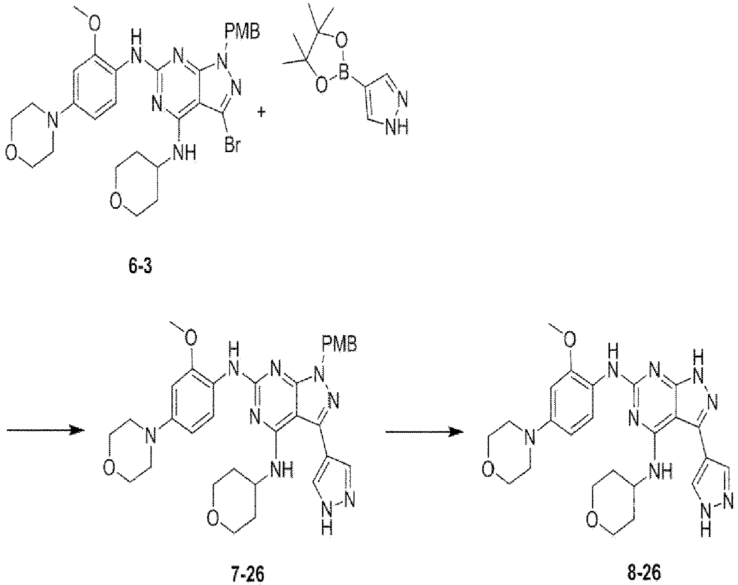

### Step 1. Synthesis of Compound 7-26

Compound **6-3** (50 mg, 0.08 mmol) and 4-(4,4,5,5-tetramethyl-1,3,2-dioxaborolan-2-yl)-1H-pyrazole (23.3 mg, 0.12 mmol) were subjected to the same preparation method as in Compound **7-1** to obtain Compound **7-26** as a white solid (15 mg, 28%).

¹H-NMR (400 MHz, DMSO-d₆) δ 1.88 - 1.19 (m, 21H), 2.08 (s, 3H), 3.11 - 3.06 (m, 4H), 3.70 (s, 3H), 3.75 (t, J= 4.8 Hz, 4H), 3.87 (s, 3H), 3.96 (s, 3H), 5.32 (s, 2H), 6.51 (d, J= 8.0 Hz, 2H), 6.66 (d, J= 2.3 Hz, 2H), 6.88 (d, J= 8.5 Hz, 2H), 7.21 (d, J= 8.4 Hz, 2H), 7.38 (s, 1H), 7.66 - 7.54 (m, 6H), 7.85 (d, J= 2.4 Hz, 1H), 8.25 (d, J= 8.9 Hz, 1H), 9.62 (d, J= 7.2 Hz, 1H).

### Step 2. Preparation of Compound 8-26

Compound **7-26** (15 mg, 0.02 mmol) was subjected to the same preparation method as in Compound **8-1** to obtain Compound **8-26** as a white solid (13 mg, > 99%)

¹H-NMR (400 MHz, DMSO-d₆) δ 1.56 - 1.44 (m, 3H), 1.95 (d, J= 12.4 Hz, 2H), 3.08 (t, J= 4.9 Hz, 4H), 3.44 (t, J= 11.2 Hz, 4H), 3.75 (t, J= 4.8 Hz, 4H), 3.82 (s, 1H), 3.84 (s, 3H), 4.19 (d, J= 11.2 Hz, 1H), 5.72 (d, J= 7.5 Hz, 1H), 6.49 (dd, J= 9.0, 2.5 Hz, 1H), 6.65 (d, J= 2.6 Hz, 1H), 7.40 (s, 1H), 7.83 (s, 1H), 8.05 (d, J= 8.8 Hz, 2H), 12.77 (s, 1H), 13.17 (s, 1H).

### Example 27. Preparation of N⁶-(2-methoxy-4-morpholinophenyl)-3-(pyridin-3-yl)-N⁴-(tetrahydro-2H-pyran-4-yl)-1H-pyrazolo[3,4-d]pyrimidine-4,6-diamine (Compound 8-27)

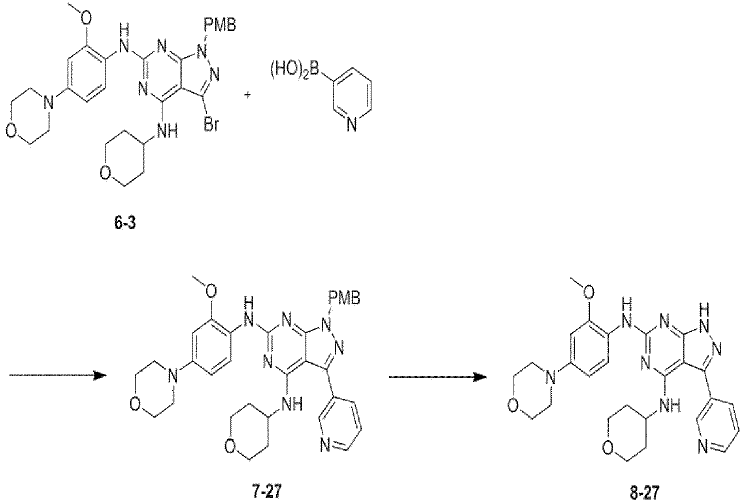

### Step 1. Synthesis of Compound 7-27

Compound **6-3** (30 mg, 0.04 mmol) and 3-pyridinylboronic acid (9 mg, 0.07 mmol) were subjected to the same preparation method as in Compound **7-1** to obtain Compound **7-27** as a pale brown solid (31.3 mg, 100%) .

¹H NMR (300 MHz, DMSO-d₆) δ 1.41-1.58 (m, 2H), 1.83-1.93 (m, 2H), 3.04-3.13 (m, 4H), 3.35-3.46 (m, 2H), 3.70 (s, 3H), 3.71-3.78 (m, 4H), 3.78-3.83 (m, 2H), 3.86 (s, 3H), 4.12-4.26 (m, 1H), 5.36 (s, 2H), 6.19 (d, J = 7.4 Hz, 1H), 6.53 (dd, J = 9.0, 2.5 Hz, 1H), 6.66 (d, J = 2.5 Hz, 1H), 6.89 (d, J = 8.7 Hz, 2H), 7.27 (d, J = 8.4 Hz, 2H), 7.49-7.61 (m, 2H), 7.98-8.06 (m, 1H), 8.08-8.20 (m, 1H), 8.63 (dd, J = 4.8, 1.6 Hz, 1H), 8.82-8.85 (m, 1H).

### Step 2. Preparation of Compound 8-27

Compound **7-27** (30.3 mg, 0.04 mmol) was subjected to the same preparation method as in Compound **8-1** to obtain Compound **8-27** as a pale brown solid (5.6 mg, 22%) .

¹H NMR (300 MHz, DMSO-d₆) δ 1.41-1.60 (m, 2H), 1.84-1.94 (m, 2H), 3.05-3.14 (m, 4H), 3.36-3.47 (m, 2H), 3.72-3.79 (m, 4H), 3.79-3.88 (m, 2H), 3.84 (s, 3H), 4.13-4.31 (m, 1H), 6.04 (d, J = 7.4 Hz, 1H), 6.50 (dd, J = 8.8, 2.5 Hz, 1H), 6.65 (d, J = 2.5 Hz, 1H), 7.49 (s, 1H), 7.51-7.57 (m, 1H), 7.99-8.06 (m, 2H), 8.64 (dd, J = 4.8, 1.7 Hz, 1H), 8.86 (dd, J = 2.2, 0.9 Hz, 1H), 13.11 (s, 1H).

### Example 28. Preparation of N⁶-(2-methoxy-4-morpholinophenyl)-3-(pyridin-4-yl)-N⁴-(tetrahydro-2H-pyran-4-yl)-1H-pyrazolo[3,4-d]pyrimidine-4,6-diamine (Compound 8-28)

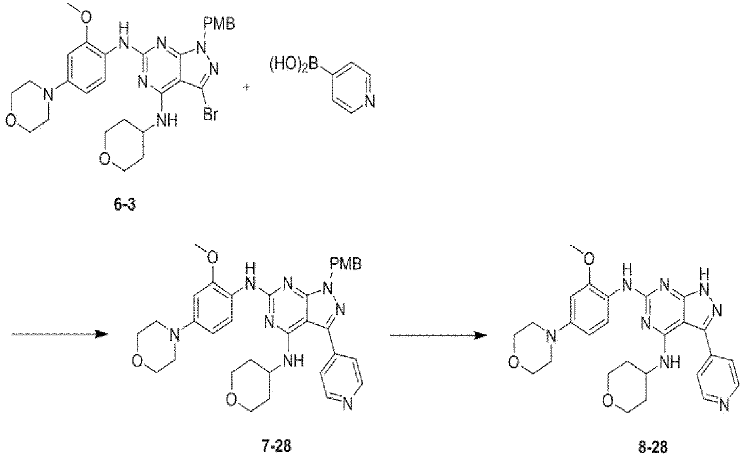

### Step 1. Synthesis of Compound 7-28

Compound **6-3** (15 mg, 0.02 mmol) and 4-pyridinylboronic acid (5 mg, 0.03 mmol) were subjected to the same preparation method as in Compound **7-1** to obtain Compound **7-28** as a white solid (17.5 mg, 100%).

¹H NMR (500 MHz, DMSO-d₆) δ 1.48-1.60 (m, 2H), 1.87-1.94 (m, 2H), 3.08-3.12 (m, 4H), 3.41 (t, J = 11.3 Hz, 2H), 3.70 (m, 3H), 3.73-3.78 (m, 4H), 3.81-3.84 (m, 2H), 3.85 (s, 3H), 4.16-4.25 (m, 1H), 5.36 (s, 2H), 6.26 (d, J = 7.3 Hz, 1H), 6.53 (dd, J = 8.9, 2.6 Hz, 1H), 6.66 (d, J = 2.6 Hz, 1H), 6.87-6.91 (m, 2H), 7.26 (d, J = 8.7 Hz, 2H), 7.60 (s, 1H), 7.63-7.65 (m, 2H), 8.10 (d, J = 9.7 Hz, 1H), 8.66-8.70 (m, 2H).

### Step 2. Preparation of Compound 8-28

Compound **7-28** (15 mg, 0.02 mmol) was subjected to the same preparation method as in Compound **8-1** to obtain Compound **8-28** as a pale brown solid (11.4 mg, 94%).

¹H NMR (500 MHz, DMSO-d₆) δ 1.50-1.59 (m, 2H), 1.88-1.95(m, 2H), 3.09 (t, J = 4.8 Hz, 4H), 3.42 (t, J = 10.9 Hz, 2H), 3.75 (t, J = 4.7 Hz, 4H), 3.84 (s, 3H), 3.85-3.87 (m, 2H), 4.18-4.27 (m, 1H), 6.12 (d, J = 7.3 Hz, 1H), 6.50 (dd, J = 8.8, 2.5 Hz, 1H), 6.65 (d, J = 2.5 Hz, 1H), 7.52 (s, 1H), 7.66 (d, J = 5.6 Hz, 2H), 7.99 (d, J = 8.8 Hz, 1H), 8.69 (d, J = 5.6 Hz, 2H), 13.21 (s, 1H).

### Example 29. Preparation of N⁶-(2-methoxy-4-morpholinophenyl)-3-(pyrimidin-5-yl)-N⁴-(tetrahydro-2H-pyran-4-yl)-1H-pyrazolo[3,4-d]pyrimidine-4,6-diamine (Compound 8-29)

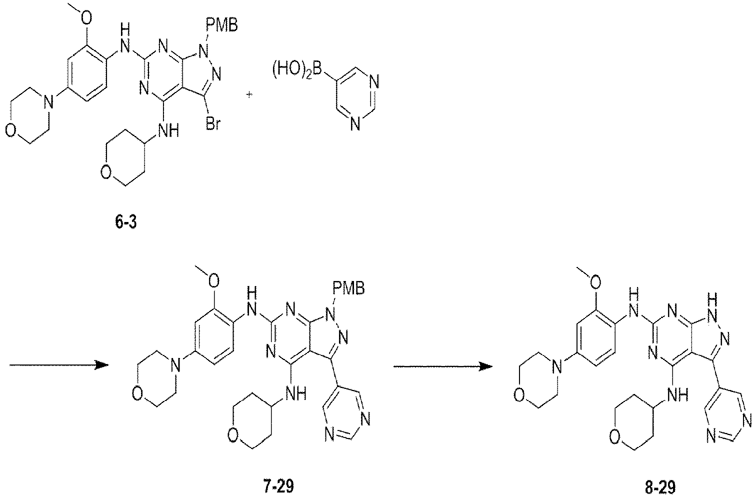

### Step 1. Synthesis of Compound 7-29

Compound **6-3** (15 mg, 0.02 mmol) and boronic acid (9 mg, 0.07 mmol) were subjected to the same preparation method as in Compound **7-1** to obtain Compound **7-29** as a light yellow solid (17.4 mg, 57%).

¹H-NMR (300 MHz, DMSO-d₆) δ 1.47-1.62 (m, 2H), 1.88-1.99 (m, 2H), 3.03-3.11 (m, 4H), 3.43 (t, J = 11.2 Hz, 2H), 3.70 (s, 3H), 3.71-3.78 (m, 4H), 3.80-3.84 (m, 2H), 3.85 (s, 3H), 3.91 (s, 3H), 4.13-4.23 (m, 1H), 5.29 (s, 2H), 5.89 (d, J = 7.3 Hz, 1H), 6.52 (dd, J = 8.9, 2.5 Hz, 1H), 6.65 (d, J = 2.5 Hz, 1H), 6.88 (d, J = 8.6 Hz, 2H), 7.22 (d, J = 8.6 Hz, 2H), 7.50 (s, 1H), 7.71 (s, 1H), 8.04 (s, 1H), 8.15 (d, J = 8.8 Hz, 1H).

### Step 2. Preparation of Compound 8-29

Compound **7-29** (17.7 mg, 0.02 mmol) was subjected to the same preparation method as in Compound **8-1** to obtain Compound **8-29** as a gray solid (5.7 mg, 39%).

¹H NMR (300 MHz, DMSO-d₆) δ 1.43-1.60 (m, 2H), 1.81-1.97 (m, 2H), 3.04-3.16 (m, 4H), 3.36-3.47 (m, 2H), 3.71-3.80 (m, 4H), 3.85 (s, 3H), 3.86-3.92 (m, 2H), 4.13-4.31 (m, 1H), 6.46-6.58 (m, 2H), 6.66 (s, 1H), 7.52 (s, 1H), 8.00 (d, J = 8.7 Hz, 1H), 9.02 (s, 2H), 9.24 (s, 1H), 13.25 (br s, 1H).

### Example 30. Preparation of N⁴-cyclohexyl-3-(1-methyl-1H-pyrazol-4-yl)-N⁶-(4-morpholinocyclohexyl)-1H-pyrazolo[3,4-d]pyrimidine-4,6-diamine (Compound 8-30)

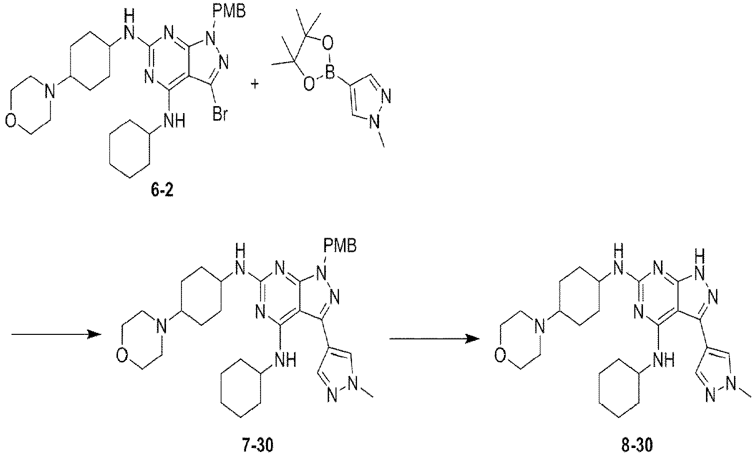

### Step 1. Synthesis of Compound 7-30

Compound **6-2** (30 mg, 0.05 mmol) and 1-methyl-4-(4,4,5,5-tetramethyl-1,3,2-dioxaborolan-2-yl)-1H-pyrazole (16 mg, 0.07 mmol) were used to obtain Compound **7-30** (32.1 mg), which was used for a next step.

¹H NMR (400 MHz, DMSO-d₆) δ 1.21 (dd, J = 35.5, 11.3 Hz, 14H), 1.64 (s, 5H), 1.93 (d, J = 48.5 Hz, 9H), 2.16 (s, 1H), 3.56 (d, J = 5.4 Hz, 4H), 3.70 (s, 3H), 3.90 (s, 3H), 4.03 (dd, J = 14.8, 7.6 Hz, 2H), 5.20 (s, 2H), 5.37 (s, 1H), 5.58 (s, 1H), 6.53 (s, 1H), 6.67 (s, 1H), 6.86 (d, J = 8.1 Hz, 2H), 7.14 (s, 1H), 7.25 (s, 1H), 7.66 (s, 1H), 7.99 (s, 1H).

### Step 2. Preparation of Compound 8-30

Compound **7-30** (32.1 mg, 0.05 mmol) was subjected to the same preparation method as in Compound **8-1** to obtain Compound **8-30** as a white solid (5.2 mg, 20%).

¹H NMR (500 MHz, DMSO-d₆) δ 1.16-1.39 (m, 9H), 1.50-1.76 (m, 3H), 1.83-2.03 (m, 6H), 2.15 (t, J = 8.9 Hz, 1H), 2.47 (t, J = 4.5 Hz, 4H), 3.56 (t, J = 4.6 Hz, 4H), 3.63 (s, 1H), 3.92 (s, 3H), 4.01 (s, 1H), 5.29-5.37 (m, 1H), 6.42 (d, J = 8.0 Hz, 1H), 7.68 (s, 1H), 7.99 (s, 1H), 12.34-12.71 (m, 1H).

### <Example B. Preparation of Compound 11 by Reaction Scheme 2>

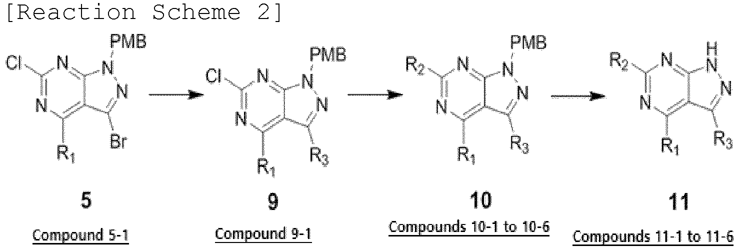

### Example 31. Preparation of N⁴-cyclohexyl-N⁶-(2-methoxy-4-(4-methylpiperazin-1-yl)phenyl)-3-(1-methyl-1H-pyrazol-4-yl)-1H-pyrazolo[3,4-d]pyrimidine-4,6-diamine (Compound 11-1)

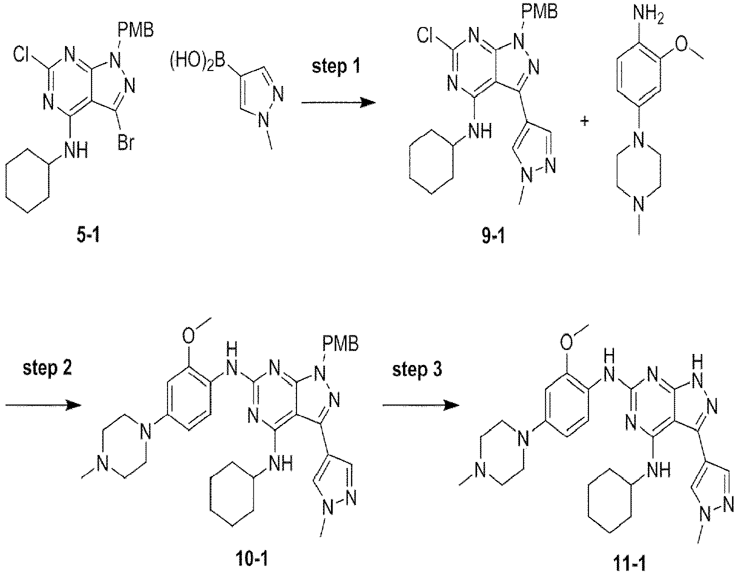

### Step 1. Synthesis of Compound 9-1

Compound **5-1** (1.0 g, 2.22 mmol), 1-methylpyrazole-4-boronic acid (370 mg, 1.33 mmol), and K₂CO₃ (613 mg, 4.44 mmol) were dissolved in dioxane:water (4:1 in v/v, 10 mL) and then purged with argon gas to remove oxygen. Thereafter, Pd(PPh₃)₄ (84.3 mg, 0.033 mmol) was added, followed by stirring at 100°C for 2 hours. Water (10 mL) was added to the mixture, followed by extraction with EA (20 mL X2). The organic layer was subjected to moisture removal (Na₂SO₄), filtered, and then concentrated under reduced pressure. The residue was separated by silica gel column chromatography (EA:HX = 2:1) to obtain Compound **9-1** as a white solid (422 mg, 42.2 %).

¹H-NMR (300 MHz, CDCl₃-d₆) δ 7.69 (s, 2H), 7.31 (d, J= 6.3 Hz, 2H), 6.81 (d, J= 6.4 Hz, 2H), 5.53 (d, J = 6.0 Hz, 1H), 5.41 (s, 2H), 4.24-4.18 (m, 1H), 3.97 (s, 3H), 3.74 (s, 3H), 2.03-1.97 (m, 2H), 1.69-1.57 (m, 2H), 1.49-1.39 (m, 2H), 1.27-1.14 (m, 4H).

### Step 2. Synthesis of Compound 10-1

Compound **9-1** (60 mg, 0.13 mmol) was dissolved in 2-butanol (1 mL), and then 2-methoxy-4-(4-methylpiperazin-1-yl)aniline (44.2 mg, 0.2 mmol) and K₂CO₃ (29.4 mg, 0.21 mmol) were added. The reaction mixture was purged with argon gas for 5 minutes to remove oxygen, and Pd₂dba₃ (30.4 mg, 0.033 mmol) and XPhos (23.4 mg, 0.049 mmol) were added, followed by stirring at 110°C for one day. The mixture was filtered through a celite pad and washed with EA (10 mL), and then the combined filtrate was concentrated under reduced pressure. The residue was separated by silica gel column chromatography (MC:MeOH = 97:3) to obtain Compound **10-1** as a pink solid (38.9, 45.9 %).

¹H NMR (500 MHz, CDCl₃) δ 8.54 (d, J = 9.4 Hz, 1H), 7.70 (d, J = 19.1 Hz, 2H), 7.40 - 7.32 (m, 3H), 6.87 - 6.79 (m, 2H), 6.60 - 6.53 (m, 2H), 5.38 (s, 2H), 5.24 (d, J = 7.8 Hz, 1H), 4.19 - 4.09 (m, 1H), 3.97 (s, 3H), 3.92 (s, 3H), 3.76 (s, 3H), 3.19 (t, J = 4.9 Hz, 4H), 2.63 (t, J = 4.9 Hz, 4H), 2.38 (s, 3H), 2.05 (d, J = 9.7 Hz, 2H), 1.67 (ddd, J = 36.2, 9.2, 4.2 Hz, 5H), 1.44 (q, J = 12.1 Hz, 2H), 1.26 - 1.18 (m, 3H).

### Step 3. Preparation of Compound 11-1

Compound **10-1** (31.7 mg, 0.049 mmol) was subjected to the same preparation method as in Compound **8-1** to obtain Compound **11-1** as a green solid (2.4 mg, 9.5 %).

¹H NMR (400 MHz, DMSO) δ 12.75 (s, 1H), 8.10 - 7.99 (m, 2H), 7.70 (s, 1H), 7.36 (s, 1H), 6.63 (d, J = 2.5 Hz, 1H), 6.47 (dd, J = 8.9, 2.5 Hz, 1H), 5.58 (d, J = 7.7 Hz, 1H), 4.24 (dd, J = 5.7, 2.3 Hz, 1H), 4.10 (s, 1H), 4.00 (dt, J = 9.7, 4.1 Hz, 2H), 3.92 (s, 3H), 3.84 (s, 3H), 3.17 (s, 4H), 3.11 (t, J = 4.9 Hz, 4H), 2.25 (s, 3H), 1.99 - 1.89 (m, 3H), 1.61 (ddt, J = 30.0, 12.7, 4.6 Hz, 3H), 1.44 - 1.26 (m, 7H).

### Example 32. Synthesis of N⁴-cyclohexyl-N⁶-(2-methoxy-4-(4-methyl-4H-1,2,4-triazol-3-yl)phenyl)-3-(1-methyl-1H-pyrazol-4-yl)-1H-pyrazolo[3,4-d]pyrimidine-4,6-diamine (Compound 11-2)

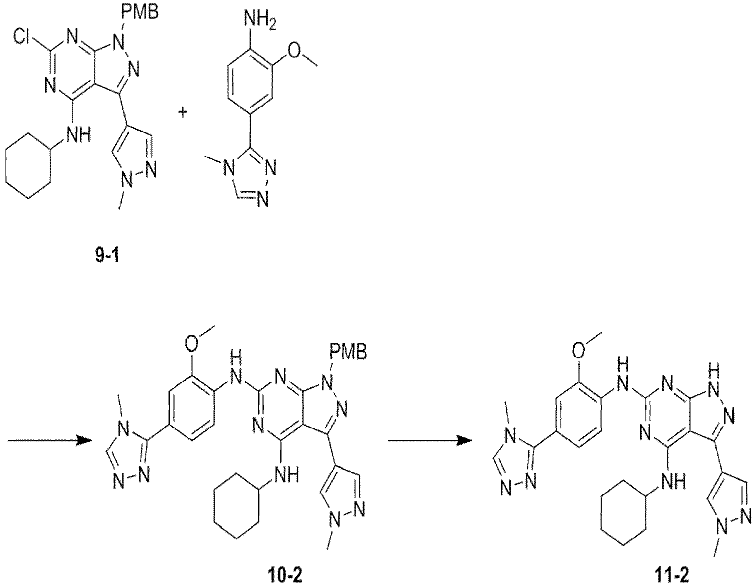

### Step 1. Synthesis of Compound 10-2

Compound **9-1** (60 mg, 0.13 mmol) and 2-methoxy-4-(4-methyl-4H-1,2,4-triazol-3-yl)aniline (32.5 mg, 0.16 mmol) were subjected to the same preparation method as in Compound **10-1** to obtain Compound **10-2** as a brown solid (20.1 mg, 24.4 %).

¹H NMR (300 MHz, CDCl₃) δ 8.87 (d, J = 8.5 Hz, 1H), 8.17 (s, 1H), 7.79 - 7.71 (m, 2H), 7.70 (s, 1H), 7.42 - 7.30 (m, 3H), 7.18 (dd, J = 8.4, 1.9 Hz, 1H), 6.88 - 6.78 (m, 2H), 5.42 (s, 2H), 5.33 (d, J = 7.9 Hz, 1H), 4.25 - 4.11 (m, 1H), 3.98 (d, J = 7.2 Hz, 6H), 3.77 (d, J = 16.4 Hz, 6H), 2.05 (d, J = 12.5 Hz, 2H), 1.79 - 1.56 (m, 3H), 1.45 (q, J = 11.8 Hz, 2H), 1.31 - 1.19 (m, 3H).

### Step 2. Preparation of Compound 11-2

Compound **10-2** (20 mg, 0.032 mmol) was subjected to the same preparation method as in Compound **11-1** to obtain Compound **11-2** as a white solid (8.1 mg, 50.9 %).

¹H NMR (300 MHz, CDCl₃) δ 10.52 (s, 1H), 8.78 (d, J = 8.5 Hz, 1H), 8.16 (s, 1H), 7.83 - 7.66 (m, 3H), 7.32 (d, J = 1.9 Hz, 1H), 7.16 (d, J = 8.4 Hz, 1H), 5.41 (d, J = 7.8 Hz, 1H), 4.19 (d, J = 10.0 Hz, 1H), 3.99 (d, J = 13.1 Hz, 6H), 3.77 (s, 3H), 2.09 (d, J = 12.2 Hz, 2H), 1.71 (t, J = 19.4 Hz, 4H), 1.47 (q, J = 12.1 Hz, 2H), 1.36 - 1.17 (m, 6H).

### Example 33. Synthesis of 4-((4-(cyclohexylamino)-3-(1-methyl-1H-pyrazol-4-yl)-1H-pyrazolo[3,4-d]pyrimidin-6-yl)amino)-3-methoxy-N-methylbenzamide (Compound 11-3)

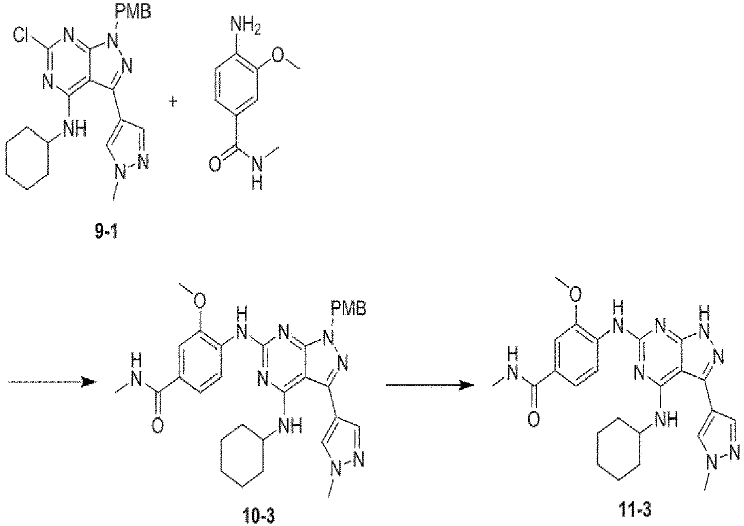

### Step 1. Synthesis of Compound 10-3

Compound **9-1** (60 mg, 0.13 mmol) and 4-amino-3-methoxy-N-methylbenzamide (27.7 mg, 0.16 mmol) were subjected to the same preparation method as in Compound **10-1** to obtain Compound **10-3** as a brown solid (48.9 mg, 60.6 %).

¹H NMR (500 MHz, CDCl₃) δ 8.78 (d, J = 8.5 Hz, 1H), 7.74 (d, J = 11.1 Hz, 2H), 7.70 (s, 1H), 7.46 (d, J = 1.9 Hz, 1H), 7.35 (d, J = 8.6 Hz, 2H), 7.28 (dd, J = 8.4, 1.9 Hz, 1H), 6.87 - 6.80 (m, 2H), 6.17 (q, J = 4.7 Hz, 1H), 5.40 (s, 2H), 5.33 (d, J = 7.9 Hz, 1H), 4.21 - 4.12 (m, 1H), 3.99 (d, J = 7.6 Hz, 6H), 3.75 (s, 3H), 3.02 (d, J = 4.8 Hz, 3H), 2.06 (dd, J = 12.6, 4.5 Hz, 2H), 1.79 - 1.60 (m, 5H), 1.51 - 1.39 (m, 3H), 1.35 - 1.16 (m, 8H).

### Step 2. Preparation of Compound 11-3

Compound **10-3** (37 mg, 0.062 mmol) was subjected to the same preparation method as in Compound **11-1** to obtain Compound **11-3** as a white solid (9.1 mg, 30.8 %).

¹H NMR (300 MHz, CDCl₃) δ 10.64 (s, 1H), 8.65 (d, J = 8.4 Hz, 1H), 7.76 (d, J = 6.7 Hz, 3H), 7.44 (d, J = 1.9 Hz, 1H), 7.25 - 7.15 (m, 1H), 6.24 (d, J = 5.0 Hz, 1H), 5.41 (d, J = 7.8 Hz, 1H), 4.17 (d, J = 8.9 Hz, 1H), 3.98 (d, J = 13.9 Hz, 6H), 3.00 (d, J = 4.8 Hz, 3H), 2.08 (d, J = 12.6 Hz, 2H), 1.71 (t, J = 19.1 Hz, 3H), 1.47 (q, J = 12.1 Hz, 2H), 1.25 (d, J = 10.5 Hz, 3H).

### Example 34. Preparation of (4-((4-(cyclohexylamino)-3-(1-methyl-1H-pyrazol-4-yl)-1H-pyrazolo[3,4-d]pyrimidin-6-yl)amino)-3-methoxyphenyl)(morpholino)methanone (Compound 11-4)

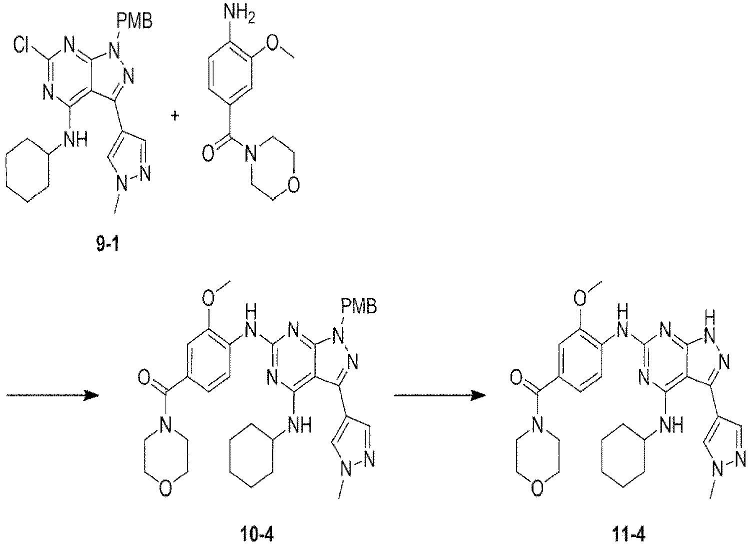

### Step 1. Synthesis of Compound 10-4

Compound **9-1** (54 mg, 0.12 mmol) and (4-amino-3-methoxyphenyl)(morpholino)methanone (45 mg, 0.19 mmol) were subjected to the same preparation method as in Compound **10-1** to obtain Compound **10-4** as a white solid (48.1 mg, 73.7 %).

¹H NMR (400 MHz, CDCl₃) δ 8.75 (d, J = 8.2 Hz, 1H), 7.76 - 7.64 (m, 3H), 7.34 (d, J = 8.5 Hz, 2H), 7.02 (d, J = 7.8 Hz, 2H), 6.87 - 6.77 (m, 2H), 5.40 (s, 2H), 5.32 (d, J = 7.9 Hz, 1H), 4.22 - 4.11 (m, 1H), 3.96 (d, J = 4.6 Hz, 6H), 3.73 (d, J = 15.6 Hz, 11H), 2.06 (d, J = 10.8 Hz, 2H), 1.68 (ddt, J = 30.7, 13.0, 3.9 Hz, 3H), 1.54 - 1.36 (m, 2H), 1.26 - 1.14 (m, 3H).

### Step 2. Preparation of Compound 11-4

Compound **10-4** (27.1 mg, 0.042 mmol) was subjected to the same preparation method as in Compound **11-1** to obtain Compound **11-4** as a white solid (11.1 mg, 50.2 %).

¹H NMR (400 MHz, CDCl₃) δ 8.84 (s, 1H), 8.32 (d, J = 8.2 Hz, 1H), 7.87 (s, 1H), 7.75 (s, 1H), 7.07 - 6.94 (m, 2H), 5.96 (s, 1H), 4.11 (d, J = 9.1 Hz, 1H), 4.02 (s, 3H), 3.93 (s, 3H), 3.71 (s, 8H), 2.06 (t, J = 8.6 Hz, 2H), 1.85 - 1.60 (m, 3H), 1.34 (dq, J = 50.7, 11.9 Hz, 5H).

### Example 35. Preparation of (4-((4-(cyclohexylamino)-3-(1-methyl-1H-pyrazol-4-yl)-1H-pyrazolo[3,4-d]pyrimidin-6-yl)amino)-3-methoxyphenyl) (4-methylpiperazin-1-yl)methanone (Compound 11-5)

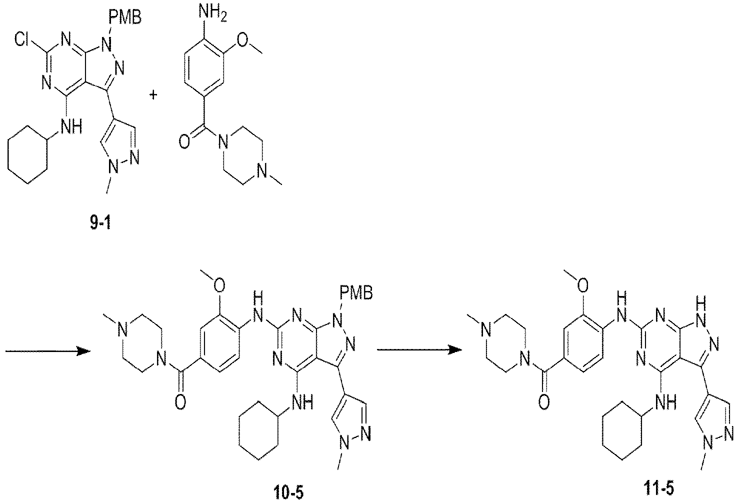

### Step 1. Synthesis of Compound 10-5

Compound **9-1** (60 mg, 0.13 mmol) and (4-amino-3-methoxyphenyl)(4-methylpiperazin-1-yl)methanone (39.6 mg, 0.16 mmol) were subjected to the same preparation method as in Compound **10-1** to obtain Compound **10-5** as a yellow solid (60.2 mg, 68.1 %)..

¹H NMR (500 MHz, CDCl₃) δ 8.75 (d, J = 8.7 Hz, 1H), 7.73 (s, 1H), 7.69 (d, J = 4.4 Hz, 2H), 7.38 - 7.32 (m, 2H), 7.06 - 7.00 (m, 2H), 6.87 - 6.79 (m, 2H), 5.41 (s, 2H), 5.32 (d, J = 7.8 Hz, 1H), 4.21 - 4.11 (m, 1H), 3.97 (d, J = 7.5 Hz, 6H), 3.76 (s, 7H), 2.45 (s, 4H), 2.33 (s, 3H), 2.10 - 2.00 (m, 2H), 1.72 (dt, J = 12.6, 4.3 Hz, 2H), 1.68 - 1.59 (m, 1H), 1.44 (dddd, J = 14.7, 11.7, 7.6, 3.7 Hz, 2H), 1.23 (hept, J = 7.8 Hz, 3H).

### Step 2. Preparation of Compound 11-5

Compound **10-5** (44 mg, 0.066 mmol) was subjected to the same preparation method as in Compound **11-1** to obtain Compound **11-5** as a white solid (12.1 mg, 33.7 %).

¹H NMR (500 MHz, CDCl₃) δ 10.42 (s, 1H), 8.66 (d, J = 8.5 Hz, 1H), 7.76 (d, J = 16.8 Hz, 2H), 7.66 (s, 1H), 7.01 (d, J = 6.5 Hz, 2H), 5.38 (d, J = 7.8 Hz, 1H), 4.23 - 4.10 (m, 1H), 3.98 (d, J = 36.4 Hz, 7H), 3.70 (s, 4H), 2.40 (d, J = 54.2 Hz, 7H), 2.13 - 2.02 (m, 2H), 1.79 - 1.70 (m, 2H), 1.66 (d, J = 12.9 Hz, 1H), 1.46 (q, J = 12.5 Hz, 2H), 1.34 - 1.26 (m, 4H).

### Example 36. Preparation of 4-((4-(cyclohexylamino)-3-(1-methyl-1H-pyrazol-4-yl)-1H-pyrazolo[3,4-d]pyrimidin-6-yl)amino)-3-methoxy-N-(1-methylpiperidin-4-yl)benzamide (Compound 11-6)

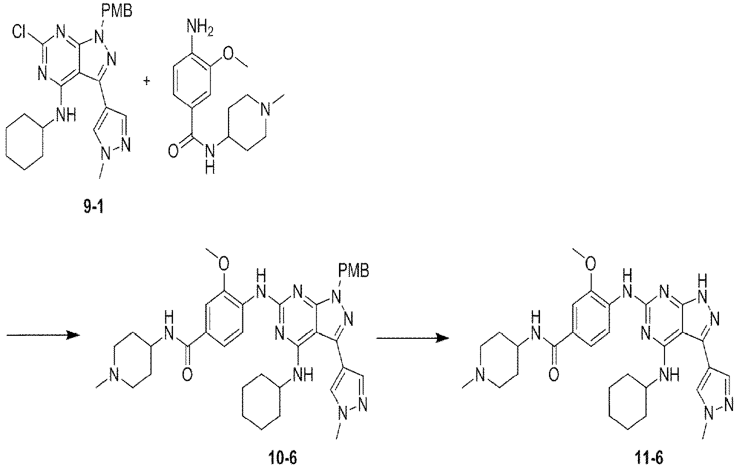

### Step 1. Synthesis of Compound 10-6

Compound **9-1** (60 mg, 0.13 mmol) and 4-amino-3-methoxy-N-(1-methylpiperidin-4-yl)benzamide (41.8 mg, 0.16 mmol) were subjected to the same preparation method as in Compound **10-1** to obtain Compound **10-6** as a green solid (48.2 mg, 54.7 %).

¹H NMR (400 MHz, CDCl₃) δ 8.77 (d, J = 8.4 Hz, 1H), 7.76 (s, 1H), 7.71 (d, J = 10.3 Hz, 2H), 7.43 (d, J = 1.9 Hz, 1H), 7.41 - 7.30 (m, 3H), 6.87 - 6.79 (m, 2H), 6.70 (d, J = 8.1 Hz, 1H), 5.39 (s, 2H), 5.33 (d, J = 7.8 Hz, 1H), 4.27 - 4.11 (m, 2H), 3.98 (d, J = 4.1 Hz, 6H), 3.75 (s, 3H), 3.34 (d, J = 12.2 Hz, 4H), 2.72 (td, J = 12.0, 3.7 Hz, 2H), 2.65 (s, 3H), 2.18 (tt, J = 14.1, 7.3 Hz, 4H), 2.04 (dt, J = 12.8, 4.2 Hz, 2H), 1.76 - 1.67 (m, 2H), 1.46 (dt, J = 14.7, 11.2 Hz, 2H), 1.29 (dd, J = 12.4, 5.6 Hz, 3H).

### Step 2. Preparation of Compound 11-6

Compound **10-6** (35.6 mg, 0.052 mmol) was subjected to the same preparation method as in Compound **11-1** to obtain Compound **11-6** as a yellow solid (13.1 mg, 47.5 %).

¹H NMR (300 MHz, CDCl₃) δ 8.66 (d, J = 8.5 Hz, 1H), 7.84 - 7.65 (m, 3H), 7.39 (d, J = 1.8 Hz, 1H), 7.24 - 7.18 (m, 1H), 6.06 (d, J = 8.1 Hz, 1H), 5.79 (s, 1H), 5.39 (d, J = 7.9 Hz, 1H), 4.18 (s, 2H), 3.99 (d, J = 13.5 Hz, 6H), 2.90 (d, J = 11.3 Hz, 2H), 2.35 (s, 3H), 2.21 (t, J = 11.5 Hz, 3H), 2.04 (s, 5H), 1.81 - 1.64 (m, 5H), 1.55 - 1.40 (m, 2H), 1.25 (d, J = 10.1 Hz, 4H).

### <Example C. Preparation of Compound 14 by Reaction Scheme 3>

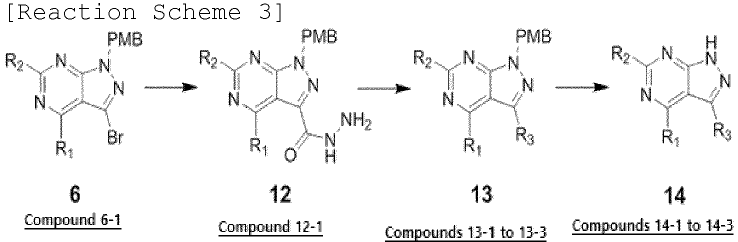

### Example 37. Preparation of N⁴-cyclohexyl-N⁶-(2-methoxy-4-morpholinophenyl)-3-(4-methyl-4H-1,2,4-triazol-3-yl)-1H-pyrazolo[3,4-d]pyrimidine-4,6-diamine (Compound 14-1)

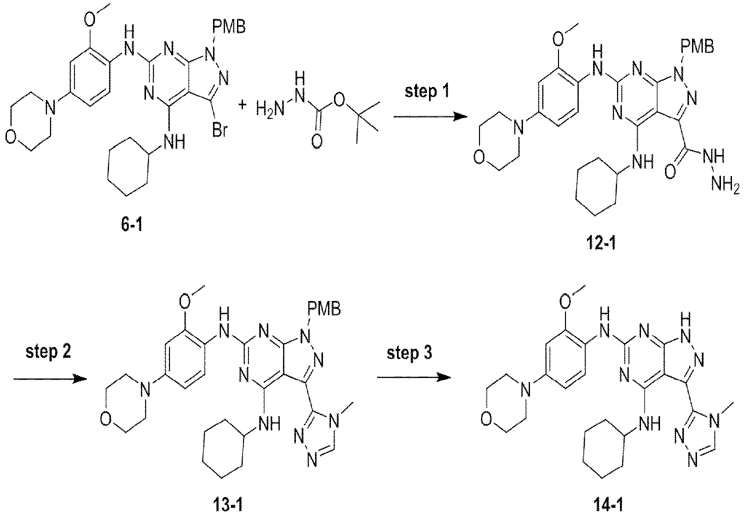

### Step 1. Synthesis of Compound 12-1

Compound **6-1** (3.10 g, 5 mmol), molybdenum hexacarbonyl (Mo(CO)₆; 1.60 g, 6 mmol), and tert-butyl carbazate (2.0 g, 15 mmol) were dissolved in dioxane (30 mL), and then 1,8-diazavid cycle [5.4.0]undensen-7 (DBU, 2.2 mL, 15 mmol) was added. The mixture was purged with argon gas to remove oxygen, and Pd(dppf)Cl₂ (180 mg, 0.012 mmol) was added, followed by stirring at 110°C for 10 minutes. The reaction product was concentrated, and separated by silica gel column chromatography (EA:Hx = 1:2) to obtain tert-butyl hydrazine carboxylate as a white solid (2.03 g, 58%).

Tert-butyl hydrazine carboxylate (1.5 g, 2.1372 mmol) was dissolved in MC (10.0 mL), and then TFA (20 mL) was added, followed by stirring at room temperature for 16 hours. The reaction product was concentrated under reduced pressure, and the pH was adjusted to 7 by an aqueous solution of sat'd NaHCO₃. EA (50 mL) was added, and then mixture was wahsed with water (50 mL), subjected to moisture removal (Na₂SO₄), and then concentrated under reduced pressure. The residue was separated by silica gel column chromatography (EA:Hx = 1:1) to obtain Compound **12-1** as a pale brown solid (800 mg, 66%).

¹H NMR (300 MHz, DMSO-d₆) δ 1.36 (s, 8H), 1.58 (s, 2H), 1.73 (s, 3H), 3.09 (t, J = 4.8 Hz, 5H), 3.71 (s, 3H), 3.75 (t, J = 4.8 Hz, 5H), 3.86 (s, 3H), 4.60 (s, 2H), 5.33 (s, 2H), 6.51 (dd, J = 8.9, 2.5 Hz, 1H), 6.66 (d, J = 2.5 Hz, 1H), 6.89 (d, J = 8.7 Hz, 2H), 7.23 (d, J = 8.7 Hz, 2H), 7.48 (s, 1H), 8.17 (d, J = 8.8 Hz, 1H), 9.40 (d, J = 7.6 Hz, 1H), 9.91 (s, 1H).

### Step 2. Synthesis of Compound 13-1

Compound **12-1** (60 mg, 0.10 mmol) was dissolved in N,N-dimethylform amide (DMF-DMA; 1 mL) and stirred at 100°C for 2 hours. Hx (2 mL) was added to the reaction mixture, and the produced solid was filtered and dissolved in MeCN (1 mL), and then one drop of AcOH and methylamine (2M in THF; 0.2 mL, 0.4 mmol) were added. The mixture was stirred at 85°C for 24 hours and cooled at room temperature, and the resultant solid was filtered to obtain Compound **13-1** as a brown solid (20.1 mg, 32.4%).

¹H NMR (400 MHz, CDCl₃) δ 8.75 (d, J = 8.2 Hz, 1H), 7.76 - 7.64 (m, 3H), 7.34 (d, J = 8.5 Hz, 2H), 7.02 (d, J = 7.8 Hz, 2H), 6.87 - 6.77 (m, 2H), 5.40 (s, 2H), 5.32 (d, J = 7.9 Hz, 1H), 4.22 - 4.11 (m, 1H), 3.96 (d, J = 4.6 Hz, 6H), 3.73 (d, J = 15.6 Hz, 11H), 2.06 (d, J = 10.8 Hz, 2H), 1.68 (ddt, J = 30.7, 13.0, 3.9 Hz, 3H), 1.54 - 1.36 (m, 2H), 1.26 - 1.14 (m, 3H).

### Step 3. Preparation of Compound 14-1

Compound **13-1** (23 mg, 0.037 mmol) was subjected to the same preparation method as in Compound **8-1** to obtain Compound **14-1** as a white solid (13.1 mg., 70.2 %)

¹H NMR (500 MHz, CDCl₃) δ 1.38 (d, J = 11.8 Hz, 1H), 1.60 - 1.43 (m, 3H), 1.64 (d, J = 12.5 Hz, 1H), 1.85 (dq, J = 12.7, 4.4 Hz, 3H), 2.10 (dd, J = 11.6, 5.7 Hz, 2H), 3.19 - 3.04 (m, 4H), 3.87 (d, J = 6.5 Hz, 7H), 4.08 (s, 3H), 4.26 - 4.13 (m, 1H), 6.52 (d, J = 8.9 Hz, 2H), 7.33 (s, 1H), 8.12 (s, 1H), 8.41 (d, J = 8.5 Hz, 1H), 10.25 (d, J = 7.4 Hz, 1H), 11.28 (s, 1H).

### Example 38. Preparation of N⁴-cyclohexyl-N⁶-(2-methoxy-4-morpholinophenyl)-3-(1,3,4-oxadiazol-2-yl)-1H-pyrazolo[3,4-d]pyrimidine-4,6-diamine (Compound 14-2)

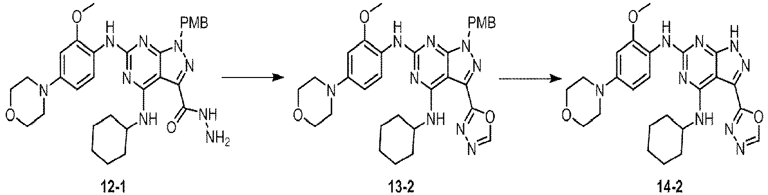

### Step 1. Preparation of Compound 13-2

Compound **12-1** (60 mg, 0.10 mmol) was dissolved in formamide (1 mL) and stirred at 100°C for 1 hour. The reaction mixture was concentrated, and then the produced solid was dissolved in phosphorus oxychloride (POCl₃; 2 mL) and stirred at 100°C for 24 hours. The reaction mixture was cooled to room temperature and slowly added to ice water (10 mL), and the produced solid was filtered to obtain Compound **13-2** as a brown solid (43.0 mg, 70.5%).

¹H NMR (300 MHz, DMSO-d₆) δ 1.37 - 1.46 (m, 4H), 1.53 (s, 1H), 1.74 (s, 3H), 1.87 - 2.04 (m, 4H), 3.08 (d, J = 6.0 Hz, 4H), 3.71 (s, 3H), 3.75 (t, J = 4.7 Hz, 4H), 3.86 (d, J = 1.6 Hz, 3H), 5.39 (d, J = 5.9 Hz, 2H), 6.50 (d, J = 8.6 Hz, 1H), 6.66 (d, J = 2.9 Hz, 1H), 6.90 (d, J = 8.4 Hz, 2H), 7.19 - 7.27 (m, 2H), 7.53 (d, J = 6.4 Hz, 1H), 8.07 - 8.17 (m, 2H), 11.70 (d, J = 22.5 Hz, 1H) .

### Step 2. Preparation of Compound 14-2

Compound **13-2** (22 mg, 0.0359 mmol) was subjected to the same preparation method as in Compound **8-1** to obtain Compound **14-2** as a yellow solid (9.8 mg., 50.5 %)

¹H NMR (400 MHz, DMSO-d₆) δ 1.39 (dt, J = 19.7, 10.5 Hz, 7H), 1.58 (s, 1H), 1.74 (s, 2H), 1.96 (d, J = 11.9 Hz, 2H), 3.05 - 3.11 (m, 4H), 3.75 (t, J = 4.8 Hz, 4H), 3.84 (s, 3H), 6.48 (dd, J = 8.8, 2.6 Hz, 1H), 6.64 (d, J = 2.5 Hz, 1H), 7.42 (s, 1H), 8.03 (d, J = 8.8 Hz, 1H), 9.35 (d, J = 7.8 Hz, 1H), 9.90 (s, 1H), 13.26 (s, 1H) .

### Example 39. Preparation of 5-(4-(cyclohexylamino)-6-((2-methoxy-4-morpholinophenyl)amino)-1H-pyrazolo[3,4-d]pyrimidin-3-yl)-1,3,4-oxadiazol-2(3H)-one (Compound 14-3)

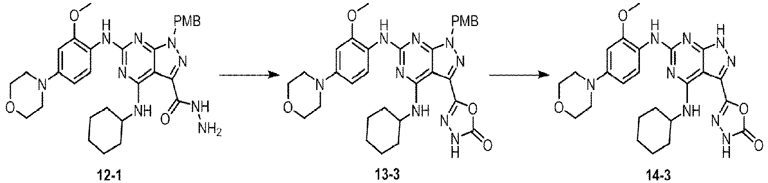

### Step 1. Preparation of Compound 13-3

Compound **12-1** (30 mg, 0.05 mmol) was dissolved in THF (2 mL), and then 1,1'-carbonyldiimidazole (CDI; 12 mg, 0.075mmol) was added, followed by stirring at 60°C for 14 hours. The reaction product was concentrated, and then separated by silica gel column chromatography (MC:MeOH = 10:1) to obtain Compound **13-3** as a light gray solid (13 mg, 41%).

¹H NMR (500 MHz, DMSO-d₆) δ 1.36 (s, 4H), 1.37 - 1.45 (m, 3H), 1.57 (s, 1H), 1.71 (d, J = 12.6 Hz, 2H), 1.94 - 2.00 (m, 2H), 3.09 (t, J = 4.7 Hz, 4H), 3.71 (s, 3H), 3.75 (s, 4H), 3.85 (s, 3H), 4.07 (s, 1H), 5.35 (s, 2H), 6.51 (dd, J = 8.7, 2.5 Hz, 1H), 6.66 (d, J = 2.6 Hz, 1H), 6.89 (d, J = 8.4 Hz, 2H), 7.25 (d, J = 8.3 Hz, 2H), 7.63 (s, 1H).

### Step 2. Preparation of Compound 14-3

Compound **13-3** (13 mg, 0.0207 mmol) was subjected to the same preparation method as in Compound **8-1** to obtain Compound **14-3** as a yellow solid (4.6 mg., 46 %)

¹H NMR (300 MHz, DMSO-d₆) δ 1.71 (s, 6H), 1.92 (s, 5H), 3.09 (dd, J = 5.8, 3.7 Hz, 5H), 3.76 (t, J = 4.8 Hz, 6H), 3.84 (s, 4H), 4.10 (s, 1H), 6.49 (dd, J = 8.8, 2.5 Hz, 1H), 6.65 (d, J = 2.6 Hz, 1H), 6.71 (d, J = 7.9 Hz, 1H), 7.52 (s, 1H), 7.97 (d, J = 8.7 Hz, 1H), 8.24 (s, 1H), 13.41 (s, 1H).

### <Example D. Preparation of Compound 18 by Reaction Scheme 4>

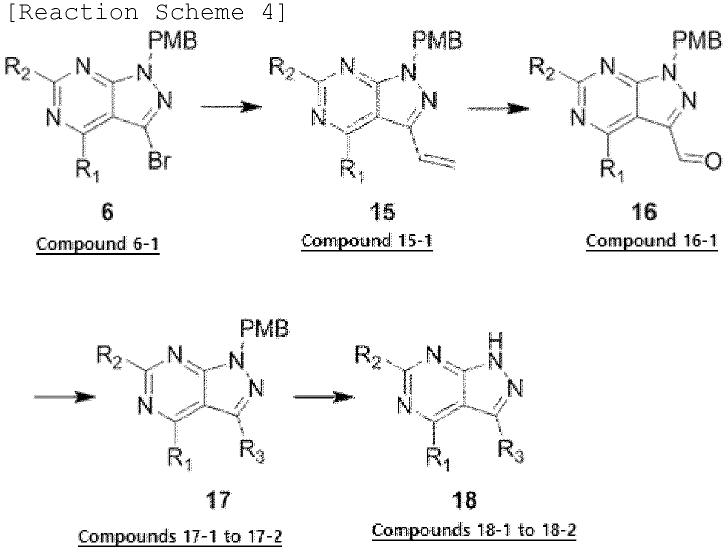

### Example 40. Preparation of N⁴-cyclohexyl-N⁶-(2-methoxy-4-morpholinophenyl)-3-(oxazol-5-yl)-1H-pyrazolo[3,4-d]pyrimidine-4,6-diamine (Compound 18-1)

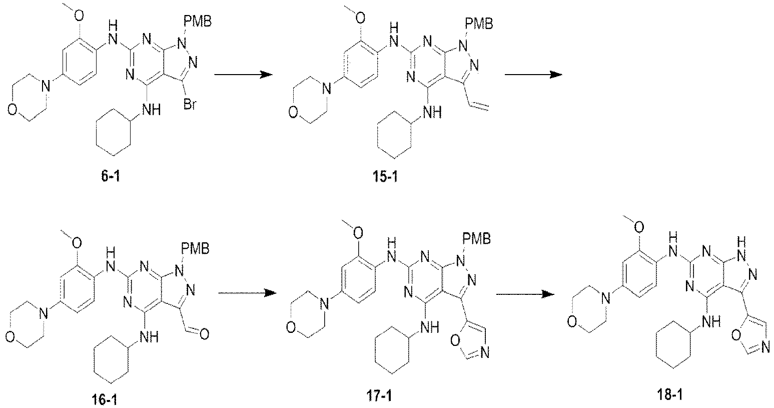

### Step 1. Synthesis of Compound 15-1

Compound **6-1** (6.81 g, 10.9385 mmol), potassium vinyltrifluoroborate (CH₂=CHBF₃K; 2.93 g, 21.8770 mmol), and potassium carbonate (K₂CO₃) (4.5 g, 32.8155 mmol) were dissolved in toluene:EtOH (ethanol) =3:1 (200 mL), and then purged with argon gas to remove oxygen. Thereafter, Pd(PPh₃)₄ (1.26 g, 1.0938 mmol) was added, followed by stirring at 80°C for 14 hours. The mixture was concentrated under reduced pressure, and then EA (200 mL) was added. The mixture was washed with water (200 mL), subjected to moisture removal (Na₂SO₄), and concentrated under reduced pressure. The residue was separated by silica gel column chromatography (EA:HX = 2:3) to obtain Compound **15-1** as a pale brown solid (5.26 g, 84.4%)

¹H NMR (500 MHz, DMSO-d₆) δ 1.17 (dd, J = 9.8, 6.7 Hz, 1H), 1.30 - 1.49 (m, 5H), 1.65 (d, J = 12.8 Hz, 1H), 1.76 (d, J = 12.3 Hz, 3H), 1.92 (d, J = 12.1 Hz, 2H), 2.09 (d, J = 1.7 Hz, 1H), 3.07 - 3.10 (m, 4H), 3.71 (d, J = 1.7 Hz, 3H), 3.75 (t, J = 4.8 Hz, 4H), 3.86 (d, J = 1.8 Hz, 3H), 4.11 (s, 1H), 5.28 (s, 2H), 5.37 (dd, J = 11.0, 2.1 Hz, 1H), 5.92 (dd, J = 17.1, 2.1 Hz, 1H), 6.48 - 6.53 (m, 2H), 6.66 (t, J = 2.1 Hz, 1H), 6.87 - 6.90 (m, 2H), 7.16 - 7.24 (m, 3H), 7.41 (s, 1H), 8.17 (d, J = 8.8 Hz, 1H).

### Step 2. Synthesis of Compound 16-1

Compound **15-1** (5.26 g, 9.2327 mmol) and NMO (2.68 g, 18.4655 mmol) were dissolved in THF:H₂O = 3:1, and then O_{S}O₄ (2.5 wt% in H₂O; 8.4 mL) was slowly added dropwise, followed by stirring at O°C for 1 hour. The mixture was concentrated under reduced pressure, and then ethyl acetate (200 mL) was added. The mixture was washed with water (200 mL), subjected to moisture removal (Na₂SO₄), and concentrated under reduced pressure. The residue was separated by silica gel column chromatography (EA:Hx = 1:1) to obtain a dihydroxy compound as a yellow solid (4.2 g, 75.4%).

The dihydroxy compound (4.2 g, 6.96 mmol) was dissolved in THF:H₂O = 3:1, and then NaIO₄ (2.68 g, 20.8706 mmol) was added, followed by stirring at room temperature for 2 hours. The mixture was concentrated under reduced pressure, and then ethyl acetate (200 mL) was added, followed by washing with water (200 mL). The organic layer was subjected to moisture removal (Na₂SO₄) and concentrated under reduced pressure. The residue was separated by silica gel column chromatography (EA:MC = 1:20) to obtain Compound **16-1** as a brown solid (70.7%).

¹H NMR (300 MHz, DMSO-d₆) δ 1.39 (q, J = 10.3, 9.7 Hz, 5H), 1.59 (s, 1H), 1.72 (s, 2H), 1.99 (s, 2H), 3.10 (t, J = 4.8 Hz, 4H), 3.72 (s, 3H), 3.73 - 3.77 (m, 4H), 3.85 (s, 3H), 4.03 (q, J = 7.1 Hz, 1H), 5.42 (s, 2H), 6.52 (dd, J = 8.9, 2.5 Hz, 1H), 6.66 (d, J = 2.5 Hz, 1H), 6.88 - 6.93 (m, 2H), 7.28 (d, J = 8.6 Hz, 2H), 7.69 (s, 1H), 7.89 (d, J = 7.7 Hz, 1H), 8.06 (d, J = 8.7 Hz, 1H), 9.82 (s, 1H).

### Step 3. Synthesis of Compound 17-1

Compound **16-1** (200 mg, 0.35 mmol), p-toluenesulfonylmethyl isocyanide (TosMIC; 75 mg, 0.38 mmol), and K₂CO₃ (145 mg, 1.05 mmol) were added to MeOH (5 mL), and stirred at 50°C for 1 hour. EA (25 mL) was added to the mixture, followed by washing with water (25 mL), moisture removal (Na₂SO₄), and concentration under reduced pressure. The residue was separated by silica gel column chromatography (EA:HX:MC = 2:1:1) to obtain Compound **17-1** as a pale brown solid (166 mg, 78%).

¹H NMR (500 MHz, DMSO-d₆) δ 1.25 (s, 2H), 1.41 (t, J = 9.6 Hz, 5H), 1.63 (d, J = 12.8 Hz, 1H), 1.75 (s, 3H), 2.01 (d, J = 9.2 Hz, 3H), 3.10 (t, J = 4.8 Hz, 4H), 3.71 (s, 3H), 3.76 (t, J = 4.8 Hz, 4H), 3.86 (s, 3H), 4.07 (s, 1H), 5.35 (s, 2H), 6.52 (dd, J = 8.8, 2.5 Hz, 1H), 6.67 (d, J = 2.5 Hz, 1H), 6.75 (d, J = 7.5 Hz, 1H), 6.89 (d, J = 8.7 Hz, 2H), 7.23 (d, J = 8.4 Hz, 2H), 7.59 (d, J = 5.6 Hz, 2H), 8.12 (d, J = 8.8 Hz, 1H), 8.64 (s, 1H) .

### Step 4. Preparation of Compound 18-1

Compound **17-1** (417 mg, 0.6828mmol) was subjected to the same preparation method as in Compound **8-1** to obtain Compound **18-1** (245 mg, 73.3%).

¹H NMR (400 MHz, DMSO-d₆) δ 1.41 (t, J = 9.3 Hz, 5H), 1.63 (d, J = 12.5 Hz, 1H), 1.74 (s, 3H), 2.01 (d, J = 11.1 Hz, 3H), 3.09 (t, J = 4.8 Hz, 4H), 3.75 (dd, J = 5.9, 3.6 Hz, 4H), 3.85 (s, 3H), 4.09 (s, 1H), 6.49 (dd, J = 8.8, 2.6 Hz, 1H), 6.65 - 6.68 (m, 2H), 7.50 (s, 1H), 7.59 (s, 1H), 8.00 (d, J = 8.8 Hz, 1H), 8.63 (s, 1H), 13.23 (s, 1H).

### Example 41. Preparation of N⁴-cyclohexyl-3-(1H-imidazol-5-yl)-N⁶-(2-methoxy-4-morpholinophenyl)-1H-pyrazolo[3,4-d]pyrimidine-4,6-diamine (Compound 18-2)

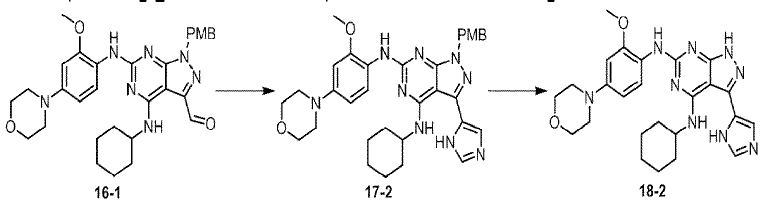

### Step 1. Synthesis of Compound 17-2

Compound **16-1** (50 mg, 0.0875 mmol), p-toluenesulfonylmethyl isocyanide (TosMIC; 19 mg, 0.095 mmol), and K₂CO₃ (36 mg, 0.262 mmol) were added to MeOH (2 mL), and stirred at 50°C for 1 hour. EA (5 mL) was added to the mixture, followed by washing with water (5 mL), moisture removal (Na₂SO₄), and concentration under reduced pressure. Ammonia (7 M in MeOH; 1 mL) was added to the residue, and stirred under reflux for 4 hours to obtain crude Compound **17-2** (9 mg).

¹H NMR (300 MHz, Chloroform-d) δ 1.26 (s, 4H), 1.50 (s, 5H), 1.83 (s, 3H), 2.11 (s, 2H), 3.06 - 3.18 (m, 4H), 3.75 (s, 3H), 3.89 (t, J = 4.7 Hz, 4H), 3.91 (s, 3H), 5.40 (s, 2H), 6.54 (dd, J = 4.7, 2.3 Hz, 2H), 6.82 (d, J = 8.6 Hz, 2H), 7.28 - 7.38 (m, 3H), 7.60 (dd, J = 2.1, 1.3 Hz, 1H), 7.68 (d, J = 1.3 Hz, 1H), 8.60 (d, J = 9.5 Hz, 1H), 9.26 (s, 1H), 10.28 (d, J = 7.5 Hz, 1H).

### Step 2. Preparation of Compound 18-2

Compound **17-2** (9 mg) was subjected to the same preparation method as in Compound **8-1** to obtain Compound **18-2** as a light gray solid (1.7 mg).

¹H NMR (400 MHz, Chloroform-d) δ 1.51 (t, J = 9.1 Hz, 3H), 1.84 (s, 5H), 2.11 (s, 3H), 3.12 (t, J = 4.8 Hz, 3H), 3.89 (d, J = 4.0 Hz, 5H), 4.22 (s, 1H), 6.54 (s, 1H), 7.30 (s, 1H), 7.64 (s, 1H), 7.72 (s, 1H), 8.44 (d, J = 8.8 Hz, 1H), 9.38 (s, 1H), 10.42 (s, 1H).

### <Example E. Preparation of Compound 21 by Reaction Scheme 5>

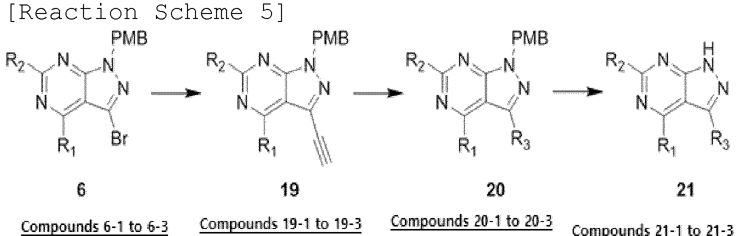

### Example 42. Preparation of N⁴-cyclohexyl-N⁶-(2-methoxy-4-morpholinophenyl)-3-(1H-1,2,3-triazol-4-yl)-1H-pyrazolo[3,4-d]pyrimidine-4,6-diamine (Compound 21-1)

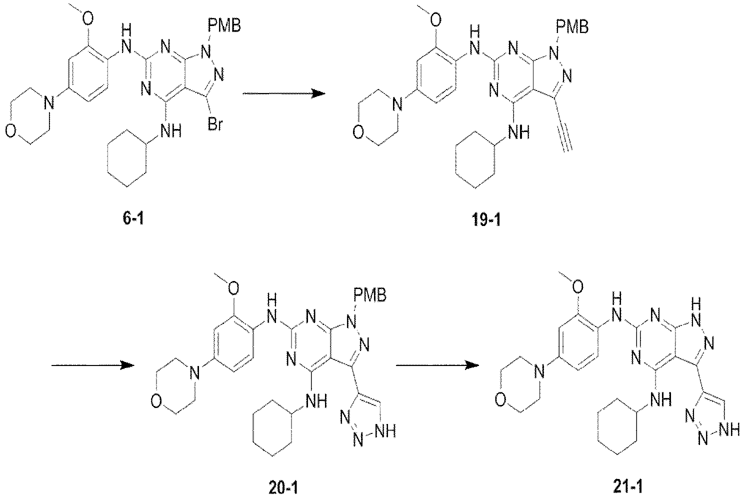

The target compound was prepared from Compound **6-1** through three steps by Reaction Scheme 5.

### Step 1. Synthesis of Compound 19-1

Compound **6-1** (1.87 g, 3.003 mmol) and cuprous iodide (CuI; 57 mg, 10% mmol) were dissolved in dimethylformamide (DMF; 25 mL) and triethylamine (TEA; 5 mL), and then trimethylsilylactylene (0.6 mL, 4.2051 mmol) was added. The mixture was purged with argon gas to remove oxygen, and PdCl₂(PPh₃)₂ (105 mg, 5%mmol) was added to remove gas, followed by stirring at 80°C for 2 hours. The mixture was concentrated under reduced pressure, and then the residue was separated by silica gel column chromatography (EA:HX = 1:2). The obtained compound was dissolved in tetrahydrofuran (20 mL), and tetra-n-butylammonium fluoride (TBAF; 1.3 mL, 1.2 eq) was added, followed by stirring at room temperature for 1 hour. The mixture was concentrated under reduced pressure, and then ethyl acetate (50 mL) was added. The mixture was washed with water (200 mL), subjected to moisture removal (Na₂SO₄), and concentrated under reduced pressure. The residue was separated by silica gel column chromatography (EA:HX = 1:2) to obtain Compound **19-1** as a pale brown solid (1.18 g, 69%)

¹H NMR (300 MHz, DMSO-d₆) δ 1.23 (d, J = 2.8 Hz, 2H), 1.36 (d, J = 12.2 Hz, 8H), 1.70 (s, 3H), 1.96 (s, 3H), 3.09 (t, J = 4.8 Hz, 4H), 3.71 (s, 3H), 3.75 (t, J = 4.8 Hz, 4H), 3.85 (s, 3H), 4.03 (s, 1H), 4.74 (s, 1H), 5.27 (s, 2H), 5.77 (s, 1H), 6.00 (d, J = 7.7 Hz, 1H), 6.48 - 6.54 (m, 1H), 6.66 (d, J = 2.5 Hz, 1H), 6.89 (d, J = 8.6 Hz, 2H), 7.21 (d, J = 8.6 Hz, 2H), 7.61 (s, 1H), 8.08 (d, J = 8.9 Hz, 1H).

### Step 2. Synthesis of Compound 20-1

Compound **19-1** (114 mg, 0.15 mmol) and cuprous iodide (CuI; 4 mg, 10% mmol) were dissolved in dimethylformamide (DMF):methanol (MeOH) =9:1 (2 mL), and then trimethylsilyl azide(0.040 mL, 0.3 mmol) was added, followed by stirring at 100°C for 6 hours. The mixture was concentrated under reduced pressure, and then EA (10 mL) was added. The mixture was washed with water (10 mL), subjected to moisture removal (Na₂SO₄), and concentrated under reduced pressure. The residue was separated by silica gel column chromatography (EA:HX = 2:3) to obtain Compound **20-1** as a pale brown solid (87 mg, 76%)

¹H NMR (400 MHz, DMSO-d₆) δ 1.44 (d, J = 8.4 Hz, 5H), 1.59 (s, 1H), 1.77 (s, 2H), 2.01 (s, 2H), 2.19 (s, 1H), 3.06 - 3.11 (m, 4H), 3.70 (s, 3H), 3.75 (dd, J = 5.9, 3.6 Hz, 4H), 3.87 (s, 3H), 4.14 (s, 1H), 5.34 (s, 2H), 6.51 (dd, J = 8.9, 2.5 Hz, 1H), 6.67 (d, J = 2.1 Hz, 1H), 6.89 (d, J = 9.0 Hz, 3H), 7.19 - 7.25 (m, 2H), 7.45 (s, 1H), 8.23 (d, J = 8.8 Hz, 1H), 8.43 (s, 1H), 9.52 (s, 1H).

### Step 3. Preparation of Compound 21-1

Compound **20-1** (87 mg, 0.1424mmol) was subjected to the same preparation method as in Compound **8-1** to obtain Compound **21-1** as a white solid (48 mg, 68%).

¹H NMR (300 MHz, DMSO-d₆) δ 1.43 (d, J = 8.7 Hz, 5H), 1.79 (s, 3H), 1.96 - 2.06 (m, 3H), 3.09 (t, J = 4.8 Hz, 4H), 3.73 - 3.78 (m, 4H), 3.86 (s, 3H), 3.96 (s, 1H), 4.12 (s, 1H), 6.49 (dd, J = 8.9, 2.5 Hz, 1H), 6.66 (d, J = 2.6 Hz, 1H), 7.36 (s, 1H), 8.12 (d, J = 8.8 Hz, 1H), 8.68 - 8.76 (m, 1H), 9.66 (d, J = 7.4 Hz, 1H), 12.98 (s, 1H).

### Example 43. Preparation of N⁴-cyclohexyl-N⁶-(2-methoxy-4-morpholinophenyl)-3-(1-methyl-1H-1,2,3-triazol-4-yl)-1H-pyrazolo[3,4-d]pyrimidine-4,6-diamine (Compound 21-2)

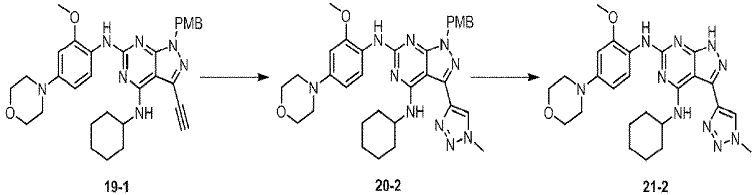

### Step 1. Synthesis of Compound 20-2

Compound **19-1** (400 mg, 0.7046 mmol) was dissolved in a mixture, obtained by adding and dissolving sodium azide (100 mg) and iodomethane (0.1 mL) in dimethyl sulfoxide (20 mL) followed by stirring for 12 hours, and then copper sulfate pentahydrate (14 mg, 8%mmol) and sodium L-ascorbate (27 mg, 20%mmol) were added, followed by stirring at room temperature for 12 hours. Ethyl acetate (100 mL) was added to the mixture, followed by washing with water (100 mL), moisture removal (Na₂SO₄), and concentration under reduced pressure. The residue was separated by silica gel column chromatography (EA:HX = 1:1) to obtain Compound **20-2** as a pale brown solid (74%)

¹H NMR (400 MHz, DMSO-d₆) δ 1.40 - 1.62 (m, 6H), 1.77 (s, 3H), 1.97 (s, 2H), 2.55 (s, 1H), 3.09 (s, 4H), 3.70 (s, 3H), 3.75 (d, J = 4.9 Hz, 3H), 3.87 (s, 3H), 4.14 (s, 4H), 5.33 (s, 2H), 6.52 (d, J = 8.6 Hz, 1H), 6.66 (d, J = 3.5 Hz, 1H), 6.89 (d, J = 8.3 Hz, 2H), 7.22 (d, J = 8.3 Hz, 2H), 7.45 (s, 1H), 8.23 (d, J = 8.8 Hz, 1H), 8.58 (s, 1H), 9.74 (d, J = 7.2 Hz, 1H).

### Step 2. Preparation of Compound 21-2

Compound **20-2** (327 mg, 0.5234 mmol) was subjected to the same preparation method as in Compound **8-1** to obtain Compound **21-2** as a pale brown solid (59%)

¹H NMR (400 MHz, DMSO-d₆) δ 1.44 (d, J = 9.5 Hz, 5H), 1.57 (s, 1H), 1.78 (s, 3H), 1.98 (d, J = 9.2 Hz, 3H), 3.08 (d, J = 5.0 Hz, 4H), 3.75 (d, J = 4.9 Hz, 4H), 3.86 (d, J = 1.8 Hz, 3H), 4.15 (d, J = 1.8 Hz, 3H), 6.49 (d, J = 8.9 Hz, 1H), 6.65 (s, 1H), 7.36 (s, 1H), 8.11 (d, J = 8.8 Hz, 1H), 8.56 (d, J = 1.8 Hz, 1H), 9.66 (d, J = 7.5 Hz, 1H), 12.94 (s, 1H).

### Example 44. Preparation of N⁶-(2-methoxy-4-morpholinophenyl)-3-(1-methyl-1H-1,2,3-triazol-4-yl)-N⁴-(tetrahydro-2H-pyran-4-yl)-1H-pyrazolo[3,4-d]pyrimidine-4,6-diamine (Compound 21-3)

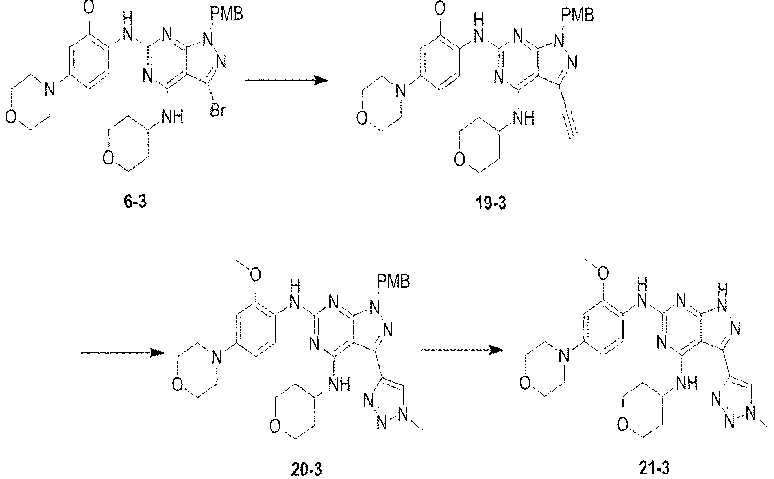

### Step 1. Synthesis of Compound 19-3

Compound **6-3** (1.87 g, 2.9942 mmol) was subjected to the same preparation method as in Compound **19-1** to obtain Compound **19-3** as a pale brown solid (64%).

¹H NMR (500 MHz, DMSO-d₆) δ 1.23 (d, J = 2.1 Hz, 2H), 1.34 (s, 1H), 1.55 - 1.64 (m, 2H), 1.88 - 1.98 (m, 3H), 3.09 (t, J = 4.7 Hz, 5H), 3.71 (s, 3H), 3.75 (t, J = 4.8 Hz, 5H), 3.84 (s, 3H), 3.87 (d, J = 13.3 Hz, 3H), 4.22 (s, 1H), 4.69 (s, 1H), 5.27 (s, 2H), 6.11 (s, 1H), 6.52 (dd, J = 8.9, 2.6 Hz, 1H), 6.65 (d, J = 2.5 Hz, 1H), 6.89 (d, J = 8.4 Hz, 2H), 7.21 (d, J = 8.3 Hz, 2H), 7.65 (d, J = 3.9 Hz, 2H), 8.04 (s, 1H).

### Step 2. Synthesis of Compound 20-3

Compound **19-3** (57 mg, 0.1000mmol) was subjected to the same preparation method as in Compound **20-2** to obtain Compound **20-3** as a white solid (32 mg, 51.6%).

¹H NMR (300 MHz, DMSO-d₆) δ 1.23 (d, J = 2.7 Hz, 5H), 1.34 (s, 1H), 1.54 - 1.60 (m, 2H), 2.04 (s, 3H), 3.09 (t, J = 4.9 Hz, 4H), 3.70 (s, 3H), 3.74 (d, J = 5.0 Hz, 4H), 3.86 (s, 3H), 4.14 (s, 3H), 5.33 (s, 2H), 6.53 (d, J = 8.7 Hz, 1H), 6.66 (d, J = 2.6 Hz, 1H), 6.86 - 6.90 (m, 2H), 7.22 (d, J = 8.2 Hz, 2H), 7.47 (s, 1H), 7.69 (s, 1H), 8.18 (d, J = 8.7 Hz, 1H), 8.58 (s, 1H), 9.77 (d, J = 7.2 Hz, 1H).

### Step 3. Preparation of Compound 21-3

Compound **20-3** (32 mg, 0.0510mmol) was subjected to the same preparation method as in Compound **8-1** to obtain Compound **21-3** as a pale brown solid (19.4 mg, 77.6%).

¹H NMR (300 MHz, DMSO-d₆) δ 1.09 (t, J = 7.0 Hz, 1H), 1.57 (dq, J = 8.3, 4.5, 3.7 Hz, 2H), 2.05 (d, J = 12.1 Hz, 3H), 3.09 (t, J = 4.8 Hz, 4H), 3.38 (q, J = 7.0 Hz, 1H), 3.47 - 3.63 (m, 3H), 3.75 (t, J = 4.8 Hz, 4H), 3.85 (s, 3H), 3.86 - 3.96 (m, 3H), 4.16 (s, 3H), 4.33 (s, 1H), 6.50 (dd, J = 8.8, 2.5 Hz, 1H), 6.65 (d, J = 2.5 Hz, 1H), 7.41 (s, 1H), 8.07 (d, J = 8.7 Hz, 1H), 8.56 (s, 1H), 9.72 (d, J = 7.2 Hz, 1H), 12.97 (s, 1H).

### <Example F. Preparation of Compound 26 by Reaction Scheme 6>

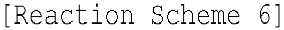

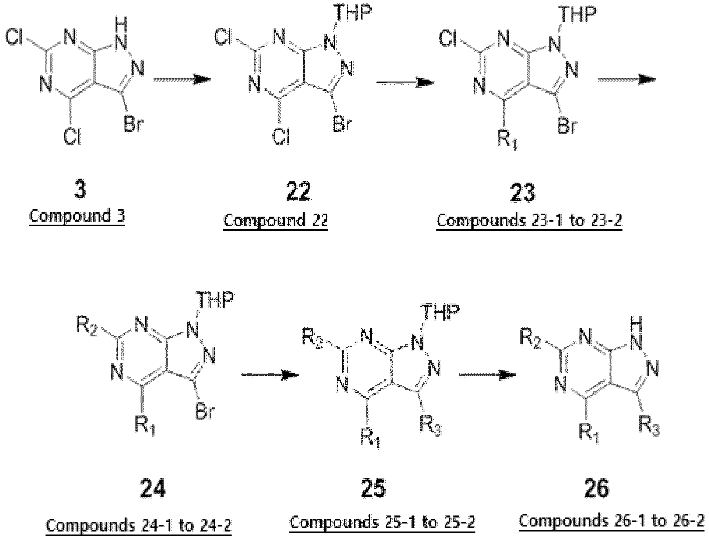

### Example 45. Preparation of N⁴-cyclohexyl-N⁶-(2-isopropoxy-4-morpholinophenyl)-3-(1-methyl-1H-pyrazol-4-yl)-1H-pyrazolo[3,4-d]pyrimidine-4,6-diamine (Compound 26-1)

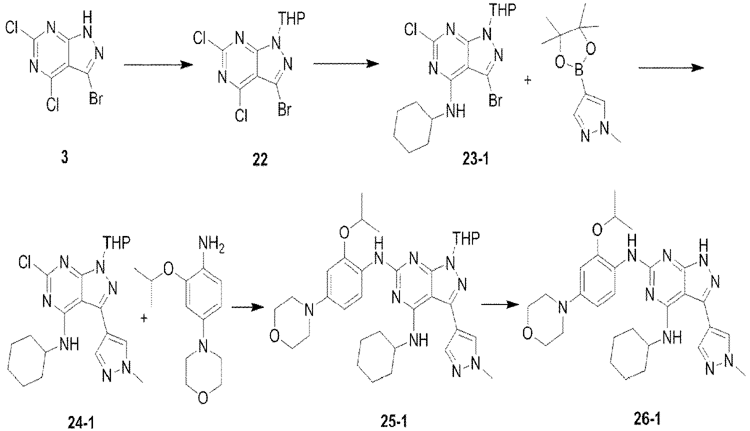

### Step 1. Synthesis of Compound 22

Compound **3** (10.75 g, 40.43 mmol) was dissolved in MC-THF (1:1 in v/v, 300 mL), and then 1,3-dihydropyran (30 mL, 328.8 mmol) and PTSA (2.01 g, 10.6 mmol) were added, followed by stirring at room temperature for 15 hours. The reaction product was concentrated under reduced pressure, dissolved in EA (200 mL), washed with saturated NaHCO₃ (100 mL) and salt water (100 mL), subjected to moisture removal (Mg₂SO₄), and concentrated under reduced pressure, to obtain Compound **22** as a white solid (11.77 g, 83.2%).

¹H NMR(300MHz, CDCl₃) δ 1.61-1.83 (m, 3H), 1.93-2.18 (m, 2H), 2.39-2.45 (m,1 H), 3.78-3.85 (m, 1H), 4.10-4.19 (m, 1H), 6.01 (dd, J = 8.9, 3.0Hz, 1H), 8.20 (s, 1H).

### Step 2. Synthesis of Compound 23-1

Compound **22** (100 mg, 0.286 mmol) was dissolved in THF/IPA (1:4, 2 mL), and then cyclohexylamine (0.052 mL, 0.457 mmol) was added, followed by stirring at room temperature for 4 hours. The reaction product was concentrated under reduced pressure, and then separated by silica gel column chromatography (EA:Hx = 1:10) to obtain Compound **23-1** as a white solid (91.1 mg, 77.1 %).

¹H NMR (400 MHz, CDCl₃) δ 6.11 (d, J = 8.2 Hz, 1H), 5.84 (dd, J = 10.6, 2.5 Hz, 1H), 4.30 - 4.17 (m, 1H), 4.09 (dp, J = 11.8, 1.9 Hz, 1H), 3.76 (td, J = 11.6, 2.5 Hz, 1H), 2.44 (tdd, J = 12.6, 10.5, 4.2 Hz, 1H), 2.06 (ddt, J = 12.8, 9.1, 3.7 Hz, 3H), 1.91 - 1.82 (m, 1H), 1.81 - 1.61 (m, 5H), 1.61 - 1.58 (m, 1H), 1.49 (dtt, J = 14.1, 11.0, 3.4 Hz, 2H), 1.39 - 1.22 (m, 3H).

### Step 3. Synthesis of Compound 24-1

Compound **23-1** (200 mg, 0.483 mmol), 1-methylpyrazole-4-boronic acid (80.98 mg, 0.642 mmol), and K₂CO₃ (133.5 mg, 0.966 mmol) were dissolved in dioxane:water (4:1 in v/v, 2 mL), and then purged with argon gas to remove oxygen. Thereafter, Pd(PPh₃)₄ (18.4 mg, 0.0159 mmol) was added, followed by stirring in a microwave reactor at 120°C for 1 hour. Water (20 mL) was added to the mixture, followed by extraction with EA (20 mL X2). The organic layer was subjected to moisture removal (Na₂SO₄), filtered, and then concentrated under reduced pressure. The residue was separated by silica gel column chromatography (EA:Hx = 1:5) to obtain Compound **24-1** as a white solid (64.7 mg, 32.2 %).

¹H NMR (400 MHz, CDCl₃) δ 8.17 (q, J = 5.7, 5.0 Hz, 1H), 8.08 (q, J = 6.4, 4.7 Hz, 1H), 5.96 (dq, J = 11.2, 4.8, 3.9 Hz, 1H), 5.50 (tt, J = 7.7, 4.0 Hz, 1H), 4.13 (d, J = 11.9 Hz, 1H), 3.97 (t, J = 4.2 Hz, 3H), 3.88 - 3.69 (m, 1H), 2.57 (q, J = 11.3 Hz, 1H), 2.18 - 2.10 (m, 1H), 1.97 - 1.82 (m, 5H), 1.67 - 1.56 (m, 4H), 1.51 (dt, J = 11.0, 5.5 Hz, 3H), 1.24 (d, J = 3.4 Hz, 4H).

### Step 4. Synthesis of Compound 25-1

Compound **24-1** (32 mg, 0.077 mmol) was dissolved in 2-butanol (1 mL), and then 2-isopropoxy-4-morpholinoaniline (21.7 mg, 0.092 mmol) and K₂CO₃ (17.0 mg, 0.123 mmol) were added. The mixture was purged with argon gas for 5 minutes to remove oxygen, and Pd₂dba₃ (17.6 mg, 0.019 mmol) and XPhos (13.5 mg, 0.028 mmol) were added, followed by stirring at 110°C for one day. The reaction product was filtered (through cellite pad) and concentrated under reduced pressure. The residue was separated by silica gel column chromatography (MC:MeOH =99:1) to obtain Compound **25-1** as a white solid (36.3 mg, 76.7%).

¹H NMR (300 MHz, CDCl₃) δ 8.59 - 8.49 (m, 1H), 7.76 (d, J = 3.0 Hz, 2H), 7.42 (s, 1H), 6.54 (d, J = 7.1 Hz, 2H), 5.84 (dd, J = 10.7, 2.4 Hz, 1H), 5.36 - 5.21 (m, 1H), 4.67 - 4.52 (m, 1H), 4.15 (d, J = 11.1 Hz, 2H), 3.98 (s, 3H), 3.92 - 3.84 (m, 4H), 3.82 (d, J = 10.7 Hz, 1H), 3.17 - 3.06 (m, 4H), 2.58 (dd, J = 16.1, 7.0 Hz, 1H), 2.08 (s, 3H), 1.91 (d, J = 13.1 Hz, 1H), 1.70 (d, J = 37.7 Hz, 9H), 1.42 (d, J = 6.1 Hz, 8H), 1.32 - 1.12 (m, 5H).

### Step 5. Preparation of Compound 26-1

Compound **25-1** (36 mg, 0.058 mmol) was dissolved in MeOH (2 mL), and then 6 N HCl (0.1 mL) was added at 0°C. Thereafter, the temperature was raised to room temperature, and the mixture was stirred at room temperature for 2 hours. The reaction product was concentrated under reduced pressure, then an aqueous solution of saturated NaHCO₃ (20 mL) was added, followed by extraction with ethyl acetate (20 mL × 2). The organic layer was subjected to moisture removal (Na₂SO₄) and concentrated under reduced pressure. The residue was separated by silica gel column chromatography (MC:MeOH = 97:3) to obtain compound **26-1** as a white solid (16.6 mg, 53.4 %).

¹H NMR (300 MHz, DMSO-d₆) δ 12.77 (s, 1H), 8.19 (d, J = 8.8 Hz, 1H), 8.04 (s, 1H), 7.72 (s, 1H), 7.31 (s, 1H), 6.66 (d, J = 2.5 Hz, 1H), 6.49 (dd, J = 8.9, 2.5 Hz, 1H), 5.76 (s, 1H), 5.67 (d, J = 7.5 Hz, 1H), 4.65 (p, J = 6.0 Hz, 1H), 4.00 (s, 1H), 3.93 (s, 3H), 3.74 (dd, J = 6.0, 3.5 Hz, 4H), 3.05 (t, J = 4.8 Hz, 4H), 1.99 (d, J = 10.2 Hz, 2H), 1.65 (s, 2H), 1.31 (t, J = 6.2 Hz, 11H).

### Example 46. Preparation of N⁶-(2-methoxy-4-morpholinophenyl)-3-(1-methyl-1H-pyrazol-4-yl)-N⁴-phenyl-1H-pyrazolo[3,4-d]pyrimidine-4,6-diamine (Compound 26-2)

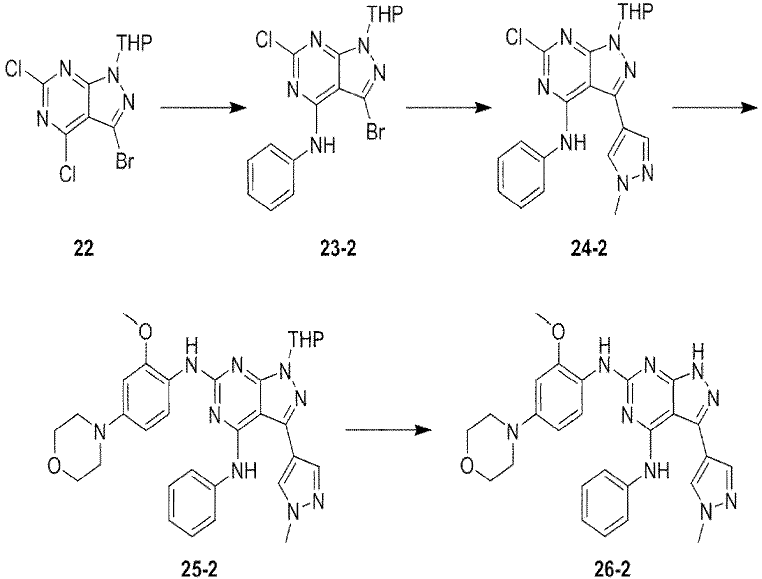

### Step 1. Synthesis of Compound 23-2

Compound **22** (200 mg, 0.57 mmol) was dissolved in isopropyl alcohol (IPA; 2.0 mL), and then aniline (0.06 mL, 0.68 mmol) and N,N-diisopropylethylamine (0.1 mL, 0.68 mmol) were added, followed by stirring at 40°C for 1 hour. After completion of the reaction, the produced solid was filtered to obtain Compound **23-2** as a white solid (175 mg, 75.4%).

¹H NMR (500 MHz, CDCl₃) δ 1.62 (s, 2H), 1.67 - 1.85 (m, 2H), 1.86 - 1.99 (m, 1H), 2.05 - 2.18 (m, 1H), 2.48 (tdd, J = 12.7, 10.4, 4.2 Hz, 1H), 3.78 (td, J = 11.7, 2.6 Hz, 1H), 4.06 - 4.19 (m, 1H), 5.91 (dd, J = 10.6, 2.6 Hz, 1H), 7.20 (t, J = 7.4 Hz, 1H), 7.37 - 7.48 (m, 2H), 7.71 (d, J = 8.0 Hz, 2H), 7.94 (s, 1H).

### Step 2. Synthesis of Compound 24-2

Compound **23-2** (175 mg, 0.43 mmol), 1-methylpyrazole-4-boronic acid (108.4 mg, 0.86 mmol), and potassium carbonate (K₂CO₃, 237.7 mg, 1.72 mmol) were dissolved in dioxane:H₂O (4:1 in v/v, 2 mL), and then purged with argon gas to remove oxygen. Thereafter, Pd(PPh₃)₄ (32.8 mg, 0.028 mmol) was added to remove gas, followed by stirring in a microwave reactor at 110°C for 1 hour. Water (10 mL) was added to the mixture, followed by extraction with ethyl acetate (20 mL X2). The combined organic layer was filtered over (Na₂SO₄) and concentrated under reduced pressure. The mixture was subjected to silica gel column chromatography (ethyl acetate:hexane = 1:1) to obtain Compound **24-2** as a white solid (71.2 mg, 40.4 %)

¹H NMR (400 MHz, CDCl₃) δ 1.61 (d, J = 8.8 Hz, 1H), 1.72 - 1.84 (m, 2H), 1.93 (d, J = 13.6 Hz, 1H), 2.10 (d, J = 5.0 Hz, 1H), 2.49 - 2.61 (m, 1H), 3.83 (td, J = 11.6, 2.4 Hz, 1H), 4.04 (s, 3H), 4.12 - 4.18 (m, 1H), 5.98 (dd, J = 10.8, 2.5 Hz, 1H), 7.15 (tt, J = 7.1, 1.1 Hz, 1H), 7.37 (tt, J = 7.5, 2.1 Hz, 2H), 7.43 - 7.49 (m, 1H), 7.59 - 7.65 (m, 2H), 7.82 - 7.87 (m, 2H).

### Step 3. Synthesis of Compound 25-2

Compound **24-2** (71.2 mg, 0.174 mmol) was dissolved in 2-butanol (2 mL), and then aniline (46.02 mg, 0.208 mmol) and pottasium carbonate (38.4 mg, 0.278 mmol) were added. The mixture was purged with argon gas for 5 minutes to remove oxygen, and tris(dibenzylideneacetone)dipalladium(0) (39.3 mg, 0.043 mmol) and XPhos (28.6 mg, 0.06 mmol) were added, followed by stirring at 110°C for 1 hour. The mixture was filtered through a cellite pad and then concentrated under reduced pressure. The residue was subjected to silica gel column chromatography (ethyl acetate:hexane = 1:1) to obtain Compound **25-2** as a white solid (38.7 mg, 38.2 %)

¹H NMR (400 MHz, DMSO) δ 4.23 (s, 3H), 7.32 (s, 1H), 7.46 (d, J = 7.6 Hz, 2H), 8.01 (s, 1H), 8.33 (d, J = 1.7 Hz, 1H), 8.86 (s, 1H), 11.03 (s, 1H).

### Step 4. Preparation of Compound 26-2

Compound **25-2** (38.7 mg, 0.066 mmol) was dissolved in methanol (2 mL), and then 6 N HCl (0.1 mL) was added at 0°C. Thereafter, the temperature was raised to room temperature, and the mixture was stirred at room temperature for 1 hour. After completion of the reaction, methanol was blown off, and then sodium bicarbonate (10 mL) was added to the mixture, followed by extraction with ethyl acetate (20 mL X2). The residue was subjected to silica gel column chromatography (dichloromethane:methanol = 19:1) to obtain Compound **26-2** as a white solid (17.5 mg, 53.3 %)

¹H NMR (400 MHz, DMSO) δ 3.11 (dd, J = 5.7, 3.9 Hz, 4H), 3.72 - 3.83 (m, 7H), 3.94 (s, 3H), 6.47 (dd, J = 8.7, 2.6 Hz, 1H), 6.65 (d, J = 2.6 Hz, 1H), 7.06 (d, J = 7.5 Hz, 1H), 7.30 (t, J = 7.9 Hz, 2H), 7.64 (d, J = 7.8 Hz, 2H), 7.79 (q, J = 13.7, 12.9 Hz, 4H), 8.14 (s, 1H), 12.93 (s, 1H).

### <Example G. Preparation of Compound 28 by Reaction Scheme 7>

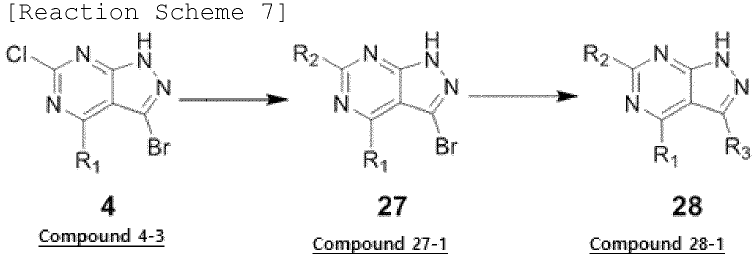

### Example 47. Preparation of N⁶-(2-methoxy-4-morpholinophenyl)-3-(1-methyl-1H-pyrazol-4-yl)-N⁴-(1-methylpiperidin-4-yl)-1H-pyrazolo[3,4-d]pyrimidine-4,6-diamine(Compound 28-1)

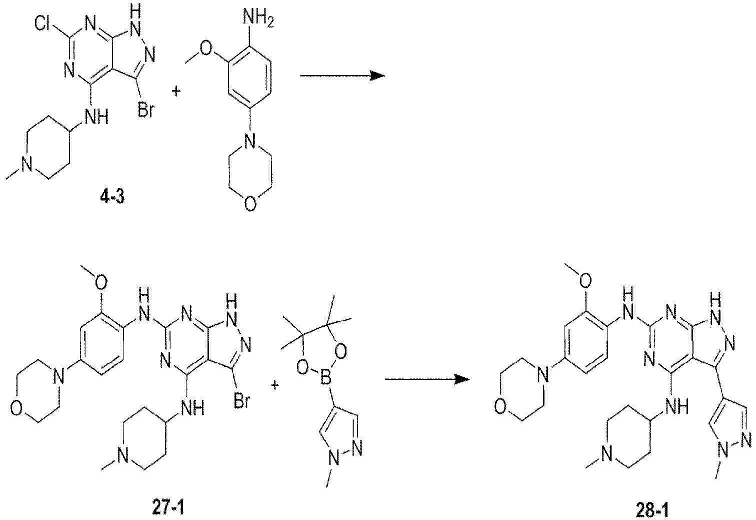

### Step 1. Synthesis of Compound 27-1

TFA (44.2 µL) was added to 2-butanol (2-BuOH; 5 mL), and then 2-methoxy-4-morpholinoaniline (126 mg, 0.60 mmol in THF 1 mL) was added dropwise at room temperature for 3 minutes. Compound **4-3** (190 mg, 0.54 mmol in THF 3 mL) was added dropwise to the mixture for 3 minutes, and stirred at 110°C for 14 hours. The pH of the reaction mixture was adjusted to 7 by an aqueous solution of sat'd NaHCO₃, and then EA (8 mL) was added. The mixture was wahsed with water (3 mL), subjected to moisture removal (Na₂SO₄), and then concentrated under reduced pressure. The residue was separated by silica gel column chromatography (MC:MeOH = 10:1) to obtain Compound **27-1** (21 mg, 7.3%)

¹H NMR (500 MHz, DMSO-d₆) δ 1.19-1.26 (m, 1H), 1.31-1.46 (m, 4H), 1.56-1.63 (m, 1H), 1.68-1.75 (m, 2H), 1.90-1.96 (m, 2H), 3.06-3.11 (m, 4H), 3.71 (s, 3H), 3.73-3.76 (m, 4H), 3.84 (s, 3H), 3.99-4.07 (m, 1H), 5.23 (s, 2H), 6.09 (d, J = 7.9 Hz, 1H), 6.50 (dd, J = 8.9, 2.6 Hz, 1H), 6.65 (d, J = 2.6 Hz, 1H), 6.89 (d, J = 8.6 Hz, 2H), 7.20 (d, J = 8.6 Hz, 2H), 7.65 (s, 1H), 8.02 (d, J = 8.8 Hz, 1H).

### Step 2. Preparation of Compound 28-1

Compound **27-1** (20 mg, 0.03 mmol) and boronate (13 mg, 0.05 mmol) were subjected to the same preparation method as in Compound **7-1** to obtain Compound **28-1** as a pale brown solid (11.3 mg, 56%).

¹H NMR (500 MHz, DMSO-d₆) δ 1.30-1.46 (m, 2H), 2.04-2.14 (m, 3H), 2.42-2.49 (m, 2H), 2.52-2.57 (m, 1H), 2.72 (s, 3H), 3.55-3.59 (m, 4H), 4.22-4.26 (m, 4H), 4.40 (s, 3H), 4.46 (s, 3H), 4.58-4.65 (m, 1H), 6.09 (d, J = 7.5 Hz, 1H), 7.01 (dd, J = 8.9, 2.6 Hz, 1H), 7.18 (d, J = 2.6 Hz, 1H), 7.80 (s, 1H), 8.19 (s, 1H), 8.42 (s, 1H), 8.94 (d, J = 8.8 Hz, 1H).

### <Example H. Preparation of Compound 33 by Reaction Scheme 8>

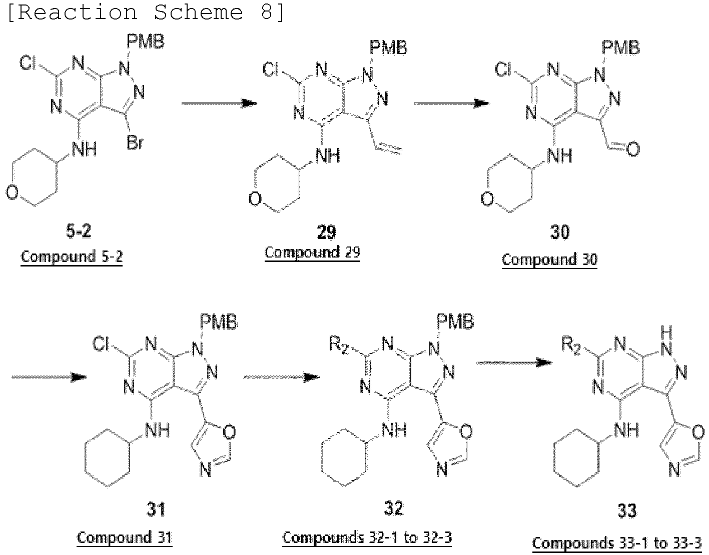

### Example 48. Preparation of N⁶-(2-methoxy-4-morpholinophenyl)-3-(oxazol-5-yl)-N⁴-(tetrahydro-2H-pyran-4-yl)-1H-pyrazolo[3,4-d]pyrimidine-4,6-diamine(Compound 33-1)

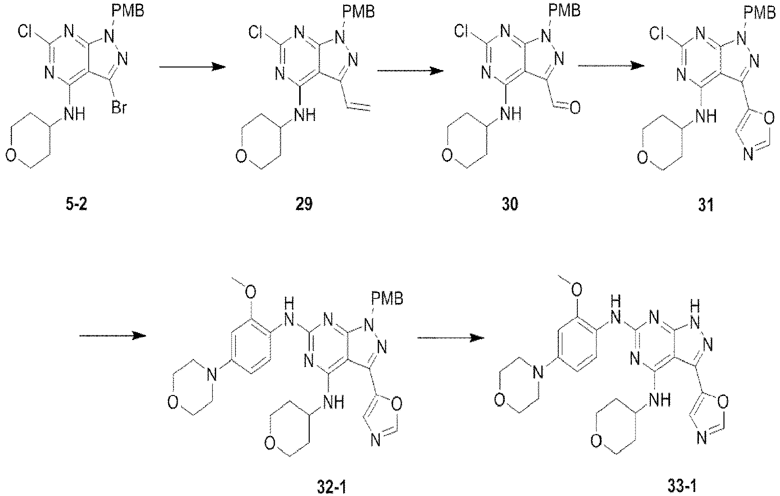

### Step 1. Preparation of Compound 29

Compound **5-2** (1.0 g, 2.208 mmol) was subjected to the same preparation method as in Compound **16-1** in Example 40 to obtain Compound **29** (633 mg, 71%).

¹H NMR (300 MHz, DMSO-d₆) δ 7.37 - 7.33 (m, 1H), 7.33 - 7.24 (m, 1H), 7.18 (d, J = 8.7 Hz, 2H), 6.91 - 6.85 (m, 2H), 6.02 (dd, J = 17.1, 1.9 Hz, 1H), 5.50 - 5.41 (m, 1H), 5.37 (s, 2H), 4.37 (d, J = 8.6 Hz, 1H), 3.95 - 3.86 (m, 2H), 3.71 (s, 3H), 3.47 - 3.36 (m, 2H), 1.79 (d, J = 5.7 Hz, 4H).

### Step 2. Preparation of Compound 30

Compound **29** (623 mg, 1.557 mmol) was subjected to the same preparation method as in Compound **16-1** in Example 40 to obtain Compound **30** (480 mg, 77%).

¹H NMR (300 MHz, DMSO-d₆) δ 9.94 (s, 1H), 8.39 (d, J = 7.6 Hz, 1H), 7.27 (d, J = 8.7 Hz, 2H), 6.91 (d, J = 8.7 Hz, 2H), 5.53 (s, 2H), 4.26 (dd, J = 7.4, 3.6 Hz, 1H), 3.88 (dt, J = 11.7, 4.0 Hz, 2H), 3.52 (ddd, J = 12.0, 10.4, 2.5 Hz, 2H), 2.01 (d, J = 8.5 Hz, 2H), 1.63 - 1.50 (m, 2H).

### Step 3. Preparation of Compound 31

Compound **30** (100 mg, 0.2488 mmol) was subjected to the same preparation method as in Compound **17-1** in Example 40 to obtain Compound 31 (65 mg, 60%).

¹H NMR (300 MHz, DMSO-d₆) δ 8.66 (s, 1H), 7.67 (s, 1H), 7.35 (d, J = 7.6 Hz, 1H), 7.23 - 7.18 (m, 2H), 6.92 - 6.87 (m, 2H), 5.45 (s, 2H), 4.31 (d, J = 10.4 Hz, 1H), 3.90 (d, J = 11.7 Hz, 2H), 3.49 (d, J = 2.3 Hz, 2H), 1.94 (d, J = 12.6 Hz, 2H), 1.77 - 1.63 (m, 2H).

### Step 4. Preparation of Compound 32-1

Compound **31** (50 mg, 0.1134 mmol), 2-methoxy-4-morpholinoaniline (28 mg, 0.136 mmol), potassium carbonate (23 mg, 0.1701 mmol), and XPhos (19 mg, 0.039 mmol) were added to 2-butanol (1 mL), followed by oxygen removal. Thereafter, Pd(dba)₃ (225 mg, 0.028 mmol) was added, and then the mixture was stirred at 110°C for 12 hours. The mixture was filtered through a cellite pad and washed with EA (20 mL), and then the organic layer was washed with water (10 mL), subjected to moisture removal (Na₂SO₄), and concentrated under reduced pressure. The residue was separated by silica gel column chromatography (EA:HX = 2:1) to obtain Compound **32-1** (19 mg, 27%).

¹H NMR (500 MHz, DMSO-d₆) δ 8.62 (d, J = 2.1 Hz, 1H), 8.07 (s, 1H), 7.63 (s, 1H), 7.58 (d, J = 2.2 Hz, 1H), 7.22 (d, J = 8.2 Hz, 2H), 6.88 (d, J = 8.2 Hz, 2H), 6.79 (d, J = 7.3 Hz, 1H), 6.65 (s, 1H), 6.52 (d, J = 8.6 Hz, 1H), 5.34 (s, 2H), 4.25 (s, 1H), 3.91 (s, 2H), 3.85 (s, 3H), 3.75 (s, 4H), 3.70 (d, J = 2.0 Hz, 3H), 3.46 (s, 2H), 3.09 (s, 4H), 1.97 (d, J = 12.6 Hz, 3H), 1.65 (d, J = 11.4 Hz, 2H).

### Step 5. Preparation of Compound 33-1

Compound **32-1** (10 mg, 0.0163 mmol) was subjected to the same preparation method as in Compound **8-1** to obtain Compound **33-1** (4 mg, 50%).

¹H NMR (300 MHz, DMSO-d₆) δ 13.26 (s, 1H), 8.62 (s, 1H), 7.96 (d, J = 8.7 Hz, 1H), 7.59 (s, 1H), 7.55 (s, 1H), 6.71 (d, J = 7.4 Hz, 1H), 6.65 (d, J = 2.6 Hz, 1H), 6.50 (d, J = 8.4 Hz, 1H), 4.28 (s, 1H), 3.91 (d, J = 11.7 Hz, 2H), 3.84 (s, 3H), 3.76 (s, 4H), 3.48 (s, 2H), 3.10 (t, J = 4.9 Hz, 4H), 2.00 (d, J = 12.7 Hz, 3H), 1.73 - 1.62 (m, 3H).

### Example 49. Preparation of 3-methoxy-N-methyl-4-((3-(oxazol-5-yl)-4-((tetrahydro-2H-pyran-4-yl)amino)-1H-pyrazolo[3,4-d]pyrimidin-6-yl)amino)benzamide(Compound 33-2)

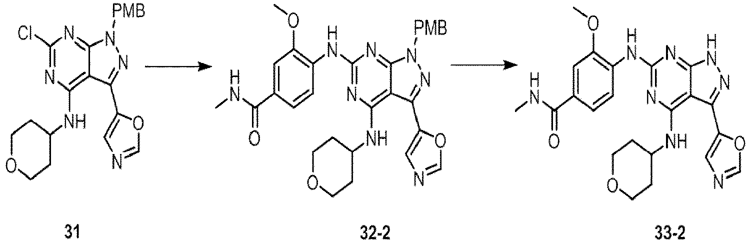

### Step 1. Preparation of Compound 32-2

Compound **31** (50 mg, 0.1134 mmol) and 4-amino-3-methoxy-N-methylbenzamide (24 mg, 0.136 mmol) were subjected to the same preparation method as in Compound **32-1** to obtain Compound **32-2** (42 mg, 63%).

¹H NMR (500 MHz, DMSO-d₆) δ 8.64 (d, J = 2.8 Hz, 1H), 8.57 (d, J = 8.4 Hz, 1H), 8.35 (s, 1H), 7.86 (s, 1H), 7.62 (d, J = 2.8 Hz, 1H), 7.52 (d, J = 11.2 Hz, 2H), 7.26 (d, J = 8.1 Hz, 2H), 6.96 (d, J = 7.3 Hz, 1H), 6.90 (d, J = 7.9 Hz, 2H), 5.42 (s, 2H), 4.34 (s, 1H), 3.95 (d, J = 3.2 Hz, 3H), 3.92 (s, 2H), 3.70 (s, 3H), 3.53 (d, J = 11.6 Hz, 3H), 2.80 (d, J = 4.2 Hz, 3H), 2.02 - 1.97 (m, 2H), 1.70 (d, J = 11.9 Hz, 2H).

### Step 2. Preparation of Compound 33-2

Compound **32-2** (30 mg, 0.0513 mmol) was subjected to the same preparation method as in Compound **8-1** to obtain Compound **33-2** (5.1 mg, 16%).

¹H NMR (300 MHz, DMSO-d₆) δ 13.47 (s, 1H), 8.65 (s, 1H), 8.56 (d, J = 8.8 Hz, 1H), 8.34 (d, J = 4.9 Hz, 1H), 7.78 (s, 1H), 7.63 (s, 1H), 7.51 (d, J = 1.8 Hz, 1H), 7.49 (s, 1H), 6.90 (d, J = 7.4 Hz, 1H), 4.34 (s, 1H), 3.95 (s, 3H), 3.92 (s, 2H), 3.53 (t, J = 11.2 Hz, 2H), 2.80 (d, J = 4.4 Hz, 3H), 2.02 (d, J = 15.0 Hz, 3H), 1.70 (d, J = 9.8 Hz, 2H).

### Example 50. Preparation of N⁶-(2-methoxy-4-(4-methyl-4H-1,2,4-triazol-3-yl)phenyl)-3-(oxazol-5-yl)-N⁴-(tetrahydro-2H-pyran-4-yl)-1H-pyrazolo[3,4-d]pyrimidine-4,6-diamine (Comoound 33-3)

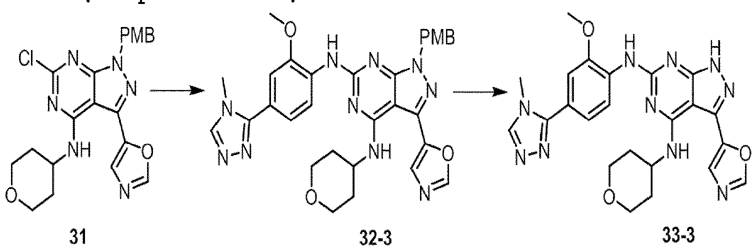

### Step 1. Preparation of Compound 32-3

Compound **31** (50 mg, 0.1134 mmol) and 2-methoxy-4-(4-methyl-4H-1,2,4-triazol-3-yl)aniline (27 mg, 0.136 mmol) were subjected to the same preparation method as in Compound **32-1** to obtain Compound **32-3** (17 mg, 24%).

### Step 2. Preparation of Compound 33-3

Compound **32-3** (17 mg, 0.027 mmol) was subjected to the same preparation method as in Compound **8-1** to obtain Compound **33-3** (2.1 mg, 16%).

¹H NMR (400 MHz, DMSO-d₆) δ 13.47 (s, 1H), 8.65 (d, J = 2.3 Hz, 1H), 8.62 (dd, J = 8.2, 2.2 Hz, 1H), 8.56 (d, J = 2.3 Hz, 1H), 7.82 (d, J = 2.1 Hz, 1H), 7.63 (d, J = 2.3 Hz, 1H), 7.37 (s, 1H), 7.33 (dd, J = 8.3, 2.2 Hz, 1H), 6.90 - 6.85 (m, 1H), 4.36 (s, 1H), 3.97 (d, J = 2.3 Hz, 3H), 3.92 (s, 2H), 3.78 (d, J = 2.3 Hz, 3H), 3.51 (d, J = 11.3 Hz, 3H), 2.03 (d, J = 11.7 Hz, 2H), 1.70 (d, J = 12.2 Hz, 2H).

### Example 51. Preparation of N⁶-(2-methoxy-4-morpholinophenyl)-3-(1-methyl-1H-pyrazol-4-yl)-N⁴-((tetrahydrofuran-2-yl)methyl)-1H-pyrazolo[3,4-d]pyrimidine-4,6-diamine(Compound 8-34)

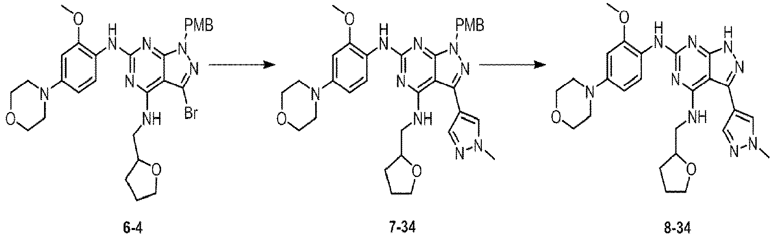

### Step 1. Preparation of Compound 7-34

Compound **6-4** (100 mg, 0.160 mmol) and 1-methyl-4-(4,4,5,5-tetramethyl-1,3,2-dioxaborolan-2-yl)-1H-pyrazole (50 mg, 0.240 mmol) were subjected to the same preparation method as in Compound **7-1** of Example 1 to obtain Compound **7-34** as a brown solid (96 mg, 96%).

¹H NMR (500 MHz, Chloroform-d) δ 1.26 (s, 1H), 1.64 (d, J = 7.7 Hz, 1H), 1.91 (tdd, J = 9.1, 7.1, 3.7 Hz, 2H), 2.05 (s, 2H), 3.13 (s, 4H), 3.47 (s, 1H), 3.76 (s, 4H), 3.91 (s, 9H), 3.97 (s, 3H), 4.11 (s, 2H), 5.40 (s, 2H), 5.72 (s, 1H), 6.55 (s, 2H), 6.84 (s, 2H), 7.26 (s, 1H), 7.36 (s, 3H), 7.75 (d, J = 32.8 Hz, 2H), 8.55 (d, J = 8.5 Hz, 1H).

### Step 2. Preparation of Compound 8-34

Compound **7-34** (96 mg, 0.149 mmol) was subjected to the same preparation method as in Compound **8-1** in Example 1 to obtain Compound **8-34** as an ivory solid (43 mg, 57%).

¹H NMR (300 MHz, DMSO-d₆) δ 1.59 (s, 1H), 1.83 (s, 3H), 3.09 (d, J = 4.7 Hz, 4H), 3.45 (dq, J = 14.1, 8.1, 7.0 Hz, 1H), 3.67 (s, 2H), 3.75 (s, 5H), 3.84 (s, 3H), 3.92 (s, 3H), 4.04 (s, 2H), 5.95 (s, 1H), 6.49 (dd, J = 8.8, 2.5 Hz, 1H), 6.65 (d, J = 2.5 Hz, 1H), 7.40 (s, 1H), 7.70 (d, J = 0.8 Hz, 1H), 8.01 (s, 2H), 12.82 (s, 1H) .

### Example 52. Preparation of 3-(Isoxazol-4-yl)-N⁶-(2-methoxy-4-morpholinophenyl)-N⁴-((tetrahydrofuran-2-yl)methyl)-1H-pyrazolo[3,4-d]pyrimidine-4,6-diamine(Compound 8-35)

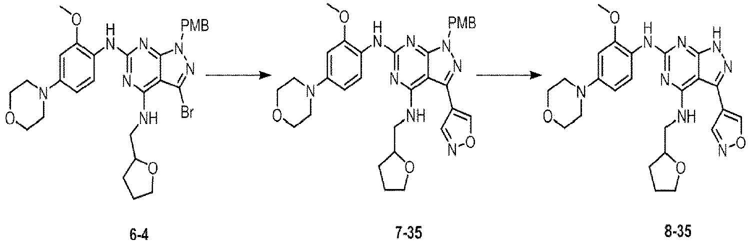

### Step 1. Preparation of Compound 7-35

Compound **6-4** (100 mg, 0.160 mmol) and 4-(4,4,5,5-tetramethyl-1,3,2-dioxaborolan-2-yl)isoxazole (47 mg, 0.240 mmol) were subjected to the same preparation method as in Compound **7-1** to obtain Compound **7-35** (20 mg, 27%).

¹H NMR (400 MHz, Chloroform-d) δ 1.26 (s, 2H), 1.69 (ddt, J = 16.6, 12.4, 6.0 Hz, 1H), 1.94 (d, J = 6.9 Hz, 2H), 3.13 (s, 4H), 3.67 (s, 1H), 3.76 (s, 3H), 3.78 - 3.85 (m, 2H), 3.88 (s, 4H), 3.92 (s, 3H), 3.94 (s, 3H), 4.17 (tq, J = 7.1, 3.5, 3.1 Hz, 1H), 5.33 (s, 2H), 5.57 (s, 1H), 6.55 (s, 2H), 6.85 (s, 2H), 7.33 (s, 2H), 7.42 (s, 1H), 8.51 (s, 1H).

### Step 2. Preparation of Compound 8-35

Compound **7-35** (20 mg, 0.0326 mmol) was subjected to the same preparation method as in Compound **8-1** to obtain Compound **8-35** as a white solid (2 mg, 13%).

¹H NMR (300 MHz, DMSO-d₆) δ 0.91 (s, 2H), 1.29 (d, J = 36.2 Hz, 6H), 1.64 - 1.97 (m, 5H), 3.07 (s, 3H), 3.47 (s, 1H), 3.60 (s, 4H), 3.80 (d, J = 29.2 Hz, 7H), 4.00 - 4.11 (m, 1H), 6.49 (s, 1H), 6.65 (s, 1H), 7.31 (d, J = 9.2 Hz, 2H), 7.96 (s, 1H), 8.09 (s, 1H), 8.53 (s, 1H), 12.56 (s, 1H).

### <Test Example 1: Evaluation of TTK activity inhibitory ability>

To determine the ability of a compound represented by Chemical Formula 1 according to the present invention to inhibit TTK activity, the following experiment was conducted.

A recombinant human TTK protein was mixed with the compound in a reaction solution (8 mM MOPS pH 7.0, 0.2 mM EDTA, 0.33 mg/mL MBP, 10 mM Magnesium acetate, 45 uM [gamma-33P-ATP]) and then reacted at room temperature for 40 minutes. After that, the reaction was terminated by adding phosphoric acid to a concentration of 0.5%, and 10 µl of the reaction solution was dropped onto a P30 filter. The resultant filter was washed four times with a 0.425% phosphoric acid solution, each wash lasting for 4 minutes, and then washed with methanol once more, and dried. The activity of TTK was measured through scintillation counting. The activity value from the group with no compound was set as 100% of control, and the degree of TTK inhibition by the compound was calculated. Table 1 below summarizes the TTK inhibitory activities of example compounds.

**TABLE 1**

| **Compound** | **IC**₅₀ **(nM)** | **Compound** | **IC**₅₀ **(nM)** |
|---|---|---|---|
| Compound 8-6 | ++ | Compound 11-3 | +++ |
| Compound 8-7 | + | Compound 11-4 | +++ |
| Compound 8-8 | ++ | Compound 11-5 | ++ |
| Compound 8-12 | ++ | Compound 14-1 | ++ |
| Compound 8-13 | +++ | Compound 14-2 | ++ |
| Compound 8-14 | ++ | Compound 14-3 | ++ |
| Compound 8-15 | +++ | Compound 18-1 | +++ |
| Compound 8-16 | ++ | Compound 18-2 | ++ |
| Compound 8-18 | ++ | Compound 21-2 | +++ |
| Compound 8-19 | ++ | Compound 21-3 | ++ |
| Compound 8-20 | +++ | Compound 26-1 | ++ |
| Compound 8-21 | ++ | Compound 26-2 | +++ |
| Compound 8-22 | +++ | Compound 28-1 | ++ |
| Compound 8-23 | +++ | Compound 33-1 | +++ |
| Compound 8-24 | +++ | Compound 33-2 | +++ |
| Compound 8-25 | +++ | Compound 33-3 | +++ |
| Compound 8-27 | +++ | Compound 8-34 | +++ |
| Compound 8-28 | +++ | Compound 8-35 | +++ |
| Compound 8-30 | + | Comparative Example 1 (CFI-402257) | ++ |
| Compound 11-2 | +++ | | |
| -: >300 nM IC₅₀ values, +: 100~300 nM IC₅₀ values | | | |
| ++: 30~100 nM IC₅₀ values, +++: <30 nM IC₅₀ values | | | |

Therefore, the compounds represented by Chemical Formula 1 according to the present invention, as confirmed in Test Example 1, had an excellent effect of inhibiting TTK activity, and consequently, the compounds can be helpfully used as TTK activity inhibitors and can be advantageously used as compositions for the prevention or treatment of cancer induced therefrom.

### <Test Example 2: Evaluation of kinase activity inhibitory ability>

The kinase activity inhibitory ability results of Compound 8-8 at a concentration of 500 nM are shown in Table 2 below.

**TABLE 2**

| **Kinase** | **Activity %** | **Inhibition %** |
|---|---|---|
| ALK (h) | 2 | 98 |
| IR(h), activated | 8 | 92 |
| IRR(h) | 7 | 93 |
| LTK(h) | 2 | 98 |
| TTK(h) | -7 | 107 |

Five types of kinases, ALK(h), IR(h) activated, IRR(h), and LTK(h), including TTK(h) targeted by the present invention, were inhibited by more than 90%. In particular, 500 nM of Compound **8-8** completely inhibited TTK activity and had the ability to inhibit other types of kinases as well.

### <Test Example 3: Evaluation of cytotoxicity in TTK-expressed MDA-MB-231 cells>

To determine the cytotoxicity of a compound represented by Chemical Formula 1 according to the present invention to MDA-MB-231 cells, the following test was conducted.

MDA-MB-231 triple negative breast cancer cell line purchased from ATCC was inoculated into each well of a 96-well plate at a volume of 90 µL containing 2,000 cells. Thereafter, an example compound of the present invention was added to respective wells at concentrations of 10 µM, 3 µM, 1 µM, 0.3 µM, 0.1 µM, and 0 µM, and the cells were cultured in a cell incubator at 37°C for 5 days. Five days later, 10 µL of Cyto X solution (manufacturer: LPS Solution) was added to each of the wells, and the absorbance was measured at 450 nm using an Epoch microplate spectrophotometer (manufacturer: BioTek Instruments Inc.) according to the product's user guide. For cytotoxicity, the IC₅₀ value for cytotoxicity of each compound was calculated by quantifying the number of cells using the measured value. The cytotoxicity results of example compounds in the MDA-MB-231 cells are shown in Table 3.

**TABLE 3**

| **Compound No** | **IC**₅₀ **(nM) in MDA-MB-231** | **Compound No** | **IC**₅₀ **(nM) in MDA-MB-231** |
|---|---|---|---|
| Compound 8-2 | + | Compound 8-30 | + |
| Compound 8-4 | ++ | Compound 11-2 | ++++ |
| Compound 8-5 | + | Compound 11-3 | ++++ |
| Compound 8-6 | + | Compound 11-4 | +++ |
| Compound 8-7 | +++ | Compound 11-5 | ++ |
| Compound 8-8 | ++++ | Compound 14-1 | ++++ |
| Compound 8-12 | +++ | Compound 14-2 | +++ |
| Compound 8-13 | ++ | Compound 14-3 | +++ |
| Compound 8-14 | +++ | Compound 18-1 | ++++ |
| Compound 8-15 | +++ | Compound 18-2 | ++ |
| Compound 8-16 | ++ | Compound 21-2 | ++++ |
| Compound 8-17 | ++ | Compound 21-3 | +++ |
| Compound 8-18 | +++ | Compound 26-1 | +++ |
| Compound 8-19 | +++ | Compound 26-2 | + |
| Compound 8-20 | ++++ | Compound 28-1 | ++ |
| Compound 8-21 | ++ | Compound 33-1 | ++++ |
| Compound 8-22 | ++ | Compound 33-2 | +++ |
| Compound 8-23 | ++ | Compound 33-3 | ++ |
| Compound 8-24 | +++ | Compound 8-34 | + |
| Compound 8-25 | +++ | Compound 8-35 | + |
| Compound 8-27 | ++ | Comparative Example 1 (CFI-402257) | ++++ |
| Compound 8-28 | +++ | | |
| +: >1000 nM IC₅₀ values, ++: 500~1000 nM IC₅₀ values | | | |
| +++: 150~500 nM IC₅₀ values, ++++: <150 nM IC₅₀ values | | | |

Referring to Table 3, multiple compounds among the example compounds according to the present invention showed equivalent levels of cytotoxicty IC₅₀ values to Comparative Example 1 (CFI-402257) in MDA-MB-231 cells. Therefore, the compounds represented by Chemical Formula 1 according to the present invention, as confirmed in Test Example 2, had an excellent effect of inhibiting TTK activity in MDA-MB-231 cells, and consequently, the compounds can be helpfully used as TTK activity inhibitors and can be advantageously used as compositions for the prevention or treatment of cancer induced therefrom.

### <Test Example 4: Western blotting investigation of pTTK expression inhibition in MDA-MB-231>

Test Example 3 above found that these compounds induced apoptosis in TTK-expressed MDA-MB-231, and in order to investigate whether TTK protein was involved in the apoptosis, western blotting was performed.

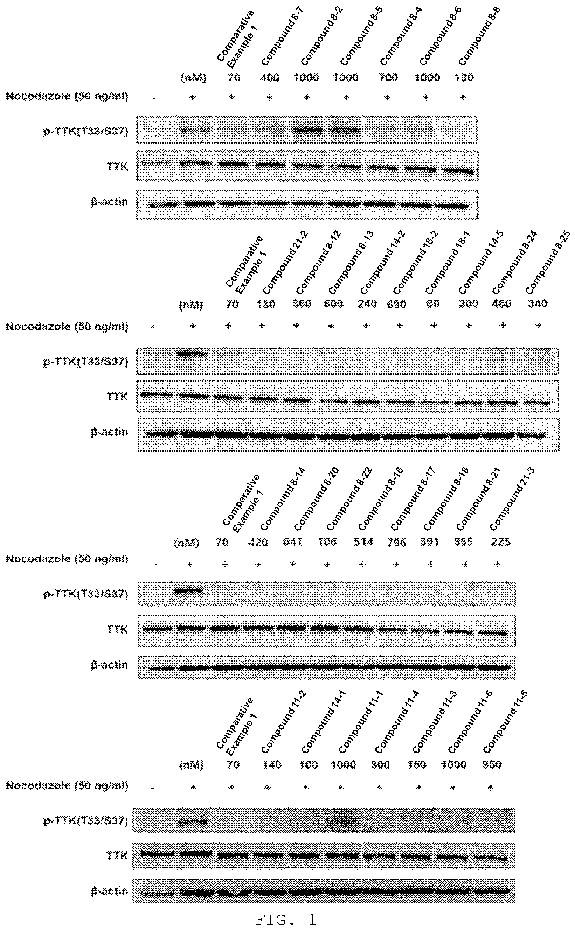

MDA-MB-231 cells were prepared in each well of a 6-well plate at 200,000 per well, treated with 50 ng/mL of nocodazole, and incubated for one day. After that, each of the example compounds of the present invention was added at a concentration corresponding to the IC₅₀ for cytotoxicity and then incubated for 4 hours, and thereafter, the cells were lysed by addition of an RIPA solution. To investigate TTK and pTTK expression in the sample, a protein-specific primary antibody (TTK primary antibody manufacturer - Cell Signaling Technology, and manufacturer for phosphorylated TTK detection of thr33 and ser37 - Invitrogen) and a secondary antibody, which were diluted to 1:10000, were used. As for the luminescence of specific proteins induced using an enhanced chemiluminescence kit (ECL, manufacturer - Santa Cruz Biotechnology), the results thereof were obtained and analyzed using the ChemiDoc XRS+ system. The results are summarized in FIG. 1.

The compounds represented by Chemical Formula 1 according to the present invention completely inhibited pTTK expression equivalent to Comparative Example 1 (CFI-402257) at the IC₅₀ concentration for cytotoxicity in MDA-MB-231 cells.

### <Test Example 5: Evaluation of cytotoxicity in TTK-expressed HCT116 cells>

To determine the cytotoxicity of a compound represented by Chemical Formula 1 according to the present invention to HCT116 cells, the following test was conducted.

HCT116 colon cancer cell line purchased from ATCC was inoculated into each well of a 96-well plate at a volume of 90 µL containing 2,000 cells. Thereafter, an example compound of the present invention was added to respective wells at concentrations of 10 µM, 3 µM, 1 µM, 0.3 µM, 0.1 µM, and 0 µM, and the cells were cultured in a cell incubator at 37°C for 5 days. After 5 days, the number of cells was measured by CCK8 test method, and the cytotoxicity was determined by calculating the IC₅₀ value for cytotoxicity of each compound. The cytotoxicity results of example compounds in the HCT116 cells are shown in Table 4.

**TABLE 4**

| **Compound No** | **IC**₅₀ **(nM) in HCT116** | **Compound No** | **IC**₅₀ **(nM) in HCT116** |
|---|---|---|---|
| Compound 8-2 | + | Compound 8-30 | NA |
| Compound 8-4 | + | Compound 11-2 | ++++ |
| Compound 8-5 | + | Compound 11-3 | +++ |
| Compound 8-6 | + | Compound 11-4 | +++ |
| Compound 8-7 | ++ | Compound 11-5 | +++ |
| Compound 8-8 | ++++ | Compound 14-1 | +++ |
| Compound 8-12 | +++ | Compound 14-2 | +++ |
| Compound 8-13 | ++ | Compound 14-3 | ++++ |
| Compound 8-14 | +++ | Compound 18-1 | ++++ |
| Compound 8-15 | +++ | Compound 18-2 | ++ |
| Compound 8-16 | ++++ | Compound 21-2 | ++++ |
| Compound 8-17 | ++ | Compound 21-3 | ++++ |
| Compound 8-18 | ++++ | Compound 26-1 | ++ |
| Compound 8-19 | ++ | Compound 26-2 | + |
| Compound 8-20 | ++++ | Compound 28-1 | + |
| Compound 8-21 | ++ | Compound 33-1 | ++++ |
| Compound 8-22 | ++++ | Compound 33-2 | ++ |
| Compound 8-23 | +++ | Compound 33-3 | + |
| Compound 8-24 | +++ | Compound 8-34 | + |
| Compound 8-25 | +++ | Compound 8-35 | + |
| Compound 8-27 | ++ | Comparative Example 1 (CFI-402257) | ++++ |
| Compound 8-28 | +++ | | |
| +: >1000 nM IC₅₀ values, ++: 500~1000 nM IC₅₀ values | | | |
| +++: 150~500 nM IC₅₀ values, ++++: <150 nM IC₅₀ values | | | |

Referring to Table 4, multiple compounds among the example compounds according to the present invention showed equivalent or lower levels of cytotoxicity IC₅₀ values to Comparative Example 1 (CFI-402257) in HCT116 cells. Therefore, the compounds represented by Chemical Formula 1 according to the present invention had an excellent effect of inhibiting TTK activity in HCT116 cells, and consequently, the compounds can be helpfully used as TTK activity inhibitors and can be advantageously used as compositions for the prevention or treatment of cancer induced therefrom.

### <Test Example 6: Western blotting investigation of pTTK expression inhibition in HCT116>

Test Example 5 above found that these compounds induced apoptosis in TTK-expressed HCT116, and in order to investigate whether TTK protein was involved in the apoptosis, western blotting was performed.

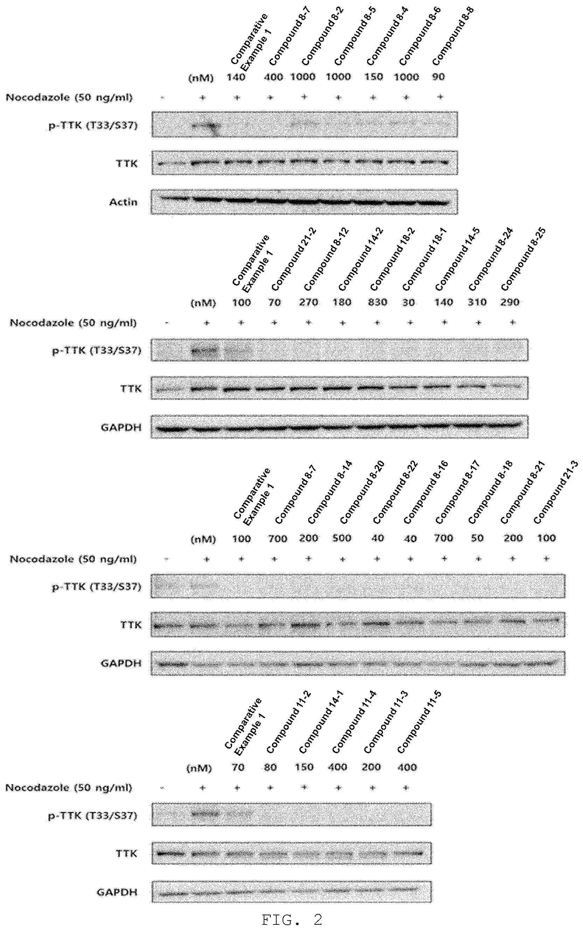

HCT116 cells were prepared in each well of a 6-well plate at 200,000 per well, treated with 50 ng/mL of nocodazole, and incubated for one day. After that, each of the example compounds of the present invention was added at a concentration corresponding to the IC₅₀ for cytotoxicity and then incubated for 4 hours, and thereafter, the cells were lysed by addition of an RIPA solution. To investigate TTK and pTTK expression in the sample, a protein-specific primary antibody (TTK primary antibody manufacturer - Cell Signaling Technology, and manufacturer for phosphorylated TTK detection of thr33 and ser37 - Invitrogen) and a secondary antibody, which were diluted to 1:10000, were used. As for the luminescence of specific proteins induced using an enhanced chemiluminescence kit (ECL, manufacturer - Santa Cruz Biotechnology), the results thereof were obtained and analyzed using the ChemiDoc XRS+ system. The results are summarized in FIG. 2.

All of the compounds represented by Chemical Formula 1 according to the present invention completely inhibited pTTK expression equivalent to Comparative Example 1 (CFI-402257) at the IC₅₀ concentration for cytotoxicity in HCT116 cells.

### <Test Example 7: Evaluation of drug efficacy on Nude mice employing MDA-MB-231>

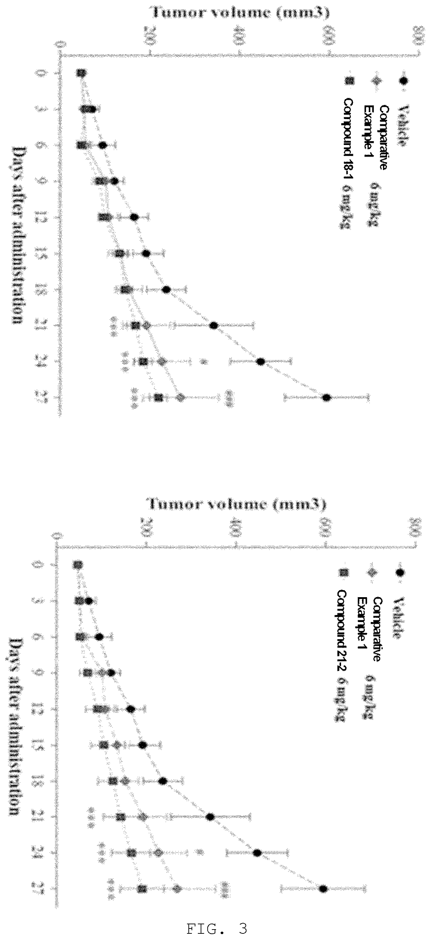

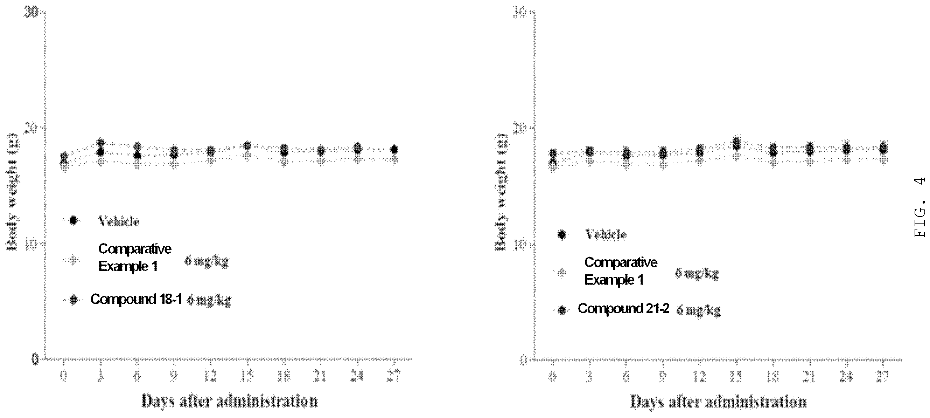

5-Week-old female nude mice were subcutaneously injected with 5×10⁶ MDA-MB-231 cells into the flank. When the tumor volume reached approximately 50 mm³, the mice were divided into groups, each containing 5 animals, which were than orally administered a drug. The dosage of the drug was 6 mg/kg and administered daily for 4 weeks. The body weight change and tumor volume were measured, and the results are shown in FIGS. 3 and 4.

FIG. 3 shows graphs depicting the tumor volume over time, observed for a drug administration control group (Control), Comparative Example 1 (CFI-402257) treatment group, and example treatment groups, in nude mice injected with MDA-MB-231 cell line. Referring to FIG. 3, the tumor volume over time was significantly increased in the control group, but reduced in the example compound treatment groups and comparative example 1 (CFI-402257) treatment group. Particularly, the example compound treatment groups showed a higher suppression of tumor volume compared with the comparative example 1 (CFI-402257) treatment group.

FIG. 4 shows graphs depicting the body weight change over time, observed for a drug administration control group (Control), Comparative Example 1 (CFI-402257) treatment group, and example treatment groups, in nude mice injected with MDA-MB-231 cell line. Referring to FIG. 4, the animal body weight over time was not reduced in all the test groups.

Therefore, the example compounds according to the present invention, as confirmed in the above test example, had an excellent effect of inhibiting TTK activity in nude mice employing MDA-MB-231 cells, and consequently, the compounds can be helpfully used as TTK activity inhibitors and can be advantageously used as compositions for the prevention or treatment of cancer induced therefrom.

### <Test Example 8: Evaluation of cytotoxicity of co-treatment with pacalitaxe in TTK-expressed MDA-MB-231 cells>

To determine the cytotoxicity of an example compound according to the present invention on MDA-MB-231 cells when co-administered with paclitaxel, the following test was conducted.

MDA-MB-231 triple negative breast cancer cell line purchased from ATCC was inoculated into each well of a 96-well plate at a volume of 90 µL containing 2,000 cells. Thereafter, paclitaxel was added at concentrations of 0 µM, 0.001 µM, and 0.003 µM, and then an example compound of the present invention was added to respective wells at concentrations of 1 µM, 0.3 µM, 0.1 µM, 0.03 µM, 0.01 µM, and 0 µM. The cells were cultured in a cell incubator at 37°C for 5 days. Five days later, 10 µL of Cyto X solution (manufacturer: LPS Solution) was added to each of the wells, and the absorbance was measured at 450 nm using an Epoch microplate spectrophotometer (manufacturer: BioTek Instruments Inc.) according to the product's user guide. For cytotoxicity, the IC₅₀ value for cytotoxicity of each compound was calculated by quantifying the number of cells using the measured value. The results are shown in Table 5.

As a result, the compound treatment groups showed a reduction in IC₅₀ in the addition of 0.001 µM paclitaxel compared with the non-addition of paclitaxel, which corresponds to equivalent or higher levels of efficacy obtained by co-treatment with paclitaxel and Comparative Example 1 (CFI-402257), and these results confirmed the possibility of co-administration of the example drugs with paclitaxel as in Comparative Example 1.

### <Test Example 9: Evaluation of cell cycle distribution of co-treatment with paclitaxel in TTK-expressed MDA-MB-231 cells>

To determine the change in cell cycle distribution when a compound represented by Chemical Formula 1 according to the present invention and paclitaxel were co-applied to MDA-MB-231 cells, the following test was conducted.

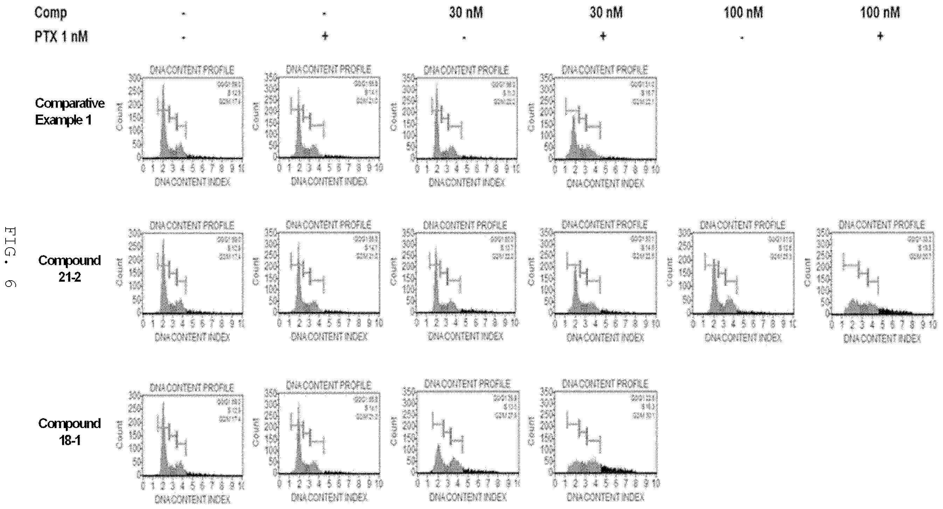

MDA-MB-231 triple negative breast cancer cell line purchased from ATCC was inoculated into each well of a 6-well plate at 100,000 cells. Thereafter, paclitaxel was added at concentrations of 0 µM and 0.001 µM and an example compound of the present invention was added at concentrations of 0 µM, 0.03 µM, or 0.1 µM, and the cells were cultured in a cell incubator at 37°C for 2 days. After 2 days, the cells were fixed with 70% ethanol, and added and into 200 µL of Muse Cell Cycle reagent in a light-shielded state and cultured for 30 minutes. Then, the cell cycle distribution was analyzed using a Muse Cell Analyzer (manufacturer: Merck, Millipore), and the results are shown in FIG. 6.

The results verified that in the normal cell cycle distribution observed in the control group, the addition of the example compound suppressed the distribution of cells in the G1 and G2/M and lead to a distribution of multiple cells as polyploids due to TTK inhibition, and these results were equivalent to or higher than those from Comparative Example 1 (CFI-402257). Additionally, both Comparative Example 1 and Example compounds showed enhanced changes in cell distribution when co-treated with 0.001 µM paclitaxel, and thus the example drugs of the present invention also showed synergistic anticancer effects when co-administered with paclitaxel.

### <Preparation Example 1. Preparation of powder formulation>

Powder formulation was prepared by mixing 2 g of Compound 8-1 and 1 g of lactose and then filling the mixture in a sealed package.

### <Preparation Example 2. Preparation of tablet formulation>

Table formulation was prepared by mixing 100 mg of Compound 8-1, 100 mg of microcrystalline cellulose, 60 mg of a lactose hydrate, 20 mg of low-substituted hydroxypropyl cellulose, and 2 mg of magnesium stearate and then compressing the mixture into a tablet through a typical tablet preparation method.

### <Preparation Example 3. Preparation of capsule formulation>

Capsule formulation was prepared by mixing 100 mg of Compound 8-1, 100 mg of microcrystalline cellulose, 60 mg of a lactose hydrate, 20 mg of low-substituted hydroxypropyl cellulose, 2 mg of magnesium stearate and then filling the mixture in a gelatin capsule through a typical capsule preparation method.

### <Preparation Example 4. Preparation of pill formulation>

Pill formulation was prepared by mixing 90 mg of Compound 8-1, 5 mg of glutinous rice starch, 5 mg of purified water, and a small amount of a hygroscopic property inhibiting additive, such as dextrin, maltodextrin, corn starch, or microcrystalline cellulose (MCC), and then making the mixture into 100 mg of pills through a typical pill preparation method.

### <Preparation Example 5. Preparation of injection formulation>

Injection formulation was prepared by mixing 10 mg of Compound 8-1, a suitable amount of sterile distilled water for an injection, and a suitable amount of a pH adjuster and then allowing each ampoule (2 mL) to contain the above ingredient contents through a typical injection preparation method.

## Claims

1. A compound of Chemical Formula 1 below, or a hydrate, solvate, prodrug, or pharmaceutically acceptable salt thereof: 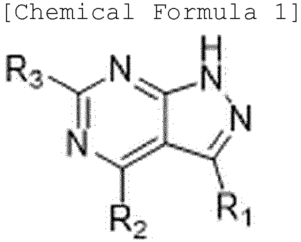 where:
R₁ is substituted or unsubstituted C₆-C₁₂ aryl, substituted or unsubstituted heteroaryl of 5 to 12 atoms, or substituted or unsubstituted heterocycloalkyl of 5 to 12 atoms;
the substituted aryl, heteroaryl, or heterocycloalkyl has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, C(=O), carbamoyl, C₁-C₆ alkylaminoketone, C₁-C₆ alkoxycarbonyl, C₁-C₆ alkylsulfonamino, C₃-C₆ cycloalkylcarboxamido or C₃-C₆ cycloalkylaminoketone;
R₂ is substituted or unsubstituted -NH-(C₆-C₁₂)aryl, substituted or unsubstituted -NH-(C₃-C₆)cycloalkyl, substituted or unsubstituted -NH-(C₃-C₆)heterocycloalkyl, or -NH-(CH₂)ₘ-(C₃-C₆) heterocycloalkyl (where m is an integer of 0, 1, or 2);
the substituted aryl, cycloalkyl, or heterocycloalkyl has one or more hydrogen atoms in a ring thereof, each of which is independently substituted with halogen, C₁-C₆ alkyl, halo C₁-C₆ alkyl, C₁-C₆ alkoxy, or halo C₁-C₆ alkoxy;
R₃ is substituted or unsubstituted -NH-(C₆-C₁₂) aryl or substituted or unsubstituted -NH-(C₃-C₆)cycloalkyl;
the substituted aryl or cycloalkyl has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, substituted or unsubstituted morpholine, substituted or unsubstituted piperazine, substituted or unsubstituted triazole, C₁-C₆ alkylaminoketone, substituted or unsubstituted -C(=O)-morpholine, substituted or unsubstituted -C(=O)-piperazine, or substituted or unsubstituted -C(=O)NH-piperidine; and
the substituted morpholine, piperazine, triazole, - C(=O)-morpholine, -C(=O)-piperazine, or -C(=O)NH-piperidine has one or more hydrogen atoms, each of which is independently substituted with halogen, C₁-C₆ alkyl, halo C₁-C₆ alkyl, C₁-C₆ alkoxy.

2. The compound of claim 1 having Chemical Formula 2-1 below, or a hydrate, solvate, prodrug, or pharmaceutically acceptable salt thereof: 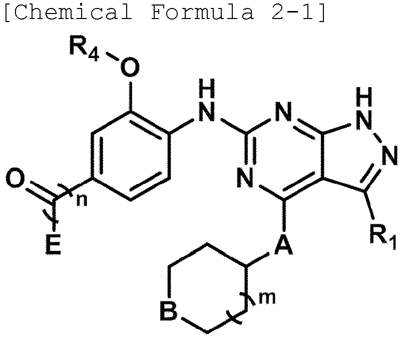 where:
R₁ is substituted or unsubstituted pyridine, substituted or unsubstituted pyrazole, substituted or unsubstituted imidazole, substituted or unsubstituted furan, substituted or unsubstituted thiazole, substituted or unsubstituted pyrimidine, substituted or unsubstituted triazole, substituted or unsubstituted oxadiazole, substituted or unsubstituted oxazole, substituted or unsubstituted isoxazole, substituted or unsubstituted benzimidazole, substituted or unsubstituted indazole, substituted or unsubstituted benzoxazole, substituted or unsubstituted 1,3,4-oxadiazolone, substituted or unsubstituted benzimidazole, substituted or unsubstituted indazole, or substituted or unsubstituted benzoxazole;
the substituted pyridine, pyrazole, imidazole, furan, thiazole, pyrimidine, triazole, oxadiazole, oxazole, isoxazole, benzimidazole, indazole, benzoxazole, 1,3,4-oxadiazolone, benzimidazole, indazole, or benzoxazole has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, C(=O), carbamoyl, C₁-C₆ alkylaminoketone, C₁-C₆ alkoxycarbonyl, C₁-C₆ alkylsulfonamino, C₃-C₆ cycloalkylcarboxamido or C₃-C₆ cycloalkylaminoketone;
R₄ is substituted with hydrogen or C₁-C₆ alkyl;
E is substituted with -NHR₄, 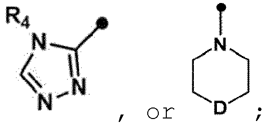
A is substituted with NH;
B is substituted with O, S, or CH₂;
D is substituted with O or NR₄;
m is an integer of 0, 1, or 2; and
n is an integer of 0 or 1.

3. The compound of claim 1 having Chemical Formula 2-2 below, or a hydrate, solvate, prodrug, or pharmaceutically acceptable salt thereof: 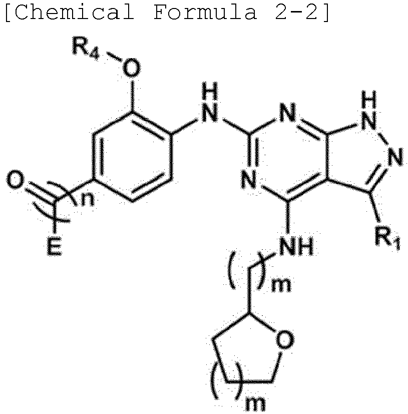 where:
R₁ is substituted or unsubstituted pyridine, substituted or unsubstituted pyrazole, substituted or unsubstituted imidazole, substituted or unsubstituted furan, substituted or unsubstituted thiazole, substituted or unsubstituted pyrimidine, substituted or unsubstituted triazole, substituted or unsubstituted oxadiazole, substituted or unsubstituted oxazole, substituted or unsubstituted isoxazole, substituted or unsubstituted benzimidazole, substituted or unsubstituted indazole, substituted or unsubstituted benzoxazole, substituted or unsubstituted 1,3,4-oxadiazolone, substituted or unsubstituted benzimidazole, substituted or unsubstituted indazole, or substituted or unsubstituted benzoxazole;
the substituted pyridine, pyrazole, imidazole, furan, thiazole, pyrimidine, triazole, oxadiazole, oxazole, isoxazole, benzimidazole, indazole, benzoxazole, 1,3,4-oxadiazolone, benzimidazole, indazole, or benzoxazole has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, C(=O), carbamoyl, C₁-C₆ alkylaminoketone, C₁-C₆ alkoxycarbonyl, C₁-C₆ alkylsulfonamino, C₃-C₆ cycloalkylcarboxamido or C₃-C₆ cycloalkylaminoketone;
R₄ is substituted with hydrogen or C₁-C₆ alkyl; 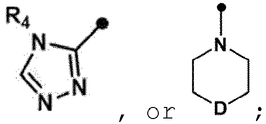
D is substituted with O or NR₄;
m is an integer of 0, 1, or 2; and
n is an integer of 0 or 1.

4. A compound of Chemical Formula 3-1 below, or a hydrate, solvate, prodrug, or pharmaceutically acceptable salt thereof: 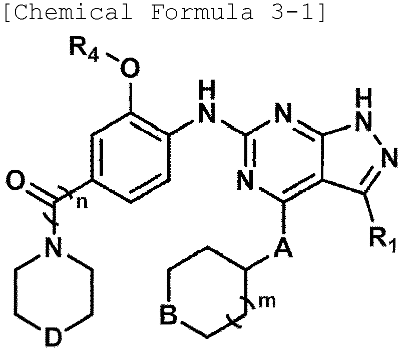 where:
R₁ is substituted or unsubstituted pyridine, substituted or unsubstituted pyrazole, substituted or unsubstituted imidazole, substituted or unsubstituted furan, substituted or unsubstituted thiazole, substituted or unsubstituted pyrimidine, substituted or unsubstituted triazole, substituted or unsubstituted oxadiazole, substituted or unsubstituted oxazole, substituted or unsubstituted isoxazole, substituted or unsubstituted benzimidazole, substituted or unsubstituted indazole, substituted or unsubstituted benzoxazole, substituted or unsubstituted 1,3,4-oxadiazolone, substituted or unsubstituted benzimidazole, substituted or unsubstituted indazole, or substituted or unsubstituted benzoxazole;
the substituted pyridine, pyrazole, imidazole, furan, thiazole, pyrimidine, triazole, oxadiazole, oxazole, isoxazole, benzimidazole, indazole, benzoxazole, 1,3,4-oxadiazolone, benzimidazole, indazole, or benzoxazole has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, C(=O), carbamoyl, C₁-C₆ alkylaminoketone, C₁-C₆ alkoxycarbonyl, C₁-C₆ alkylsulfonamino, C₃-C₆ cycloalkylcarboxamido or C₃-C₆ cycloalkylamino;
R₄ is substituted with hydrogen or C₁-C₆ alkyl;
A is substituted with NH;
B is substituted with O, S, or CH₂;
D is substituted with O or NR₄;
m is an integer of 0, 1, or 2; and
n is an integer of 0 or 1.

5. A compound of Chemical Formula 3-2 below, or a hydrate, solvate, prodrug, or pharmaceutically acceptable salt thereof: 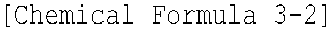 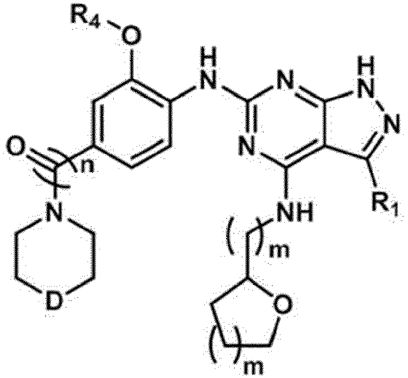 where:
R₁ is substituted or unsubstituted pyridine, substituted or unsubstituted pyrazole, substituted or unsubstituted imidazole, substituted or unsubstituted furan, substituted or unsubstituted thiazole, substituted or unsubstituted pyrimidine, substituted or unsubstituted triazole, substituted or unsubstituted oxadiazole, substituted or unsubstituted oxazole, substituted or unsubstituted isoxazole, substituted or unsubstituted benzimidazole, substituted or unsubstituted indazole, substituted or unsubstituted benzoxazole, substituted or unsubstituted 1,3,4-oxadiazolone, substituted or unsubstituted benzimidazole, substituted or unsubstituted indazole, or substituted or unsubstituted benzoxazole;
the substituted pyridine, pyrazole, imidazole, furan, thiazole, pyrimidine, triazole, oxadiazole, oxazole, isoxazole, benzimidazole, indazole, benzoxazole, 1,3,4-oxadiazolone, benzimidazole, indazole, or benzoxazole has one or more hydrogen atoms in a ring thereof, each of which is independently substituted with halogen, C₁-C₆ alkyl, halo C₁-C₆ alkyl, C₁-C₆ alkoxy, halo C₁-C₆ alkoxy, C₁-C₆ alkoxy C₁-C₆ alkoxy, C(=O), carbamoyl, C₁-C₆ alkylaminoketone, C₁-C₆ alkoxycarbonyl, C₁-C₆ alkylsulfonamino, C₃-C₆ cycloalkylcarboxamido or C₃-C₆ cycloalkylaminoketone;
R₄ is substituted with hydrogen or C₁-C₆ alkyl;
D is substituted with O or NR₄;
m is an integer of 0, 1, or 2; and
n is an integer of 0 or 1.

6. The compound of claim 1, or a hydrate, solvate, prodrug, or pharmaceutically acceptable salt thereof, wherein the compound represented by Chemical Formula 1 is selected from the group consisting of:
N⁴-cyclohexyl-3-(5-fluoropyridine-3-yl)-N⁶-(2-methoxy-4-morpholinophenyl)-1H-pyrazolo[3,4-d] pyrimidine-4,6-diamine (Compound 8-1);
3-(4-(cyclohexylamino)-6-((2-methoxy-4-morpholinophenyl)amino)-1H-pyrazolo[3,4-d]pyrimidin-3-yl)benzamide (Compound 8-2);
methyl 4-(4-(cyclohexylamino)-6-((2-methoxy-4-morphonophenyl)amino)-1H-pyrazolo [3,4-d]pyrimidin-3-yl)benzoate (Compound 8-3);
N-(3-(4-(cyclohexylamino)-6-(2-methoxy-4-morpholinophenyl)amino)-1H-pyrazolo [3,4-d]pyrimidin-3-yl)phenyl)cyclopropanecarboxamide (Compound 8-4);
3-(4-(cyclohexylamino)-6-((2-methoxy-4-morphonophenyl)amino)-1H-pyrazolo [3,4-d]pyrimidin-3-yl)-N-methylbenzamide (Compound 8-5);
N-(3-(4-(cyclohexylamino)-6-((2-methoxy-4-morpholinophenyl)amino)-1H-pyrazolo [3,4-d]pyrimidin-3-yl)phenyl)methanesulfonamide (Compound 8-6);
N⁴-cyclohexyl-N⁶-(2-methoxy-4-morpholinophenyl)-3-(1-methyl-1H-pyrazol-4-yl)-1H-pyrazolo[3,4-d]pyrimidine-4,6-diamine (Compound 8-7);
N⁴-cyclohexyl-N⁶-(2-methoxy-4-morpholinophenyl)-1-(4-methoxybenzyl)-3-(1H-pyrazol-4-yl)-1H-pyrazolo[3,4-d]pyrimidine-4,6-diamine (Compound 8-8);
N⁴-cyclohexyl-N⁶-(4-morpholinocyclohexyl)-3-(pyridin-3-yl)-1H-pyrazolo[3,4-d]pyrimidine-4,6-diamine (Compound 8-9);
N⁴-cyclohexyl-N⁶-(2-methoxy-4-morpholinophenyl)-3-(1-methyl-1H-pyrazol-5-yl)-1H-pyrazolo[3,4-d]pyrimidine-4,6-diamine (Compound 8-10);
N⁴-cyclohexyl-3-(1-isopropyl-1H-imidazol-4-yl)-N⁶-(2-methoxy-4-morpholinophenyl)-1H-pyrazolo[3,4-d]pyrimidine-4,6-diamine (Compound 8-11);
N⁴-cyclohexyl-3-(1-(difluoromethyl)-1H-pyrazol-4-yl)-N⁶-(2-methoxy-4-morpholinophenyl)-1H-pyrazolo[3,4-d]pyrimidine-4,6-diamine (Compound 8-12);
N⁴-cyclohexyl-N⁶-(2-methoxy-4-morpholinophenyl)-3-(1-methyl-1H-pyrazol-3-yl)-1H-pyrazolo[3,4-d]pyrimidine-4,6-diamine (Compound 8-13);
N⁴-cyclohexyl-3-(furan-2-yl)-N⁶-(2-methoxy-4-morpholinophenyl)-1H-pyrazolo[3,4-d]pyrimidine-4,6-diamine (Compound 8-14);
N⁴-cyclohexyl-N⁶-(2-methoxy-4-morpholinophenyl)-3-(thiazol-5-yl)-1H-pyrazolo[3,4-d]pyrimidine-4,6-diamine (Compound 8-15);
3-(1H-benzo[d]imidazol-5-yl)-N⁴-cyclohexyl-N⁶-(2-methoxy-4-morpholinophenyl)-1H-pyrazolo[3,4-d]pyrimidine-4,6-diamine (Compound 8-16);
N⁴-cyclohexyl-3-(1H-indazol-5-yl)-N⁶-(2-methoxy-4-morpholinophenyl)-1H-pyrazolo[3,4-d]pyrimidine-4,6-diamine (Compound 8-17);
N⁴-cyclohexyl-N⁶-(2-methoxy-4-morpholinophenyl)-3-(2-methylbenzo[d]oxazol-6-yl)-1H-pyrazolo[3,4-d]pyrimidine-4,6-diamine (Compound 8-18);
N⁴-cyclohexyl-N⁶-(2-methoxy-4-morpholinophenyl)-3-(1-methyl-1H-imidazol-4-yl)-1H-pyrazolo[3,4-d]pyrimidine-4,6-diamine (Compound 8-19);
N⁴-cyclohexyl-3-(isoxazol-4-yl)-N⁶-(2-methoxy-4-morpholinophenyl)-1H-pyrazolo[3,4-d]pyrimidine-4,6-diamine(Compound 8-20);
3-(Isoxazol-4-yl)-N⁶-(2-methoxy-4-morpholinophenyl)-N⁴-(tetrahydro-2H-pyran-4-yl)-1H-pyrazolo[3,4-d]pyrimidine-4,6-diamine(Compound 8-21);
N⁶-(2-methoxy-4-morpholinophenyl)-3-(oxazol-2-yl)-N⁴-(tetrahydro-2H-pyran-4-yl)-1H-pyrazolo[3,4-d]pyrimidine-4,6-diamine(Compound 8-22);
N⁶-(2-methoxy-4-morpholinophenyl)-3-(1-methyl-1H-pyrazol-4-yl)-N⁴-(tetrahydro-2H-pyran-4-yl)-1H-pyrazolo[3,4-d]pyrimidine-4,6-diamine (Compound 8-23);
3-(1-(difluoromethyl)-1H-pyrazol-4-yl)-N⁶-(2-methoxy-4-morpholinophenyl)-N⁴-(tetrahydro-2H-pyran-4-yl)-1H-pyrazolo[3,4-d]pyrimidine-4,6-diamine (Compound 8-24);
N⁶-(2-methoxy-4-morpholinophenyl)-3-(1-methyl-1H-pyrazol-3-yl)-N⁴-(tetrahydro-2H-pyran-4-yl)-1H-pyrazolo[3,4-d]pyrimidine-4,6-diamine (Compound 8-25);
N⁶-(2-methoxy-4-morpholinophenyl)-3-(1H-pyrazol-4-yl)-N⁴-(tetrahydro-2H-pyran-4-yl)-1H-pyrazolo[3,4-d]pyrimidine-4,6-diamine (Compound 8-26);
N⁶-(2-methoxy-4-morpholinophenyl)-3-(pyridin-3-yl)-N⁴-(tetrahydro-2H-pyran-4-yl)-1H-pyrazolo[3,4-d]pyrimidine-4,6-diamine (Compound 8-27);
N⁶-(2-methoxy-4-morpholinophenyl)-3-(pyridin-4-yl)-N⁴-(tetrahydro-2H-pyran-4-yl)-1H-pyrazolo[3,4-d]pyrimidine-4,6-diamine (Compound 8-28);
N⁶-(2-methoxy-4-morpholinophenyl)-3-(pyrimidin-5-yl)-N⁴-(tetrahydro-2H-pyran-4-yl)-1H-pyrazolo[3,4-d]pyrimidine-4,6-diamine (Compound 8-29);
N⁴-cyclohexyl-3-(1-methyl-1H-pyrazol-4-yl)-N⁶-(4-morpholinocyclohexyl)-1H-pyrazolo[3,4-d]pyrimidine-4,6-diamine (Compound 8-30);
N⁴-cyclohexyl-N⁶-(2-methoxy-4-(4-methylpiperazin-1-yl)phenyl)-3-(1-methyl-1H-pyrazol-4-yl)-1H-pyrazolo[3,4-d]pyrimidine-4,6-diamine (Compound 11-1);
N⁴-cyclohexyl-N⁶-(2-methoxy-4-(4-methyl-4H-1,2,4-triazol-3-yl)phenyl)-3-(1-methyl-1H-pyrazol-4-yl)-1H-pyrazolo[3,4-d]pyrimidine-4,6-diamine (Compound 11-2);
4-((4-(cyclohexylamino)-3-(1-methyl-1H-pyrazol-4-yl)-1H-pyrazolo[3,4-d]pyrimidin-6-yl)amino)-3-methoxy-N-methylbenzamide (Compound 11-3);
(4-((4-(cyclohexylamino)-3-(1-methyl-1H-pyrazol-4-yl)-1H-pyrazolo[3,4-d]pyrimidin-6-yl)amino)-3-methoxyphenyl)(morpholino)methanone (Compound 11-4);
(4-((4-(cyclohexylamino)-3-(1-methyl-1H-pyrazol-4-yl)-1H-pyrazolo[3,4-d]pyrimidin-6-yl)amino)-3-methoxyphenyl)(4-methylpiperazin-1-yl)methanone (Compound 11-5);
4-((4-(cyclohexylamino)-3-(1-methyl-1H-pyrazol-4-yl)-1H-pyrazolo[3,4-d]pyrimidin-6-yl)amino)-3-methoxy-N-(1-methylpiperidin-4-yl)benzamide (Compound 11-6);
N⁴-cyclohexyl-N⁶-(2-methoxy-4-morpholinophenyl)-3-(4-methyl-4H-1,2,4-triazol-3-yl)-1H-pyrazolo[3,4-d]pyrimidine-4,6-diamine (Compound 14-1);
N⁴-cyclohexyl-N⁶-(2-methoxy-4-morpholinophenyl)-3-(1,3,4-oxadiazol-2-yl)-1H-pyrazolo[3,4-d]pyrimidine-4,6-diamine (Compound 14-2);
5-(4-(cyclohexylamino)-6-((2-methoxy-4-morpholinophenyl)amino)-1H-pyrazolo[3,4-d]pyrimidin-3-yl)-1,3,4-oxadiazol-2(3H)-one (Compound 14-3);
N⁴-cyclohexyl-N⁶-(2-methoxy-4-morpholinophenyl)-3-(oxazol-5-yl)-1H-pyrazolo[3,4-d]pyrimidine-4,6-diamine (Compound 18-1);
N⁴-cyclohexyl-3-(1H-imidazol-5-yl)-N⁶-(2-methoxy-4-morpholinophenyl)-lH-pyrazolo[3,4-d]pyrimidine-4,6-diamine (Compound 18-2);
N⁴-cyclohexyl-N⁶-(2-methoxy-4-morpholinophenyl)-3-(1H-1,2,3-triazol-4-yl)-1H-pyrazolo[3,4-d]pyrimidine-4,6-diamine (Compound 21-1);
N⁴-cyclohexyl-N⁶-(2-methoxy-4-morpholinophenyl)-3-(1-methyl-1H-1,2,3-triazol-4-yl)-1H-pyrazolo[3,4-d]pyrimidine-4,6-diamine (Compound 21-2);
N⁶-(2-methoxy-4-morpholinophenyl)-3-(1-methyl-1H-1,2,3-triazol-4-yl)-N⁴-(tetrahydro-2H-pyran-4-yl)-1H-pyrazolo[3,4-d]pyrimidine-4,6-diamine (Compound 21-3);
N⁴-cyclohexyl-N⁶-(2-isopropoxy-4-morpholinophenyl)-3-(1-methyl-1H-pyrazol-4-yl)-1H-pyrazolo[3,4-d]pyrimidine-4,6-diamine (Compound 26-1);
4-(cyclohexyloxy)-N-(2-methoxy-4-morpholynophenyl)-3-(1-methyl-1H-pyrazole-4-yl)-1H-pyrazolo[3,4-d]pyrimidine-6-amine (Compound 26-2);
N⁶-(2-methoxy-4-morpholinophenyl)-3-(1-methyl-1H-pyrazol-4-yl)-N⁴-(1-methylpiperidin-4-yl)-1H-pyrazolo[3,4-d]pyrimidine-4,6-diamine(Compound 28-1);
N⁶-(2-methoxy-4-morpholinophenyl)-3-(oxazol-5-yl)-N⁴-(tetra-2H-pyran-4-yl)-1H-pyrazolo[3,4-d]pyrimidine-4,6-diamine(Compound 33-1);
3-methoxy-N-methyl-4-((3-(oxazol-5-yl)-4-((tetrahydro-2H-pyran-4-yl)amino)-1H-pyrazolo[3,4-d]pyrimidin-6-yl)amino)benzamide(Compound 33-2);
N⁶-(2-methoxy-4-(4-methyl-4H-1,2,4-triazol-3-yl)phenyl)-3-(oxazol-5-yl)-N⁴-(tetrahydro-2H-pyran-4-yl)-1H-pyrazolo[3,4-d]pyrimidine-4,6-diamine(Compound 33-3);
N⁶-(2-methoxy-4-morpholinophenyl)-3-(1-methyl-1H-pyrazol-4-yl)-N⁴-((tetrahydrofuran-2-yl)methyl)-1H-pyrazolo[3,4-d]pyrimidine-4,6-diamine(Compound 8-34); and
3-(isoxazol-4-yl)-N⁶-(2-methoxy-4-morpholinophenyl)-N⁴-((tetrahydrofuran-2-yl)methyl)-1H-pyrazolo[3,4-d]pyrimidine-4,6-diamine(Compound 8-35).

7. A pharmaceutical composition for prevention or treatment of cancer, the pharmaceutical composition comprising a therapeutically effective amount of the compound of any one of claims 1 to 6, or a hydrate, solvate, prodrug, or pharmaceutically acceptable salt thereof, and a pharmaceutically acceptable carrier.

8. The pharmaceutical composition of claim 7, wherein the pharmaceutical composition exhibits inhibitory activity on at least one protein kinase selected from the group consisting of TTK(h), ALK(h), IR(h) activated, IRR(h), and LTK(h).

9. The pharmaceutical composition of claim 8, wherein the pharmaceutical composition exhibits inhibitory activity on TTK kinase.

10. The pharmaceutical composition of claim 7, wherein the cancer is a solid cancer or blood cancer.

11. The pharmaceutical composition of claim 10, wherein the solid cancer is any one of brain tumor, benign astrocytoma, malignant astrocytoma, pituitary adenoma, meningioma, primary central nervous system lymphoma, oligodendroglioma, craniopharyngioma, ependymoma, brain stem glioma, head and neck tumor, laryngeal cancer, oropharyngeal cancer, nasal cavity cancer, nasopharyngeal tumor, salivary gland tumor, hypopharyngeal cancer, thyroid cancer, oral cavity tumor, oral squamous cell carcinoma, Barrett's esophagus, thoracic tumor, small cell lung cancer, non-small cell lung cancer, thymic carcinoma, mediastinal neoplasm, esophageal cancer, neuroglioma, breast cancer, male breast cancer, triple negative breast cancer (TNBC), breast sporadic basaloid carcinoma (BLC), abdominal tumor, stomach cancer, liver cancer, gallbladder cancer, biliary tract cancer, pancreatic cancer, pancreatic ductal adenocarcinoma (PDAC), small intestine cancer, colon cancer, anal cancer, colonic mucosal adenoma, bladder cancer, kidney cancer, male genital tract tumor, penile cancer, prostate cancer, female genital tract tumor, cervical cancer, endometrial cancer, ovarian cancer, ovarian adenocarcinoma, uterine sarcoma, vulvar intraepithelial neoplasia, vaginal cancer, female external genitalia cancer, female urethral cancer, and skin cancer.

12. The pharmaceutical composition of claim 10, wherein the blood cancer is any one of leukemia, malignant lymphoma, multiple myeloma, and aplastic anemia.

13. A pharmaceutical composition for the prevention or treatment of cancer, the pharmaceutical composition comprising co-administration of the compound of any one of claims 1 to 6 and an anticancer drug.

14. The pharmaceutical composition of claim 13, wherein the anticancer drug is selected from a taxane-based anticancer drug, an antitumor alkylating agent, an antitumor antimetabolite, an antitumor antibiotic, a plant-derived antitumor agent, an antitumor platinum complex, an antitumor camptothecin derivative, an antitumor kinase inhibitor, an antitumor antibody, a hormonal antitumor agent, an antitumor viral agent, and an angiogenesis inhibitor.

15. The pharmaceutical composition of claim 13, wherein the taxane-based anticancer drug is selected from paclitaxel, docetaxel, or cabazitaxel.
